# EUROPEAN PATENT SPECIFICATION

(11) **EP 4 096 618 B1**
(45) Date of publication and mention of the grant of the patent: **29.01.2025**
(21) Application number: 20842554.6
(22) Date of filing: 09.12.2020
(51) Int. Cl.: A61K 8/33, A61K 8/34, A61K 8/35, A61K 8/37, A61K 8/49, A61Q 19/00, A61L 9/01

(54) **A MIXTURE COMPRISING 1,2-ALKANEDIOLS**
MISCHUNG MIT 1,2-ALKANDIOLEN
MÉLANGE COMPRENANT DES 1,2-ALCANEDIOLS

(43) Date of publication of application: 07.12.2022
(60) Divisional application: 24195105.2
(73) Proprietor: Symrise AG, 37603 Holzminden Niedersachsen (DE)
(72) Inventor: SIEGEL, Sven, 37671 Höxter (DE); BUGDAHN, Nikolas, 37603 Holzminden (DE); LANGE, Sabine, 37603 Holzminden (DE)
(74) Representative: Fabry, Bernd
(86) International application number: PCT/EP2020/085184
(87) International publication number: WO 2022/122143

(56) References cited:
- WO-A1-2016/049389
- WO-A1-2019/029808
- WO-A1-2020/057761
- US-A1- 2019 184 049
- DATABASE GNPD [online] MINTEL; 28 August 2020 (2020-08-28), ANONYMOUS: "Cream Oil", XP055843023, retrieved from https://www.gnpd.com/sinatra/recordpage/8042717/ Database accession no. 8042717
- DATABASE GNPD [online] MINTEL; 4 November 2020 (2020-11-04), ANONYMOUS: "Refreshing Eye Serum", XP055843006, retrieved from https://www.gnpd.com/sinatra/recordpage/8236009/ Database accession no. 8236009
- DATABASE GNPD [online] MINTEL; 28 February 2020 (2020-02-28), ANONYMOUS: "Liquid Foundation", XP055843014, retrieved from https://www.gnpd.com/sinatra/recordpage/7290267/ Database accession no. 7290267
- DATABASE GNPD [online] MINTEL; 4 November 2020 (2020-11-04), ANONYMOUS: "Refreshing Eye Serum", XP055843006, retrieved from https://www.gnpd.com/sinatra/recordpage/8236009/ Database accession no. 8236009
- DATABASE GNPD [online] MINTEL; 28 February 2020 (2020-02-28), ANONYMOUS: "Liquid Foundation", XP055843014, retrieved from https://www.gnpd.com/sinatra/recordpage/7290267/ Database accession no. 7290267
- DATABASE GNPD [online] MINTEL; 28 August 2020 (2020-08-28), ANONYMOUS: "Cream Oil", XP055843023, retrieved from https://www.gnpd.com/sinatra/recordpage/8042717/ Database accession no. 8042717

## Description

### AREA OF INVENTION

The present invention refers to the area of cosmetics, particularly of malodour reduction and covers compositions comprising 1,2-alkanediols and various malodour reduction agents.

### BACKGROUND OF THE INVENTION

Unscented or scented products are desired by consumers as they may be considered more natural and discreet than scented products. Manufacturers of unscented or scented products for controlling malodors rely on malodor reduction ingredients or other technologies (e.g. filters) to reduce malodors. However, effectively controlling malodors, for example, amine-based malodors (e.g. fish and urine), thiol and sulfide-based malodors (e.g. garlic and onion), C₂-C₁₂ carboxylic acid based malodors (e.g. body and pet odor), indole based malodors (e.g. fecal and bad. breath), short chain fatty aldehyde based malodors (e.g. grease) and geosmin based malodors (e.g. mold/mildew) may be difficult, and the time required for a product to noticeably reduce malodors may create consumer doubt as to the product's efficacy on malodors. Often times, manufacturers incorporate scented perfumes to help mask these difficult malodors.

Another area of interest - to which the present invention relates - covers malodour of raw materials which are designated for use for example in cosmetics and exhibit malodour caused by impurities and/or chemical degradation products, such as butyric acid, valeric acid, caproic acid, enanthic acid, caprylic acid, pelargonic acid, capric acid, and methyl-, ethyl-esters thereof. As well as 1-Hexen-3-one, 1-Heptene-3-one, 1-Octen-3-one, 1-Nonene-3-one, 1-decene-3-one, and Octalactone. A typical example are 1,2-hexanediol and 1,2-octanediol, but also 1,2-alkanediols in general.

Unfortunately, malodor control technologies typically cover up the malodor with a stronger scent and thus interfere with the scent of the perfumed or unperfumed situs that is treated with the malodor control technology. Thus, limited nature of the current malodor control technologies is extremely constraining. Thus what is needed is a broader palette of malodor control technologies so the perfume community can deliver the desired level of character in a greater number of situations/applications. It is also well-known that in addition to blocking a malodor's access to a sensory cell, in order to achieve the desired goal, a malodor control technology must leave such sensor cell open to other molecules, for example scent molecules. Thus, the malodor control technologies disclosed herein do not unduly interfere with the scent of the perfumed or unperfumed situs that is treated with the malodor control technology.

### RELEVANT PRIOR ART

EP 1876162 A1 describes the preparation of C5 to C8-alkanediols from the corresponding olefins by means of epoxidation and subsequent hydrolysis. The crude products so obtained were purified further therein by means of subsequent treatment, in order to remove secondary products having an unpleasant smell.

The preparation of 1,2-pentanediol is nowadays generally carried out from n-pent-1-ene, which is available from petrochemical sources. The n-pent-1-ene is reacted to give the corresponding epoxide with the aid of peroxides (e.g. hydrogen peroxide) and then converted into 1,2-pentanediol with organic acids such as formic acid or mineral acids. This preparation method is described in EP 0257243 A1 or EP 0141775 A1 and has economic and ecological disadvantages. For example, the diester of 1,2-pentanediol that is formed as an intermediate in this process must be saponified in order to obtain 1,2-pentanediol. If the epoxidation of n-pent-1-ene is carried out, for example, with hydrogen peroxide and formic acid, sodium formate is formed as a coupling product in the subsequent saponification of the di-formate of 1,2-pentanediol with sodium hydroxide solution and must be disposed of, thus leading to an high load of organics in the wast water. A nother way to obtain 1,2-pentanediol from renewable sources is laid open in WO 2019/152569 A2 (SYMRISE).

US 5,767,329 A (BASF) discloses a process according to which impurities are separated from aqueous solutions of 1,6-hexanediol or 1,6-hexanediol precursors, such as adipic and 6-hydroxycaproic acid, comprises adding at least one carboxylic acid to a solution (a) of 1,6-hexanediol and subjecting this solution (a) or a solution (b) containing carboxylic acid(s) as precursor(s) of 1,6-hexanediol to a heat treatment at temperatures above room temperature in the absence of hydrogen

US 6,528,665 B1 proposes a process for the preparation of alkanediols that are as pure as possible. According to US 6,528,665 B1**,** purification is carried out at the stage of the epoxyalkanes, before they are hydrolyzed to the corresponding alkanediols.

US 2009 0312418 AA (ADEKA) suggests a less malodorous alkanediol composition, a process for producing the alkanediol composition efficiently, and a cosmetic containing the alkanediol composition. Said alkanediol composition contains 0.005 parts by mass or less of ester compound per 100 parts by mass of alkanediol compound having four or more carbon atoms.

US 2009 048471 AA (UBE) refers to 1,5-pentanediol which contains less than or equal to 0.1 weight percent each of 1,5-hexanediol and 1,4-dihydroxycyclohexane. The 1,5-pentanediol can be produced by the direct hydrogen reduction of a dicarboxylic acid mixture in the filtrate obtained by the crystallization and separation of adipic acid.

KR 2011 0052257 A (KCI) proposes a deodorization method of 1,2-alkanediols to remove mostly peroxidation material contained in 1,2- alkanediol comprises the steps of: (i) performing at least one unit process treatment selected from the treatment with a reducing agent, the treatment with activated carbon, and the treatment with steam for 1,2-alkanediols having 6-8 carbon numbers to obtain primary deodorized 1,2-alkanediols, wherein the 1,2-alkanediols comprises one or more impurities selected from aldehyde, ketone, and carboxylic acid and (ii) decompression-distilling the primary deodorized 1,2-alkanediols to obtain secondary deodorized 1,2-alkanediols.

The so-called "MORV concept" is discussed in detail in WO 2016 049389 A1 (P&G). Particularly the MORV values of the malodor reduction agents according to the present invention can be taken from this document

### OBJECT OF THE INVENTION

None of the processes cited above provide odorless 1,2-alkanediols. Due to the purification conditions the products still contain malodor causing impurities, such as butyric acid, butyric acid esters, and optionally furfuryl alcohol or its derivatives. Therefore, it has been the object of the present invention providing mixtures of 1,2-alkanediols in general and 1,2-pentanediol, 1-2hexanediol, 1,2-octanediol and their mixtures in particular with improved olfactory performance by adding suitable malodour reduction agents.

### BRIEF DESCRIPTION OF THE INVENTION

A first object of the present invention is related to a mixture comprising or consisting of
(a) at least one 1,2-alkanediol,
(b) at least one malodor causing compound existing as an impurity or degradation product within 1,2-alkanediols, and
(c) at least one malodor reduction material having a Malodor Reduction Value (MORV) of at least 0.5, selected from the group consisting of:
   ((1s,4s)-4- isopropylcyclohexyl)methanol
   (-)-alpha-Pinene
   (-)-Citroneliol
   (+)- alpha-Pinene
   (+)-Carvone
   (+)-Dihydrocarveol
   (1R, 5R)-4,6,6-trimethylbicyclo[3.1.1]hept-3-en-2-one
   (1s,4s)-4- isopropylcyclohexyl)methanol
   (2,2-dimethoxyethyl)benzene
   (2Z,5Z)-5,6,7-trimethylocta-2,5-dien-4-one
   (2Z,6E)-nona-2,6-dienenitrile
   (3Z)-1-(2-buten-1-yloxy)-3-hexene
   (d)-Citronelial
   (E)-1-(1-methoxypropoxy)hex-3-ene
   (E)-1-1-methoxypropoxylhex-3-ene
   (E)-3,7-dimethylocta-4,6-dien-3-ol
   (E)-4,8-dimethyldeca-4,9-dienal
   (E)-4-methyldec-3-en-5-ol
   (I)-Citronellal
   (R)-(-)-Linalool
   (S)-(+)-Linalool
   (S)-(1R,5R)-4,6,6-trimethylbicyclo[3.1,1]hept-3- en-2-one
   (Z)-1-((1R,2S)-2,6,6-trimethylcyclohex-3-en--1-yl)but-2-en-1-one
   (Z)-1-((2-methylallyl)oxy)hex-3-ene
   (Z)-3-dodecenal
   (Z)-3-hepten-1-yl acetate
   (Z)-3-hexen-1 yl-2 cyclopenten-1 -one
   (Z)-5-methylheptan-3-one oxime
   (Z)-6-ethylideneoctahydro-2H-5,8-methanochrome
   (Z)-dodec-4-enal
   (Z)-hex-3-en-1-yl cyclopropanecarboxylate
   (Z)-hex-3-en-1-yl isobutyrate
   (Z)-hex-3-en-1-yl methyl carbonate
   (Z)-hex-3-en-I-yl isobutyrate
   1-(3,3-dimethyleyclohexyl)ethyl formate
   1,1,2,3,3-Pentamethylindan
   1,1-dimethoxynon-2-yne,
   1,2-dihydrolinalool
   1,2-dimethyl-3-(prop-1-en-2-yl)cyclopentan-1-ol
   1,3,3-trimethyl-2- norbornanyl acetate
   1,3,5-undecatriene
   1,4-cineole
   1,8-thiocineol
   1',1',5',5'-tetramethylhexahydro-2',5'H-spiro[[1,3]dioxolane-2,8'-[2,4a]methanonaphthalene] K
   10-undecenal
   1-acetate
   1-cyclohexylethyl-(E)-but-2-enoate
   1-decanol
   1-ethoxy-4-(tert-pentyl)cyclohexane
   1-ethyl-3-methoxytricyclo[2,2.1.02,6]heptane
   1-Ethyl-3-methoxytricyclo[2.2..1.02,6]heptane
   1-hepten-1-ol,
   1-limonene
   1-methoxy- 3a,4,5,6,7,7a-hexahydro-1H-4,7-methanoindene
   1-oxaspiro(4,5)decan-2-one
   1-Phenyl-2-pentanol
   1-phenylethyl acetate
   2- Heptyl tetrahydrofuran
   2- isopropyl-4-methylthiazole
   2-(1-ethoxyethoxy)ethyl)benzene
   2-(2,2,3-trimethyleyclopent-3-en-1-yl)acetonitrile:
   2-(6.6-dimethylbicyclo[3.1.1 ]hept-2-en-2-yl)acetaldehyde
   2-(heptan-3-yl)-1,3-dioxolane
   2-(I-ethoxyethoxy)ethyl)benzene
   2-(sec-butyl)cyclohexan-1-one
   2-(tert-butyl)cyclohexan-1-ol
   2-(tert-butyl)cyclohexyl ethyl carbonate
   2,2,5-trimetriyl-5-pentylcyc1opentan-1-one
   2,4-dimethyl-4-phenyltetrahydrofuran
   2,4-Nonadienal
   2,5-dimethyl-4-methoxy-3(2H)-ruranone
   2,6,10,10-tetramethyl-1-oxaspiro[4,5]dec-6-ene
   2,6,6-trimethylester
   2,6,9,10-tetramethyl-1-oxaspiro(4.5)deca- 3,6-diene
   2,6-dimethyloct-7-en-4-one
   2,6-dimethyl-octanal
   2,6-nonadien-1-ol
   2,6-nonadienal
   2-butoxyethanol
   2-butyl-4,4,6-trimethyl-1,3-dioxane
   2-decenal
   2-ethoxy-2,6,6-trimethyl-9-methylenebicyclo[3,3.1]nonane
   2-heptyl tetrahydrofuran
   2-heptylcyclopentan-1-one
   2-hexen-1-ol
   2-hexylcyclopent-2-en-1-one
   2-hexylcyclopentan-1-one
   2-hexylidene cyciopentanone
   2-isopropoxyethylbenzene
   2-isopropyl-1,7,7-trimethylspiro[bicyclo[2.2.1 ]heptane- 2,4'-[1,3]dioxane]
   2-isopropyl-5-methyl-2-hexenal
   2-isopropyl-N,2,3-trimethylbutyramide
   2-mercapto-2-methylpentan-1-ol
   2-methoxy-3-(1-methylpropyl)pyrazine
   2-methoxynaphthalene
   2-methyl-4-phenyl-1,3-dioxolane
   2-methyldecanenitrile
   2-metoxynaphthalene
   2-nonanol
   2-nonanone
   2-nonanone propylene glycol acetal
   2-nonen-1-al
   2-pentylcyclopentan-1-ol
   2-pentylcyclopentan-1-one
   2-phenethyl propionate
   2-phenoxyethanol
   2-phenylethyl acetate
   2-propylheptanenitrile
   2-trans-6-trans- onadienal
   2-undecenal
   2-undecenenitrile
   3 -(3 isopropylphenyl)butanal
   3 -dimethyl-5(2,2,3-trimethyl-3-cyclopenten-lyl)-4-penten-2-ol
   3-(2-ethylphenyl)-2,2-dimethylpropanal
   3-(3-isopropylphenyl)butanal
   3-(4-methoxyphenyl)-2-methylpropanal
   3-(4-methylcyclohex-3-en-1-yl)butanal
   3-(p-Isopropylphenyl)propionaldehyde
   3,3-dimethyl-3-5(2,2,3-Trimethyl-3 -Cyclopentenyl)-4-penten-2-ol
   3,6-nonadien-1-ol
   3,6-dimethyl-3-oetanyi acetate
   3,7-dimethyl-1-octanol
   3,7-dimethyl-2-methylene-6-octenal
   3a,4,5,6,7,7a-hexahydro-1H-4,7-methanoinden-6-yl acetate
   3-cyclohexene-1-carboxylic acid
   3-hexenol
   3-hexenyl isovalerate
   3-hydroxybutan-2-one
   3-methoxy-3-methyl butanol
   3-methoxy-7,7-dimethyl-10-methylenebicyclo[4.3.1]decane
   3-methyl-1,2-cyclopentanedione
   3-methylphenethyl alcohol
   3-nonylacrolein
   4-(2-methoxypropan-2-yl)-3 -methylcyclohex-1-ene
   4-(tert-butyl)cyclohexyl acetate
   4-(tert-pentyl)cyclohexan-1-one
   4,5,6,7-tetrahydro-3,6-dimethylbenzofuran
   4-carvomenthenol
   4-cyclohexyl-2-methylbutan-2-ol
   4-ethylguaiacol
   4-methyl-2-phenyl-3,6-dihydro-2H-pyran
   4-methyl-2-phenyltetrahydro-2H-pyran
   4-methylquinoline
   4-pentenophenone
   4-terpinenol
   4-tetraethyl-2-phenyl-3,6-dihydro-2H-pyran
   4-vinylphenol
   5- isopropenyl-2-methyl-2-vinyltetrahydrofuran
   5-ethyl-4-hydroxy-2-methylfuran-3(2H)-one
   5-methyl-3-heptanone
   6,6"dimethyl-2-norpmene-2-propionaidehyde
   6,6-dimethoxy-2,5,5-trimethylhex-2-ene
   6,6-dimethyl-2-methylenebicyclo [3.1.1 ]heptan-3 -ol
   6,6-dimethyl-2-norpinene-2-propionaldehyde
   6,8-diethyl-2-nonanol
   6-Isopropylquinoline
   6-methylquinoline
   7-epi-alpha-Selinene
   7-epi-sesquithujene
   7-isopropyl-8,8-dimethyl-6,10-dioxaspiro[4.5]decane
   9-decenal
   Acetaldehyde benzyl 2-methoxyethyl acetal
   Acetaldehyde dipropyl acetal
   Acetaldehyde phenylethyl propyl acetal
   Acetate C9
   Acetoanisole
   Aldehyde C-11
   Alloaromadendrene
   Allyl 2-(isopentyloxy)acetate
   Allyl heptanoate
   Allyl phenethyl ether
   Allyl-2-(isopentyloxy)acetate
   alpha -Thujone
   alpha-4-dimethyl benzenepropanal
   alpha-Acetoxystyrene
   alpha-Amorphene
   alpha-Bergamotene
   alpha-Cadinene
   alpha-Cedrene epoxide
   alpha-Cubebene
   alpha-Fenchene
   alpha-Gurjunene
   alpha-Limonene
   alpha-methyl cinnamaldehyde
   alpha-Methyl-cyclohexanepropanol
   alpha-Muurolene
   alpha-Patchouiene
   alpha-Santalene
   alpha-Selinene
   alpha-Terpinyl acetate
   alpha-Terpinyl propionate
   alpha-Thujone
   Anethole
   Anisyl acetate
   Anisyl formate
   Benzyl alcohol
   Benzyl butyrate
   Benzyl dimethyl carbinol
   Benzyl isobutyrate
   Benzyl isovalerate
   Benzyl tert-butanol
   Benzylacetone
   beta-Caryophyllene
   beta-Cedrene
   beta-Copaene
   beta-Farnesene
   beta-Patchoulline
   beta-Phellandrene
   beta-Pinene
   beta-Pinene epoxide
   beta-Selinene
   beta-Sesquiphellandrene
   beta-Terpineol
   beta-Terpinyl acetate
   Bigarade oxide
   Borneol
   Bornyl acetate
   Bornyl isobutyrate
   Camphene
   Camphor
   Camphorquinone
   Capraldehyde
   Caproyl alcohol
   Caprylic acid
   Caprylic alcohol
   Caprylnitrile
   Carbitol
   Carvacrol
   Carvone
   Chloroxylenol
   Cinnamic alcohol
   Cinnamic aldehyde
   Cinnamyl formate
   Cinnamyl nitrile
   cis-2-hexenol
   cis-3,cis-6-nonadienol
   cis-3-hexen-1-ol
   cis-3-Hexenyl 2-methylbutyrate
   cis-3-hexenyl butyrate
   cis-3-hexenyl propionate
   cis-3-hexenyl tiglate
   cis-3-hexenyl valerate
   cis-3-hexenyl-cis-3-hexenoate
   cis-4-(tert-butyl)cyclohexyl acetate
   cis-4-decen-1-al
   cis-6-nonenol
   cis-Carveol
   cis-Limonene oxide
   cis-Ocimene
   cis-Pinane
   Citral
   Citral dimethyl acetal
   Citral propylene glycol acetal
   Citronellal
   Citronellyl formate
   Citronellyl nitrile
   Cosmene
   Creosol
   Cumic alcohol
   Cyclohexylethyl acetate
   Decahydro-3H-spiro[furan-2,5 '-[4,7]methanoindene]
   Decahydro-beta-naphthol
   Decanal diethyl acetal
   Decyl propionate
   delta-3-Carene
   delta-Elemente
   Dibutyl sulfide
   Dihydro linalool
   Dihydro-alpha-ionone
   Dihydro-alpha-terpinyl acetate
   Dihydrocarveol
   Dihydrocarveol
   Dihydrocarveol acetate
   Dihydrocarvone
   Dihydroisophorone
   Dihydrojasmone
   Dihydromyrcenol
   Diphenylmethane
   Diphenyloxide
   d-Limonene
   Dodecanal dimethyl acetal
   d-p-8(9)-Menthen-2-one
   Ethyl (1R, 6S)-2,2,6-trimethylcyclohexane-1-carboxylate
   Ethyl (3aR,4S,7R,7aR)-octahydro-3aH-4,7-methanoindene-3a-carboxylate
   Ethyl 2-(cyclohexyl)propionate
   Ethyl 2-ethyl-6,6-dimethylcyclohex-2-ene-1-carboxylate
   Ethyl decanoate
   Ethyl gamma-safranate
   Ethyl hexyl ketone
   Ethyl nonanoate
   Ethyl octanoate
   Ethyl-(1R,2R,3R,4R)-3-isopropylbicyclo[2.2.1]hept-5-ene-2-carboxylate
   Eucalyptol
   Fenchyl alcohol
   Furforyl hexanoate
   gamma-Cadinene
   gamma-Himachalene
   gamma-Muurolene
   gamma-Terpeninyl acetate
   gamma-Terpinene
   gamma-Terpineol
   Geranial
   Geranyl formate
   Geranyl nitriie
   Gyrane
   Heliotropin
   Heptaldehyde
   Heptyl acetate
   Heptyl alcohol
   Hexenyl tiglate
   Hexyl 2-methylbutanoate
   Hexyl butyrate
   Hexyl hexanoate
   Hexyl propanoate
   Hexyl tiglate
   Hydratopic alcohol
   Hydratropaldehyde dimethyl acetal
   Hydrocinnamyl alcohol
   Hydrotropaldehyde dimethyl acetal
   i-(3,3-dimethylcyclohexyl)ethyl acetate
   Irisnitrile
   Isoamyl isobutyrate
   Isoamyl octanoate
   Isoborneol
   Isobornyl propionate
   Isobornyl acetate
   Isobornyl isobutyrate
   Isocyclocitral
   Isomenthone
   Isononanol
   Isononyl acetate
   Isopentyrate
   Isopropyl 2-methylbiityrate
   Isopropylvinylcarbinol
   Isopulegol
   L- Carvone
   Lauraldehyde
   Leaf acetal
   Linalool oxide (furanoid)
   Linalyl formate
   Linalyl isobutyrate
   Linalyl propionate
   Maceal
   Menthone
   Methoxycitronellal
   Methoxycyclododecane
   Methoxymelonat
   Methyl (1s,4s)-1,4-dimethylcyclohexane-1-carboxylate
   Methyl (E)-non-2-enoate
   Methyl (E)-octa-4,7-dienoate
   Methyl 2-methylbutyrate
   Methyl 2-octynoate
   Methyl alpha-cyclogeranate
   Methyl chavicol
   Methyl cinnamate
   Methyl cyclopentylideneacetate
   Methyl diphenyl ether
   Methyl eugenol
   Methyl geraniate
   Methyl isoeugenol
   Methyl isoeugenol
   Methyl nonyl acetaldehyde
   Methyl nonyl acetaldehyde dimethyl acetal
   Methyl nonyl ketone
   Methyl octine carbonate
   Methyl octyl acetaldehyde
   Methyl phenyl carbinyl propionate
   Methyl phenylacetate
   Methyl phenylethyl carbinol
   Methyl-2,2-dimethyl-6-methylenecyclohexane-1-carboxylate
   Methyl-2-methylbutyrate
   Myrcene
   Myrcenol
   Myrcenyl acetate
   Myroxide
   Myrtenal
   Nerol
   Neryl Formate
   n-Hexyl-2-butenoate
   Nonaldehyde
   Nonyl alcohol
   O -Methyl linalool
   Ocimenol
   Octahydro-1H-4,7-methanoinden-5-yl acetate
   Octahydro-1H-4,7-methanoindene-1-carbaldehyde
   Octanal
   Octanal dimethyl acetal
   Octanal propylene glycol acetal
   Octyl acetate
   para-Cymen-8-ol
   p-cresyl isobutyrate
   p-Cymene
   Perillaldehyde
   Perillyl acetate
   Phenethyl alcohol
   Phenethyl formate
   Phenylacetaldehyde ethyleneglycol acetal
   p-Isopropylphenylacetaldehyde
   p-Menth-3-en-1-ol
   p-Propyl anisole
   Propyl (S)-2-(tert-pentyloxy)propanoate
   Propylene glycol
   p-t-butyl phenyl acetaldehyde
   p-tert-Amyl cyclohexanol
   Racemic alpha-Pinene
   Rhodinol
   Sabinene
   Sabinene hydrate
   Sabinol
   Safrole
   Selina-3,7(11)-diene
   Spirodecane
   Tetrahydrogeranial
   Tetrahydrojasmone
   Tetrahydrolinalool
   Tetrahydrolmalyl acetate
   Thujopsene
   Thymol
   Thymol methyl ether
   trans,trans-2,4-nonadienal
   trans-2,cis-6-nonadienal
   trans-2-decenal
   trans-2-hexenol
   trans-2-nonen-1-al
   trans-2-tert-butylcyclohexanol
   trans-3, cis-6-nonadienol
   trans-4-Decen-1-al
   trans-Anethole
   trans-beta-ocimene
   trans-beta-ociraene
   trans-Dihydrocarvone
   trans-Geraniol
   Undecanal
   Valencene

Surprisingly it has been observed, that the cited agents showing MORV values of at least 0.5 are suitable for masking the malodour of 1,2-alkanediols which is caused by certain impurities and chemical degradation products.

As used herein "MORV" is the calculated malodor reduction value for a subject material A material's MORV indicates such material's ability to decrease or even eliminate the perception of one or more malodors. For purposes of the present application, a material's MORV is calculated in accordance with method found in the test methods section of the present application.

### 1,2-alkanediols

1,2-alkanediols according to the present invention forming component (a) comprise 5 to 12 carbon atoms. Preferably they are selected from the group consisting of 1,2-pentanediol, 1,2-hexanediol, 1,2-heptanediol, 1,2-octanediol, 1,2-nonanediol, 1,2-decanediol, 1,2-undecanediol, 1,2-dodecandiole and their mixtures. The most prferred 1,2-alkanediols are 1,2-hexanediol, 1,2-octanediol and mixtures thereof.

### Malodor causing agents

The malodor causing compounds (component b) are selected from the group consisting of
(b1) butyric acid, valeric acid, caproic acid, enanthic acid, caprylic acid, pelargonic acid, capric acid, and methyl-, ethyl- esters thereof. 1-hexene-3-one, 1-heptene-3-one, 1-octen-3-one, 1-nonene-3-one, 1-decene-3-one, octalactone, 1-hexene, 1-heptene, 1-octene, 1-nonene, 1-decene, 2-ethyl-1-butene, 2-ethyl-1-pentene, 2-ethyl-1-hexene, 2-ethyl-1-heptene, 2-ethyl-1-octene, cis-3-methyl-2-pentene, cis-3-methyl-2-hexene, cis-3-methyl-2-heptene, cis-3-methyl-2-octene, cis-3-methyl-2-nonene, trans-3-methyl-2-pentene, trans-3-methyl-2-hexene, trans-3-methyl-2-heptene, trans-3-methyl-2-octene, trans-3-methyl-2-nonene, n-hexane, n-heptane, n-octane, n-nonane, n-decane, cis-2-hexene, cis-2-heptene, cis-2-octene, cis-2-nonene, cis-2-decene, trans-2-hexene, trans-2-heptene, trans-2-octene, trans-2-nonene, trans-2-decene, cis-3-hexene, cis-3-heptene, cis-3-octene, cis-3-nonene, cis-3-decene, trans-3-hexene, trans-3-heptene, trans-3-octene, trans-3-nonene, trans-3-decene, cis-2,3-hexanediol, cis-2,3-heptanediol, cis-2,3-octanediol, cis-2,3-nonenediol, cis-2,3-decanediol, trans-2,3-hexanediol, trans-2,3-heptanediol, trans-2,3-octanediol, trans-2,3-nonenediol, trans-2,3-decanediol, cis-3,4-hexanediol, cis-3,4-heptanediol, cis-3,4-octanediol, cis-3,4-nonenediol, cis-3,4-decanediol, trans-3,4-hexanediol, trans-3,4-heptanediol, trans-3,4-octanediol, trans-3,4-nonenediol, trans-3,4-decanediol, 3-methylpentane-2,3-diol, 3-methylhexane-2,3-diol, 3-methylheptane-2,3-diol, 3-methyloctane-2,3-diol, 3-methylnonane-2,3-diol, 2-ethylbutane-1,2-diol, 2-ethylpentane-1,2-diol, 2-ethylhexane-1,2-diol, 2-ethylheptane-1,2-diol, 2-ethyloctane-1,2-diol, or mixtures thereof, or
(b2) or one, two, three or more compounds according to one of the following formulas (I) to (IV) 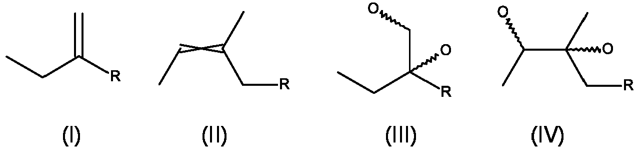 in which R stands for a linear branched alkyl radical having 1 to 10 carbon atoms, including all cis and trans stereoisomers, and

### Mixtures

In a typical embodiment the mixture according to the present invention include components (a) and (c) in a ratio by weight of from about 90:10 to about 99.99:0.01 while component (b) is present in an amount of from about 10 ppm to about 60.000 ppm (0,001 -to 6 wt.-percent)

Preferably the ratio by weight of componente (a) and (c) ranges from about 99.9:0.1.

Preferably component (b) is present in an amount of from about 50 ppm to about 20.000 ppm (0,005 to 2 wt.-percent).and more preferably in an amount of from about 100 ppm to about 10.000 ppm (0,01 to 1 wt.-percent).

In another preferred embodiment the mixtures further comprise a liquid carrier, such as for example water, C₁-C₄ aliphatic alcohols, and polyols having 2 to 12 carbon atoms and 1 to 5 hydroxyl groups, and lipid phases like natural oils and fatty acid esters, and mixtures thereof. The carrier may represent about 5 to about 95, preferably about 10 to about 80 and more preferably about 25 to about 50 wt.-percent of the mixture.

### CONSUMER PRODUCTS

Another object of the present invention refers to consumer products comprising the compositions as explained above. These products may represent a cosmetic composition, a pharmaceutical composition, an oral composition, a detergent composition or a food composition.

More particularly said consumer product can be chosen from the group consisting of products for baby care, beauty care, fabric and home care, family care, feminine care, health care, snack and/or beverage products or devices. Such products include but are not limited to plastic films garbage bags, storage bags, storage wraps, diapers, bibs, wipes, garments, textiles including sheets and towels, composters; products for and/or methods relating to treating hair (human, dog, and/or cat), including, bleaching, coloring, dyeing, conditioning, wet or dry shampooing, styling, scalp treatments; deodorants and antiperspirants; personal cleansing; cosmetics; skin care including application of creams, lotions, fine fragrances and other topically applied products for consumer use; and shaving products, products for and/or methods relating to treating fabrics, hard surfaces and any other surfaces in the area of fabric and home care, including; air care including air filtration, car care, dishwashing, fabric conditioning (including softening), laundry detergency, laundry and rinse additive and/or care, hard surface cleaning and/or treatment, towel bowl cleaners and other cleaning and/or malodor treatments for consumer, agricultural, industrial or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening.

The products may comprise said mixtures in an amount of from about 10 to about 0.01 wt.-percent and preferably in an amount of from about 5 to about 1 wt.-percent - calculated on the composition.

The compositions pursuant the present invention may contain abrasives, anti-acne agents, agents against ageing of the skin, anti-cellulitis agents, antidandruff agents, antiinflammatory agents, irritation-preventing agents, irritation-inhibiting agents, antioxidants, astringents, perspiration-inhibiting agents, antiseptic agents, ant-statics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colour-protecting agents, pigments, anticorrosives, aromas, flavouring substances, odoriferous substances, polyols, surfactants, electrolytes, organic solvents or silicone derivatives and the like as additional auxiliaries and additives.

### Surfactants

Preferred auxiliaries and additives are anionic and/or amphoteric or zwitterionic surfactants. Typical examples encompass: Almondamidopropylamine Oxide, Almondamidopro-pyl Betaine, Aminopropyl Laurylglutamine, Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium Cocoyl Isethionate, Ammonium Cocoyl Sarcosinate, Ammonium C12-15 Pareth Sulfate, Ammonium C9-10 Perfluoroalkylsulfonate, Ammonium Dinonyl Sulfosuccinate, Ammonium Dodecylbenzenesulfonate, Ammonium Isostearate, Ammonium Laureth-6 Carboxylate, Ammonium Laureth-8 Carboxylate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauroyl Sarcosinate, Ammonium Lauryl Sulfate, Ammonium Lauryl Sulfosuccinate, Ammonium Myreth Sulfate, Ammonium Myristyl Sulfate, Ammonium Nonoxynol-4 Sulfate, Ammonium Nonoxynol-30 Sulfate, Ammonium Oleate, Ammonium Palm Kernel Sulfate, Ammonium Stearate, Ammonium Tallate, AMPD-Isostearoyl Hydrolyzed Collagen, AMPD-Rosin Hydrolyzed Collagen, AMP-Isostearoyl Hydrolyzed Collagen, AMP-Isostearoyl Hydrolyzed Keratin, AMP-Isostearoyl Hydrolyzed Soy Protein, AMP-Isostearoyl Hydrolyzed Wheat Protein, Apricotamidopropyl Betaine, Arachidic Acid, Arginine Hexyldecyl Phosphate, Avocadamidopropyl Betaine, Avocado Oil Glycereth-8 Esters, Babassu Acid, Babassuamidopropylamine Oxide, Babassuamidopropyl Betaine, Beeswax Acid, Behenamidopropyl Betaine, Behenamine Oxide, Beheneth-25, Beheneth-30, Behenic Acid, Behenyl Betaine, Bis- Butyldimethicone Polyglyceryl-3, Butoxynol-5 Carboxylic Acid, Butoxynol-19 Carboxylic Acid, Butyldimoniumhydroxypropyl Butylglucosides Chloride, Butyldimoniumhydroxypropyl Laurylglucosides Chloride, Butyl Glucoside, Butylglucoside Caprate, Butylglucosides Hydroxypropyltrimonium Chloride, Butyloctanoic Acid, C18-36 Acid, C20-40 Acid, C30-50 Acid, C16-22 Acid Amide MEA, Calcium Dodecylbenzenesulfonate, Calcium Lauroyl Taurate, C9-16 Alkane/Cycloalkane, C10-14 Alkyl Benzenesulfonic Acid, C12-14 Alkyl Di-aminoethylglycine HCL, C9-15 Alkyl Phosphate, Candida Bombicola/Glucose/Methyl Rapeseedate Ferment, Canolamidopropyl Betaine, Capric Acid, Caproic Acid, Caproyl Ethyl Glucoside, Capryl/Capramidopropyl Betaine, Capryleth-4 Carboxylic Acid, Capryleth-6 Carboxylic Acid, Capryleth-9 Carboxylic Acid, Caprylic Acid, Capryloyl Collagen Amino Acids, Capryloyl Glycine, Capryloyl Hydrolyzed Collagen, Capryloyl Hydrolyzed Keratin, Capryloyl Keratin Amino Acids, Capryloyl Silk Amino Acids, Caprylyl/Capryl Glucoside, Caprylyl/Capryl Wheat Bran/Straw Glycosides, Caprylyl Glucoside, Caprylyl Glyceryl Ether, Caprylyl Pyrrolidone, Carnitine, Ceteareth-20, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Ceteareth-55, Ceteareth-60, Ceteareth-80, Ceteareth-100, Ceteareth-25 Carboxylic Acid, Ceteareth-2 Phosphate, Ceteareth-4 Phosphate, Ceteareth-5 Phosphate, Ceteareth-10 Phosphate, Ceteth-20, Ceteth-23, Ceteth-24, Ceteth-25, Ceteth-30, Ceteth-40, Ceteth-45, Ceteth-150, Ceteth-8 Phosphate, Ceteth-10 Phosphate, Ceteth-20 Phosphate, Cetoleth-22, Cetoleth-24, Cetoleth-25, Cetoleth-30, Cetyl Betaine, Chrysanthemum Sinense Flower Extract, C12-14 Hydroxyalkyl Hydroxyethyl Beta-Alanine, C12-14 Hydroxyalkyl Hydroxyethyl Sarcosine, Co-camidoethyl Betaine, Cocamidopropylamine Oxide, Cocamidopropyl Betainamide MEA Chloride, Cocamidopropyl Betaine, Cocamidopropyl Hydroxysultaine, Cocamine Oxide, Cocami-nobutyric Acid, Cocaminopropionic Acid, Coceth-7 Carboxylic Acid, Coceth-4 Glucoside, Co-coamphodipropionic Acid, Cocobetainamido Amphopropionate, Coco-Betaine, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Coco-Glucoside, Cocoglucosides Hydroxypropyltrimonium Chloride, Coco- Hydroxysultaine, Coco-Morpholine Oxide, Coconut Acid, Coconut Oil Glycereth-8 Esters, Coco/Oleamidopropyl Betaine, Coco-Sultaine, Coco/Sunfloweramidopropyl Betaine, Cocoylcholine Methosulfate, Cocoyl Glutamic Acid, Cocoyl Hydrolyzed Collagen, Cocoyl Hydrolyzed Keratin, Cocoyl Hydrolyzed Oat Protein, Cocoyl Hydrolyzed Rice Protein, Cocoyl Hydrolyzed Silk, Cocoyl Hydrolyzed Soy Protein, Cocoyl Hydrolyzed Wheat Protein, Cocoyl Sarcosine, Corn Acid, Cottonseed Acid, Cottonseed Oil Glycereth-8 Esters, C10-16 Pareth-1, C10-16 Pareth-2, C11-13 Pareth-6, C11-13 Pareth-9, C11-13 Pareth-10, C11-15 Pareth-30, C11-15 Pareth-40, C12-13 Pareth-1, C12-13 Pareth- 23, C12-14 Pareth-5, C12-14 Pareth-9, C13-15 Pareth-21, C14-15 Pareth-8, C20-22 Pareth-30, C20- 40 Pareth-40, C20-40 Pareth-95, C22-24 Pareth-33, C30-50 Pareth-40, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth- 12 Carboxylic Acid, C14-15 Pareth-8 Carboxylic Acid, C6-10 Pareth-4 Phosphate, C12-13 Pareth-2 Phosphate, C12-13 Pareth-10 Phosphate, C12-15 Pareth-6 Phosphate, C12-15 Pareth-8 Phosphate, C12-15 Pareth-10 Phosphate, C12-16 Pareth-6 Phosphate, C4-18 Perfluoroalkylethyl Thiohydroxypropyltrimonium Chloride, Cupuassuami-dopropyl Betaine, DEA-C12-13 Alkyl Sulfate, DEA-C12-15 Alkyl Sulfate, DEA-Ceteareth-2 Phosphate, DEA-Cetyl Sulfate, DEA- Cocoamphodipropionate, DEA-C12-13 Pareth-3 Sulfate, DEA-Cyclocarboxypropyloleate, DEA- Dodecylbenzenesulfonate, DEA-Isostearate, DEA-Laureth Sulfate, DEA-Lauryl Sulfate, DEA- Linoleate, DEA-Methyl Myristate Sulfonate, DEA-Myreth Sulfate, DEA-Myristate, DEA-Myristyl Sulfate, DEA-Oleth-5 Phosphate, DEA-Oleth-20 Phosphate, DEA PG-Oleate, Deceth-7 Carboxylic Acid, Deceth-7 Glucoside, Deceth-9 Phosphate, Decylamine Oxide, Decyl Betaine, Decyl Glucoside, Decyltetradeceth-30, Decyltetradecylamine Oxide, Diammonium Lauramido-MEA Sulfosuccinate, Diammonium Lauryl Sulfosuccinate, Diammonium Oleamido PEG-2 Sulfosuccinate, Dibutoxymethane, Di-Cl 2-15 Pareth-2 Phosphate, Di-Cl 2-15 Pareth-4 Phosphate, Di-Cl 2-15 Pareth-6 Phosphate, Di-C12-15 Pareth-8 Phosphate, Di-Cl 2-15 Pareth-10 Phosphate, Didodecyl Butanetetracarbox-ylate, Diethylamine Laureth Sulfate, Diethylhexyl Sodium Sulfosuccinate, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone PEG-7 Phosphate, Dimethicone PEG-10 Phosphate, Dimethicone PEG/PPG-7/4 Phosphate, Dimethicone PEG/PPG-12/4 Phosphate, Dimethicone/Polyglycerin-3 Crosspolymer, Dimethicone Propyl PG- Betaine, Dimyristyl Phosphate, Dioleoylamidoethyl Hydroxyethyl-monium Methosulfate, DIPA- Hydrogenated Cocoate, DIPA-Lanolate, DIPA-Myristate, Dipotassium Capryloyl Glutamate, Dipotassium Lauryl Sulfosuccinate, Dipotassium Undecylenoyl Glutamate, Disodium Babassuamido MEA-Sulfosuccinate, Disodium Caproamphodiacetate, Disodium Caproamphodipropionate, Disodium Capryloamphodiacetate, Disodium Caprylo-amphodipropionate, Disodium Capryloyl Glutamate, Disodium Cetearyl Sulfosuccinate, Disodium Cetyl Phenyl Ether Disulfonate, Disodium Cetyl Sulfosuccinate, Disodium Cocamido MEA-Sulfosuccinate, Disodium Cocamido MIPA PEG-4 Sulfosuccinate, Disodium Cocamido MIPA-Sulfosuccinate, Disodium Cocamido PEG-3 Sulfosuccinate, Disodium Coceth-3 Sulfosuccinate, Disodium Cocoamphocarboxyethylhydroxypropylsulfonate, Disodium Cocoamphodiacetate, Disodium Cocoamphodipropionate, Disodium Coco-Glucoside Sulfosuccinate, Disodium Coco-Sulfosuccinate, Disodium Cocoyl Butyl Gluceth-10 Sulfosuccinate, Disodium Cocoyl Glutamate, Disodium C12-14 Pareth-1 Sulfosuccinate, Disodium C12-14 Pareth-2 Sulfosuccinate, Disodium C12-15 Pareth Sulfosuccinate, Disodium C12-14 Sec-Pareth-3 Sulfosuccinate, Disodium C12-14 Sec-Pareth-5 Sulfosuccinate, Disodium C12-14 Sec-Pareth-7 Sulfosuccinate, Disodium C12-14 Sec-Pareth-9 Sulfosuccinate, Disodium C12-14 Sec-Pareth-12 Sulfosuccinate, Disodium Deceth-5 Sulfosuccinate, Disodium Deceth-6 Sulfosuccinate, Disodium Decyl Phenyl Ether Disulfonate, Disodium Dihydroxyethyl Sulfosuccinylundecyle-nate, Disodium Ethylene Dicocamide PEG-15 Disulfate, Disodium Hydrogenated Cottonseed Glyceride Sulfosuccinate, Disodium Hydrogenated Tallow Glutamate, Disodium Hydroxydecyl Sorbitol Citrate, Disodium Isodecyl Sulfosuccinate, Disodium Isostearamido MEA-Sulfosuccinate, Disodium Isostearamido MIPA-Sulfosuccinate, Disodium Isostearoamphodi-acetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido MIPA Glycol Sulfosuccinate, Disodium Lauramido PEG-2 Sulfosuccinate, Disodium Lauramido PEG-5 Sulfosuccinate, Disodium Laureth-5 Carboxyamphodiacetate, Disodium Laureth-7 Citrate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauriminobishydroxypropyl-sulfonate, Disodium Lauriminodiacetate, Disodium Lauriminodipropionate, Disodium Lauriminodipropionate Tocopheryl Phosphates, Disodium Lauroamphodiacetate, Disodium Lauro-amphodipropionate, Disodium N- Lauroyl Aspartate, Disodium Lauroyl Glutamate, Disodium Lauryl Phenyl Ether Disulfonate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Phosphate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-12 Dimethicone Sulfosuccinate, Disodium PEG-8 Palm Glycerides Sulfosuccinate, Disodium PPG-2-Isodeceth-7 Carboxyamphodiacetate, Disodium Ricino-leamido MEA-Sulfosuccinate, Disodium Sitostereth-14 Sulfosuccinate, Disodium Soyamphodiacetate, Disodium Stearamido MEA-Sulfosuccinate, Disodium Steariminodi-propionate, Disodium Stearoamphodiacetate, Disodium Stearoyl Glutamate, Disodium Stearyl Sulfosuccinamate, Disodium Stearyl Sulfosuccinate, Disodium 2-Sulfolaurate, Disodium 2-Sulfopalmitate, Disodium Tallamido MEA-Sulfosuccinate, Disodium Tallowamido MEA-Sulfosuccinate, Disodium Tallowamphodiacetate, Disodium Tallowiminodipropionate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Undecylenoyl Glutamate, Disodium Wheat Germamido MEA-Sulfosuccinate, Disodium Wheat Germamido PEG-2 Sulfosuccinate, Disodium Wheatgermamphodiacetate, Di-TEA-Cocamide Diacetate, Di-TEA-Oleamido PEG-2 Sulfosuccinate, Di-TEA-Palmitoyl Aspartate, Ditridecyl Sodium Sulfosuccinate, Dodecylbenzene Sulfonic Acid, Erucamidopropyl Hydroxysultaine, Ethylhexeth-3 Carboxylic Acid, Ethyl PEG-15 Cocamine Sulfate, Glyceryl Capryl Ether, Hex-yldecanoic Acid, Hydrogenated Coconut Acid, Hydrogenated Laneth-25, Hydrogenated Men-haden Acid, Hydrogenated Palm Acid, Hydrogenated Palm Kernel Amine Oxide, Hydrogenated Tallow Acid, Hydrogenated Tallowamine Oxide, Hydrogenated Tallow Betaine, Hydrogenated Talloweth-25, Hydrogenated Tallowoyl Glutamic Acid, Hydrolyzed Candida Bombicola Extract, Hydroxyceteth-60, Hydroxyethyl Acetomonium PG-Dimethicone, Hydroxyethylbutyl-amine Laureth Sulfate, Hydroxyethyl Carboxymethyl Cocamidopropylamine, Hydroxyethyl Hydroxypropyl C12-15 Alkoxypropylamine Oxide, Hydroxylauryl/Hydroxymyristyl Betaine, Hydroxystearic Acid, Hydroxysuccinimidyl C10-40 Isoalkyl Acidate, Hydroxysuccinimidyl C21-22 Isoalkyl Acidate, Hydroxysultaines, IPDI/PEG-15 Soyamine Oxide Copolymer, IPDI/PEG-15 Soyethonium Ethosulfate Copolymer, IPDI/PEG-15 Soy Glycinate Copolymer, Isoceteth-30, Isolaureth-4 Phosphate, lsopolyglyceryl-3 Dimethicone, lsopolyglyceryl-3 Dimethiconol, Isopropanolamine Lanolate, Isopropylamine Dodecylbenzenesulfonate, Isostearamidopropyla-mine Oxide, Isostearamidopropyl Betaine, Isostearamidopropyl Morpholine Oxide, Isos-teareth-8, Isosteareth-16, Isosteareth-22, Isosteareth-25, Isosteareth-50, Isostearic Acid, Isostearoyl Hydrolyzed Collagen, Jojoba Oil PEG-150 Esters, Jojoba Wax PEG-80 Esters, Jojoba Wax PEG-120 Esters, Laneth-20, Laneth-25, Laneth-40, Laneth-50, Laneth-60, Laneth-75, Lanolin Acid, Lauramidopropylamine Oxide, Lauramidopropyl Betaine, Lauramidopropyl Hydroxysultaine, Lauramine Oxide, Lauraminopropionic Acid, Laurdimoniumhydroxypropyl Decylglucosides Chloride, Laurdimoniumhydroxypropyl Laurylglucosides Chloride, Laureth-16, Laureth-20, Laureth-21, Laureth-23, Laureth-25, Laureth-30, Laureth-38, Laureth-40, Laureth-3 Carboxylic Acid, Laureth-4 Carboxylic Acid, Laureth-5 Carboxylic Acid, Laureth- 6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Laureth-6 Citrate, Laureth-7 Citrate, Laureth-1 Phosphate, Laureth-2 Phosphate, Laureth-3 Phosphate, Laureth-4 Phosphate, Laureth-7 Phosphate, Laureth-8 Phosphate, Laureth-7 Tartrate, Laurie Acid, Laurimino Bispropanediol, Lauriminodipropionic Acid, Lauroamphodipropionic Acid, Lauroyl Beta-Alanine, Lauroyl Collagen Amino Acids, Lauroyl Ethyltrimonium Methosulfate, Lauroyl Hydrolyzed Collagen, Lauroyl Hydrolyzed Elastin, Lauroyl Methyl Glucamide, Lauroyl Sarcosine, Lauroyl Silk Amino Acids, Lauryl Betaine, Lauryl Dimethicone/Polyglycerin-3 Crosspolymer, Lauryldimoniumhydroxypropyl Cocoglucosides Chloride, Lauryl Glucoside, Laurylglucosides Hydroxypropyltrimonium Chloride, Lauryl Glycol Hydroxypropyl Ether, Lauryl Hydroxysultaine, Lauryl Malamide, Lauryl Methylglucamide, Lauryl/Myristyl Glycol Hydroxypropyl Ether, Lauryl/Myristyl Wheat Bran/Straw Glycosides, Lauryl Polyglyceryl-3 Polydimethylsiloxyethyl Dimethicone, Lauryl Pyrrolidone, Lauryl Sultaine, Linoleic Acid, Linolenic Acid, Linseed Acid, Lysine Cocoate, Macadamia Seed Oil Glycereth-8 Esters, Magnesium Coceth Sulfate, Magnesium Coco-Sulfate, Magnesium Isododecylbenzene-sulfonate, Magnesium Laureth-11 Carboxylate, Magnesium Laureth Sulfate, Magnesium Laureth-5 Sulfate, Magnesium Laureth-8 Sulfate, Magnesium Laureth-16 Sulfate, Magnesium Laureth-3 Sulfosuccinate, Magnesium Lauryl Hydroxypropyl Sulfonate, Magnesium Lauryl Sulfate, Magnesium Methyl Cocoyl Taurate, Magnesium Myreth Sulfate, Magnesium Oleth Sulfate, Magnesium/TEA-Coco-Sulfate, Manicouagan Clay, MEA-Cocoate, MEA-Laureth-6 Carboxylate, MEA- Laureth Sulfate, MEA-Lauryl Sulfate, MEA PPG-6 Laureth-7 Carboxylate, MEA-PPG-8-Steareth-7 Carboxylate, MEA-Undecylenate, Meroxapol 108, Meroxapol 174, Meroxapol 178, Meroxapol 254, Meroxapol 255, Meroxapol 258, Meroxapol 314, Methoxy PEG-450 Amidoglutaroyl Succinimide, Methoxy PEG-450 Amido Hydroxysuccinimidyl Suc-cinamate, Methoxy PEG-450 Maleimide, Methyl Morpholine Oxide, Milkamidopropyl Amine Oxide, Milkamidopropyl Betaine, Minkamidopropylamine Oxide, Minkamidopropyl Betaine, MIPA C12-15 Pareth Sulfate, MIPA-Dodecylbenzenesulfonate, MIPA-Laureth Sulfate, MIPA-Lauryl Sulfate, Mixed Isopropanolamines Lanolate, Mixed Isopropanolamines Lauryl Sulfate, Mixed Isopropanolamines Myristate, Morpholine Oleate, Morpholine Stearate, Myreth-3 Carboxylic Acid, Myreth-5 Carboxylic Acid, Myristalkonium Chloride, Myristamidopropylamine Oxide, Myristamidopropyl Betaine, Myristamidopropyl Dimethylamine Phosphate, Myristamidopropyl Hydroxysultaine, Myristamidopropyl PG-Dimonium Chloride Phosphate, Myrista-mine Oxide, Myristaminopropionic Acid, Myristic Acid, Myristoyl Ethyltrimonium Methosulfate, Myristoyl Glutamic Acid, Myristoyl Hydrolyzed Collagen, Myristoyl Sarcosine, Myristyl Betaine, Myristyl/Cetyl Amine Oxide, Myristyldimoniumhydroxypropyl Cocoglucosides Chloride, Myristyl Glucoside, Myristyl Phosphate, Nonoxynol-20, Nonoxynol-23, Nonoxynol-25, Nonoxynol-30, Nonoxynol-35, Nonoxynol-40, Nonoxynol-44, Nonoxynol-50, Nonoxynol-100, Nonoxynol-120, Nonoxynol-5 Carboxylic Acid, Nonoxynol-8 Carboxylic Acid, Nonoxynol-10 Carboxylic Acid, Nonoxynol-3 Phosphate, Nonoxynol-4 Phosphate, Nonoxynol-6 Phosphate, Nonoxynol-9 Phosphate, Nonoxynol-10 Phosphate, Nonyl Nonoxynol-30, Nonyl Nonoxynol-49, Nonyl Nonoxynol-100, Nonyl Nonoxynol-150, Nonyl Nonoxynol-7 Phosphate, Nonyl Nonoxynol-8 Phosphate, Nonyl Nonoxynol-9 Phosphate, Nonyl Nonoxynol-10 Phosphate, Nonyl Nonoxynol-11 Phosphate, Nonyl Nonoxynol-15 Phosphate, Nonyl Nonoxynol-24 Phosphate, Oatamidopropyl Betaine, Octoxynol-16, Octoxynol-25, Octoxynol-30, Octoxynol-33, Octoxynol-40, Octoxynol-70, Octoxynol-20 Carboxylic Acid, Octyldodeceth-20, Octyldodeceth-25, Octyldodeceth-30, Oleamidopropylamine Oxide, Oleamidopropyl Betaine, Oleamidopropyl Hydroxysultaine, Oleamine Oxide, Oleic Acid, Oleoyl Hydrolyzed Collagen, Oleoyl Sarcosine, Oleth-20, Oleth-23, Oleth-24, Oleth-25, Oleth-30, Oleth-35, Oleth-40, Oleth-44, Oleth-50, Oleth-3 Carboxylic Acid, Oleth-6 Carboxylic Acid, Oleth-10 Carboxylic Acid, Oleyl Betaine, Olivamidopropylamine Oxide, Olivamidopropyl Betaine, Olive Acid, Olivoyl Hydrolyzed Wheat Protein, Ophiopogon Extract Stearate, Ozonized Oleth-10, Ozonized PEG-10 Oleate, Ozonized PEG-14 Oleate, Ozonized Polysorbate 80, Palm Acid, Pal-mamidopropyl Betaine, Palmeth-2 Phosphate, Palmitamidopropylamine Oxide, Palmitami-dopropyl Betaine, Palmitamine Oxide, Palmitic Acid, Palmitoyl Collagen Amino Acids, Palmitoyl Glycine, Palmitoyl Hydrolyzed Collagen, Palmitoyl Hydrolyzed Milk Protein, Palmitoyl Hydrolyzed Wheat Protein, Palmitoyl Keratin Amino Acids, Palmitoyl Oligopeptide, Palmitoyl Silk Amino Acids, Palm Kernel Acid, Palm Kernelamidopropyl Betaine, Peach Kernel Oil Glycereth-8 Esters, Peanut Acid, PEG-10 Castor Oil, PEG-40 Castor Oil, PEG-44 Castor Oil, PEG-50 Castor Oil, PEG-54 Castor Oil, PEG-55 Castor Oil, PEG-60 Castor Oil, PEG-80 Castor Oil, PEG-100 Castor Oil, PEG-200 Castor Oil, PEG-11 Cocamide, PEG-6 Cocamide Phosphate, PEG-4 Cocamine, PEG-8 Cocamine, PEG-12 Cocamine, PEG-150 Dibehenate, PEG-90 Diisostearate, PEG-75 Dilaurate, PEG-150 Dilaurate, PEG-75 Dioleate, PEG-150 Dioleate, PEG-75 Distearate, PEG-120 Distearate, PEG-150 Distearate, PEG-175 Distearate, PEG-190 Distearate, PEG-250 Distearate, PEG-30 Glyceryl Cocoate, PEG-40 Glyceryl Cocoate, PEG-78 Glyceryl Cocoate, PEG-80 Glyceryl Cocoate, PEG-30 Glyceryl Isostearate, PEG-40 Glyceryl Isostearate, PEG-50 Glyceryl Isostearate, PEG-60 Glyceryl Isostearate, PEG-90 Glyceryl Isostearate, PEG-23 Glyceryl Laurate, PEG-30 Glyceryl Laurate, PEG-25 Glyceryl Oleate, PEG-30 Glyceryl Oleate, PEG-30 Glyceryl Soyate, PEG-25 Glyceryl Stearate, PEG-30 Glyceryl Stearate, PEG-40 Glyceryl Stearate, PEG-120 Glyceryl Stearate, PEG-200 Glyceryl Stearate, PEG-28 Glyceryl Tallowate, PEG-80 Glyceryl Tallowate, PEG-82 Glyceryl Tallowate, PEG-130 Glyceryl Tallowate, PEG-200 Glyceryl Tallowate, PEG-45 Hydrogenated Castor Oil, PEG-50 Hydrogenated Castor Oil, PEG-54 Hydrogenated Castor Oil, PEG-55 Hydrogenated Castor Oil, PEG-60 Hydrogenated Castor Oil, PEG-80 Hydrogenated Castor Oil, PEG-100 Hydrogenated Castor Oil, PEG-200 Hydrogenated Castor Oil, PEG-30 Hydrogenated Lanolin, PEG-70 Hydrogenated Lanolin, PEG-50 Hydrogenated Palmamide, PEG-2 Isostearate, PEG-3 Isostearate, PEG-4 Isostearate, PEG-6 Isostearate, PEG-8 Isostearate, PEG-10 Isostearate, PEG-12 Isostearate, PEG-20 Isostearate, PEG-30 Isostearate, PEG-40 Isostearate, PEG- 26 Jojoba Acid, PEG-40 Jojoba Acid, PEG-15 Jojoba Alcohol, PEG-26 Jojoba Alcohol, PEG-40 Jojoba Alcohol, PEG-35 Lanolin, PEG-40 Lanolin, PEG-50 Lanolin, PEG-55 Lanolin, PEG-60 Lanolin, PEG- 70 Lanolin, PEG-75 Lanolin, PEG-85 Lanolin, PEG-100 Lanolin, PEG-150 Lanolin, PEG-75 Lanolin Oil, PEG-2 Lauramide, PEG-3 Lauramine Oxide, PEG-20 Laurate, PEG-32 Laurate, PEG-75 Laurate, PEG-150 Laurate, PEG-70 Mango Glycerides, PEG-20 Mannitan Laurate, PEG-8 Methyl Ether Dimethicone, PEG-120 Methyl Glucose Dioleate, PEG-80 Methyl Glucose Laurate, PEG-120 Methyl Glucose Trioleate, PEG-4 Montanate, PEG-30 Oleamine, PEG-20 Oleate, PEG-23 Oleate, PEG-32 Oleate, PEG-36 Oleate, PEG-75 Oleate, PEG-150 Oleate, PEG-20 Palmitate, PEG-150 Polyglyceryl-2 Tristearate, PEG/PPG-28/21 Acetate Dimethicone, PEG/PPG-24/18 Butyl Ether Dimethicone, PEG/PPG-3/17 Copolymer, PEG/PPG-5/35 Copolymer, PEG/PPG-8/55 Copolymer, PEG/PPG-10/30 Copolymer, PEG/PPG-10/65 Copolymer, PEG/PPG-12/35 Copolymer, PEG/PPG-16/17 Copolymer, PEG/PPG-20/9 Copolymer, PEG/PPG-20/20 Copolymer, PEG/PPG-20/60 Copolymer, PEG/PPG- 20/65 Copolymer, PEG/PPG-22/25 Copolymer, PEG/PPG-28/30 Copolymer, PEG/PPG-30-35 Copolymer, PEG/PPG-30/55 Copolymer, PEG/PPG-35/40 Copolymer, PEG/PPG-50/40 Copolymer, PEG/PPG-150/35 Copolymer, PEG/PPG-160/30 Copolymer, PEG/PPG-190/60 Copolymer, PEG/PPG-200/40 Copolymer, PEG/PPG-300/55 Copolymer, PEG/PPG-20/22 Methyl Ether Dimethicone, PEG-26-PPG-30 Phosphate, PEG/PPG-4/2 Propylheptyl Ether, PEG/PPG-6/2 Propylheptyl Ether, PEG-7/PPG-2 Propylheptyl Ether, PEG/PPG-8/2 Propylheptyl Ether, PEG/PPG- 10/2 Propylheptyl Ether, PEG/PPG-14/2 Propylheptyl Ether, PEG/PPG-40/2 Propylheptyl Ether, PEG/PPG-10/2 Ricinoleate, PEG/PPG-32/3 Ricinoleate, PEG-55 Propylene Glycol Oleate, PEG-25 Propylene Glycol Stearate, PEG-75 Propylene Glycol Stearate, PEG-120 Propylene Glycol Stearate, PEG-5 Rapeseed Sterol, PEG-10 Rapeseed Sterol, PEG-40 Ricinoleamide, PEG-75 Shea Butter Glycerides, PEG-75 Shorea Butter Glycerides, PEG-20 Sorbitan Cocoate, PEG-20 Sorbitan Isostearate, PEG-40 Sorbitan Lanolate, PEG-75 Sorbitan Lanolate, PEG-10 Sorbitan Laurate, PEG-40 Sorbitan Laurate, PEG-44 Sorbitan Laurate, PEG-75 Sorbitan Laurate, PEG-80 Sorbitan Laurate, PEG-20 Sorbitan Oleate, PEG-80 Sorbitan Palmitate, PEG-40 Sorbitan Stearate, PEG-60 Sorbitan Stearate, PEG-160 Sorbitan Triisostearate, PEG-40 Soy Sterol, PEG-2 Stearamide Carboxylic Acid, PEG-9 Stearamide Carboxylic Acid, PEG-20 Stearate, PEG-23 Stearate, PEG-25 Stearate, PEG-30 Stearate, PEG-32 Stearate, PEG-35 Stearate, PEG-36 Stearate, PEG-40 Stearate, PEG-45 Stearate, PEG-50 Stearate, PEG-55 Stearate, PEG-75 Stearate, PEG-90 Stearate, PEG-100 Stearate, PEG- 120 Stearate, PEG-150 Stearate, PEG-45 Stearate Phosphate, PEG-20 Tallate, PEG-50 Tallow Amide, PEG-2 Tallowamide DEA, PEG-20 Tallowate, PEG-66 Trihydroxystearin, PEG-200 Trihydroxystearin, PEG-60 Tsubakiate Glycerides, Pelargonic Acid, Pentadoxynol-200, Pheneth-6 Phosphate, Poloxamer 105, Poloxamer 108, Poloxamer 182, Poloxamer 183, Poloxamer 184, Poloxamer 188, Poloxamer 217, Poloxamer 234, Poloxamer 235, Poloxamer 237, Poloxamer 238, Poloxamer 288, Poloxamer 334, Poloxamer 335, Poloxamer 338, Poloxamine 908, Poloxamine 1508, Polydimethylsiloxy PEG/PPG-24/19 Butyl Ether Silsesquioxane, Polydimethylsiloxy PPG-13 Butyl Ether Silsesquioxane, Polyglyceryl-6 Caprate, Polyglyceryl-10 Dilaurate, Polyglyceryl-20 Heptacaprylate, Polyglyceryl-20 Hexacaprylate, Polyglyceryl-2 Lauryl Ether, Polyglyceryl-10 Lauryl Ether, Polyglyceryl-20 Octaisononanoate, Polyglyceryl-6 Pentacaprylate, Polyglyceryl-10 Pentacaprylate, Polyglyceryl-3 Polydimethylsiloxyethyl Dimethicone, Polyglyceryl-6 Tetracaprylate, Polyglyceryl-10 Tetralaurate, Polyglyceryl-6 Tricaprylate, Polyglyceryl-10 Trilaurate, Polyquaternium- 77, Polyquaternium-78, Polyquaternium-79, Polyquaternium-80, Polyquaternium-81, Polyquaternium- 82, Pomaderris Kumerahou Flow-er/Leaf Extract, Poria Cocos Extract, Potassium Abietoyl Hydrolyzed Collagen, Potassium Babassuate, Potassium Behenate, Potassium C9-15 Alkyl Phosphate, Potassium C11-15 Alkyl Phosphate, Potassium C12-13 Alkyl Phosphate, Potassium C12-14 Alkyl Phosphate, Potassium Caprate, Potassium Capryloyl Glutamate, Potassium Capryloyl Hydrolyzed Rice Protein, Potassium Castorate, Potassium Cocoate, Potassium Cocoyl Glutamate, Potassium Cocoyl Glycinate, Potassium Cocoyl Hydrolyzed Casein, Potassium Cocoyl Hydrolyzed Collagen, Potassium Cocoyl Hydrolyzed Corn Protein, Potassium Cocoyl Hydrolyzed Keratin, Potassium Cocoyl Hydrolyzed Oat Protein, Potassium Cocoyl Hydrolyzed Potato Protein, Potassium Cocoyl Hydrolyzed Rice Bran Protein, Potassium Cocoyl Hydrolyzed Rice Protein, Potassium Cocoyl Hydrolyzed Silk, Potassium Cocoyl Hydrolyzed Soy Protein, Potassium Cocoyl Hydrolyzed Wheat Protein, Potassium Cocoyl Hydrolyzed Yeast Protein, Potassium Cocoyl PCA, Potassium Cocoyl Sarcosinate, Potassium Cocoyl Taurate, Potassium Cornate, Potassium Cyclocarboxypropyloleate, Potassium Dihydroxyethyl Cocamine Oxide Phosphate, Potassium Dimethicone PEG-7 Phosphate, Potassium Dodecylbenzenesulfonate, Potassium Hempseedate, Potassium Hydrogenated Cocoate, Potassium Hydrogenated Palmate, Potassium Hydrogenated Tallowate, Potassium Hydroxystearate, Potassium Isostearate, Potassium Lanolate, Potassium Laurate, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Laureth Phosphate, Potassium Lauroyl Collagen Amino Acids, Potassium Lauroyl Glutamate, Potassium Lauroyl Hydrolyzed Collagen, Potassium Lauroyl Hydrolyzed Pea Protein, Potassium Lauroyl Hydrolyzed Soy Protein, Potassium Lauroyl PCA, Potassium Lauroyl Pea Amino Acids, Potassium Lauroyl Sarcosinate, Potassium Lauroyl Silk Amino Acids, Potassium Lauroyl Wheat Amino Acids, Potassium Lauryl Phosphate, Potassium Lauryl Sulfate, Potassium Linoleate, Potassium Metaphosphate, Potassium Methyl Cocoyl Taurate, Potassium Myristate, Potassium Myristoyl Glutamate, Potassium Myristoyl Hydrolyzed Collagen, Potassium Octoxynol-12 Phosphate, Potassium Oleate, Potassium Oleoyl Hydrolyzed Collagen, Potassium Olivate, Potassium Olivoyl Hydrolyzed Oat Protein, Potassium Olivoyl Hydrolyzed Wheat Protein, Potassium Olivoyl/Lauroyl Wheat Amino Acids, Potassium Olivoyl PCA, Potassium Palmate, Potassium Palmitate, Potassium Palmitoyl Hydrolyzed Corn Protein, Potassium Palmitoyl Hydrolyzed Oat Protein, Potassium Palmitoyl Hydrolyzed Rice Protein, Potassium Palmitoyl Hydrolyzed Sweet Almond Protein, Potassium Palmitoyl Hydrolyzed Wheat Protein, Potassium Palm Kernelate, Potassium Peanutate, Potassium Rapeseedate, Potassium Ricinoleate, Potassium Safflowerate, Potassium Soyate, Potassium Stearate, Potassium Stearoyl Hydrolyzed Collagen, Potassium Tallate, Potassium Tallowate, Potassium Taurate, Potassium Taurine Laurate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, Potassium Trideceth-6 Phosphate, Potassium Trideceth-7 Phosphate, Potassium Tsubakiate, Potassium Undecylenate, Potassium Undecylenoyl Hydrolyzed Collagen, Potassium Undecylenoyl Hydrolyzed Rice Protein, PPG-30- Buteth-30, PPG-36-Buteth-36, PPG-38-Buteth-37, PPG-30-Capryleth-4 Phosphate, PPG-10 Cetyl Ether Phosphate, PPG-2 C9-11 Pareth-8, PPG-1-Deceth-5, PPG-3-Deceth-2 Carboxylic Acid, PPG-30 Ethylhexeth-4 Phosphate, PPG-20-Glycereth-30, PPG-2 Hydroxyethyl Coco/lsostearamide, PPG-2- Isodeceth-8, PPG-2-Isodeceth-10, PPG-2-Isodeceth-18, PPG-2-Isodeceth-25, PPG-4-Isodeceth-10, Propyltrimonium Hydrolyzed Collagen, Quaternium-24, Quaternium-52, Quaternium-87, Rapeseed Acid, Rice Bran Acid, Rice Oil Glycereth-8 Esters, Ricinoleamidopropyl Betaine, Ricinoleic Acid, Ricinoleth-40, Safflower Acid, Sapindus Oahuensis Fruit Extract, Saponaria Offici-nalis Root Powder, Saponins, Sekken-K, Sekken-Na/K, Sekken Soji, Sekken Soji-K, Sesame Oil Glycereth-8 Esters, Sesamidopropylamine Oxide, Sesamidopropyl Betaine, Shea Butterami-dopropyl Betaine, Shea Butter Glycereth-8 Esters, Sodium Arachidate, Sodium Arganam-pohoacetate, Sodium Astrocaryum Murumuruate, Sodium Avocadoate, Sodium Babassuam-phoacetate, Sodium Babassuate, Sodium Babassu Sulfate, Sodium Behenate, Sodium Bisgly-col Ricinosulfosuccinate, Sodium Bis- Hydroxyethylglycinate Coco-Glucosides Crosspolymer, Sodium Bis-Hydroxyethylglycinate Lauryl- Glucosides Crosspolymer, Sodium Borageami-dopropyl PG-Dimonium Chloride Phosphate, Sodium Butoxynol-12 Sulfate, Sodium Butylglucosides Hydroxypropyl Phosphate, Sodium C13-17 Alkane Sulfonate, Sodium C14-18 Alkane Sulfonate, Sodium C12-15 Alkoxypropyl Iminodipropionate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium C9-22 Alkyl Sec Sulfonate, Sodium C14-17 Alkyl Sec Sulfonate, Sodium Caprate, Sodium Caproamphoacetate, Sodium Caproamphohydroxypropylsulfonate, Sodium Caproamphopropionate, Sodium Caproyl Methyltaurate, Sodium Caprylate, Sodium Capryleth-2 Carboxylate, Sodium Capryleth-9 Carboxylate, Sodium Capryloamphoacetate, Sodium Capryloamphohydroxypropylsulfonate, Sodium Capryloamphopropionate, Sodium Capryloyl Glutamate, Sodium Capryloyl Hydrolyzed Wheat Protein, Sodium Caprylyl PG-Sulfonate, Sodium Caprylyl Sulfonate, Sodium Castorate, Sodium Ceteareth-13 Carboxylate, Sodium Cetearyl Sulfate, Sodium Ceteth-13 Carboxylate, Sodium Cetyl Sulfate, Sodium Cocamidopropyl PG-Dimonium Chloride Phosphate, Sodium Co-caminopropionate, Sodium Coceth Sulfate, Sodium Coceth-30 Sulfate, Sodium Cocoabut-teramphoacetate, Sodium Cocoa Butterate, Sodium Cocoamphoacetate, Sodium Cocoam-phohydroxypropylsulfonate, Sodium Cocoamphopropionate, Sodium Cocoate, Sodium Co-co/Babassu/Andiroba Sulfate, Sodium Coco/Babassu Sulfate, Sodium Cocoglucosides Hydroxypropyl Phosphate, Sodium Cocoglucosides Hydroxypropylsulfonate, Sodium Coco-Glucoside Tartrate, Sodium Cocoglyceryl Ether Sulfonate, Sodium Coco/Hydrogenated Tallow Sulfate, Sodium Cocoiminodiacetate, Sodium Cocomonoglyceride Sulfate, Sodium Cocomonoglyceride Sulfonate, Sodium Coco PG-Dimonium Chloride Phosphate, Sodium Coco-Sulfate, Sodium Coco Sulfoacetate, Sodium Cocoyl Alaninate, Sodium Cocoyl Amino Acids, Sodium Cocoyl Collagen Amino Acids, Sodium Cocoyl Glutamate, Sodium Cocoyl Glutaminate, Sodium Cocoyl Glycinate, Sodium Cocoyl/Hydrogenated Tallow Glutamate, Sodium Cocoyl Hydrolyzed Collagen, Sodium Cocoyl Hydrolyzed Keratin, Sodium Cocoyl Hydrolyzed Rice Protein, Sodium Cocoyl Hydrolyzed Silk, Sodium Cocoyl Hydrolyzed Soy Protein, Sodium Cocoyl Hydrolyzed Sweet Almond Protein, Sodium Cocoyl Hydrolyzed Wheat Protein, Sodium Cocoyl Hydrolyzed Wheat Protein Glutamate, Sodium Cocoyl Isethionate, Sodium Cocoyl Methylaminopropionate, Sodium Cocoyl Oat Amino Acids, Sodium Cocoyl/Palmoyl/Sunfloweroyl Glutamate, Sodium Cocoyl Proline, Sodium Cocoyl Sarcosinate, Sodium Cocoyl Taurate, Sodium Cocoyl Threoninate, Sodium Cocoyl Wheat Amino Acids, Sodium C12-14 Olefin Sulfonate, Sodium C14-16 Olefin Sulfonate, Sodium C14- 18 Olefin Sulfonate, Sodium C16-18 Olefin Sulfonate, Sodium Cornamphopropionate, Sodium Cotton-seedamphoacetate, Sodium C13-15 Pareth-8 Butyl Phosphate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C14-15 Pareth-8 Carboxylate, Sodium C12-14 Sec-Pareth-8 Carboxylate, Sodium C14-15 Pareth-PG Sulfonate, Sodium C12-13 Pareth-2 Phosphate, Sodium C13-15 Pareth-8 Phosphate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium C12-15 Pareth-3 Sulfonate, Sodium C12-15 Pareth-7 Sulfonate, Sodium C12-15 Pareth-15 Sulfonate, Sodium Deceth-2 Carboxylate, Sodium Deceth Sulfate, Sodium Decylbenzenesulfonate, Sodium Decylglucosides Hydroxypropyl Phosphate, Sodium Decylglucosides Hydroxypropylsulfonate, Sodium Dilaureth-7 Citrate, Sodium Dilaureth-10 Phosphate, Sodium Dilinoleamidopropyl PG-Dimonium Chloride Phosphate, Sodium Dilinoleate, Sodium Dioleth-8 Phosphate, Sodium Dodecylbenzenesulfonate, Sodium Ethyl 2- Sulfolaurate, Sodium Glyceryl Oleate Phosphate, Sodium Grapeseedamidopropyl PG-Dimonium Chloride Phosphate, Sodium Grapeseedamphoacetate, Sodium Grapeseedate, Sodium Hempseedamphoacetate, Sodium Hexeth-4 Carboxylate, Sodium Hydrogenated Cocoate, Sodium Hydrogenated Cocoyl Methyl Isethionate, Sodium Hydrogenated Palmate, Sodium Hydrogenated Tallowate, Sodium Hydrogenated Tallowoyl Glutamate, Sodium Hydroxylau-ryldimonium Ethyl Phosphate, Sodium Hydroxypropyl Palm Kernelate Sulfonate, Sodium Hydroxypropylphosphate Decylglucoside Crosspolymer, Sodium Hydroxypropylphosphate Laurylglucoside Crosspolymer, Sodium Hydroxypropylsulfonate Cocoglucoside Crosspolymer, Sodium Hydroxypropylsulfonate Decylglucoside Crosspolymer, Sodium Hydroxypropylsulfonate Laurylglucoside Crosspolymer, Sodium Hydroxystearate, Sodium Isostearate, Sodium Isosteareth-6 Carboxylate, Sodium Isosteareth- 11 Carboxylate, Sodium Isostearoampho-acetate, Sodium Isostearoamphopropionate, Sodium N- Isostearoyl Methyltaurate, Sodium Laneth Sulfate, Sodium Lanolate, Sodium Lardate, Sodium Lauramido Diacetate, Sodium Lauraminopropionate, Sodium Laurate, Sodium Laureth-3 Carboxylate, Sodium Laureth-4 Carboxylate, Sodium Laureth-5 Carboxylate, Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Laureth Sulfate, Sodium Laureth-5 Sulfate, Sodium Laureth- 7 Sulfate, Sodium Laureth-8 Sulfate, Sodium Laureth-12 Sulfate, Sodium Laureth-40 Sulfate, Sodium Laureth-7 Tartrate, Sodium Lauriminodipropionate, Sodium Lauroamphoace-tate, Sodium Lauroamphohydroxypropylsulfonate, Sodium Lauroampho PG-Acetate Phosphate, Sodium Lauroamphopropionate, Sodium Lauroyl Aspartate, Sodium Lauroyl Collagen Amino Acids, Sodium Lauroyl Glycine Propionate, Sodium Lauroyl Hydrolyzed Collagen, Sodium Lauroyl Hydrolyzed Silk, Sodium Lauroyl Hydroxypropyl Sulfonate, Sodium Lauroyl Isethionate, Sodium Lauroyl Methylaminopropionate, Sodium Lauroyl Methyl Isethionate, Sodium Lauroyl Millet Amino Acids, Sodium Lauroyl/Myristoyl Aspartate, Sodium Lauroyl Oat Amino Acids, Sodium Lauroyl Sarcosinate, Sodium Lauroyl Silk Amino Acids, Sodium Lauroyl Taurate, Sodium Lauroyl Wheat Amino Acids, Sodium Lauryl Diethylenediaminoglycinate, Sodium Lauryl Glucose Carboxylate, Sodium Laurylglucosides Hydroxypropyl Phosphate, Sodium Laurylglucosides Hydroxypropylsulfonate, Sodium Lauryl Glycol Carboxylate, Sodium Lauryl Hydroxyacetamide Sulfate, Sodium Lauryl Phosphate, Sodium Lauryl Sulfate, Sodium Lauryl Sulfoacetate, Sodium Linoleate, Sodium Macadamiaseedate, Sodium Mangoampho-acetate, Sodium Mangoseedate, Sodium/MEA Laureth-2 Sulfosuccinate, Sodium Methoxy PPG-2 Acetate, Sodium Methyl Cocoyl Taurate, Sodium Methyl Lauroyl Taurate, Sodium Methyl Myristoyl Taurate, Sodium Methyl Oleoyl Taurate, Sodium Methyl Palmitoyl Taurate, Sodium Methyl Stearoyl Taurate, Sodium Methyl 2-Sulfolaurate, Sodium Methyl 2- Sulfopalmi-tate, Sodium Methyltaurate Isopalmitamide, Sodium Methyltaurine Cocoyl Methyltaurate, Sodium Myreth Sulfate, Sodium Myristate, Sodium Myristoamphoacetate, Sodium Myristoyl Glutamate, Sodium Myristoyl Hydrolyzed Collagen, Sodium Myristoyl Isethionate, Sodium Myristoyl Sarcosinate, Sodium Myristyl Sulfate, Sodium Nonoxynol-6 Phosphate, Sodium Nonoxynol-9 Phosphate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Ethane Sulfonate, Sodium Octoxynol-2 Sulfate, Sodium Octoxynol-6 Sulfate, Sodium Octoxynol-9 Sulfate, Sodium Oleate, Sodium Oleoamphoacetate, Sodium Oleoamphohydroxypropylsulfonate, Sodium Oleoamphopropionate, Sodium Oleoyl Hydrolyzed Collagen, Sodium Oleoyl Isethionate, Sodium Oleth Sulfate, Sodium Oleyl Methyl Isethionate, Sodium Oleyl Sulfate, Sodium Olivam-phoacetate, Sodium Olivate, Sodium Olivoyl Glutamate, Sodium Palmamphoacetate, Sodium Palmate, Sodium Palm Glyceride Sulfonate, Sodium Palmitate, Sodium Palmitoyl Hydrolyzed Collagen, Sodium Palmitoyl Hydrolyzed Wheat Protein, Sodium Palmitoyl Sarcosinate, Sodium Palm Kernelate, Sodium Palm Kerneloyl Isethionate, Sodium Palmoyl Glutamate, Sodium Passiflora Edulis Seedate, Sodium Peanutamphoacetate, Sodium Peanutate, Sodium PEG-6 Cocamide Carboxylate, Sodium PEG-8 Cocamide Carboxylate, Sodium PEG-4 Cocamide Sulfate, Sodium PEG-3 Lauramide Carboxylate, Sodium PEG-4 Lauramide Carboxylate, Sodium PEG-8 Palm Glycerides Carboxylate, Sodium Pentaerythrityl Hydroxypropyl Iminodiacetate Dendrimer, Sodium Propoxy PPG-2 Acetate, Sodium Rapeseedate, Sodium Ricebranampho-acetate, Sodium Ricinoleate, Sodium Ricinoleoamphoacetate, Sodium Rose Hipsamphoace-tate, Sodium Rosinate, Sodium Safflowerate, Sodium Saffloweroyl Hydrolyzed Soy Protein, Sodium Sesameseedate, Sodium Sesamphoacetate, Sodium Sheabutteramphoacetate, Sodium Soyate, Sodium Soy Hydrolyzed Collagen, Sodium Stearate, Sodium Stearoamphoacetate, Sodium Stearoamphohydroxypropylsulfonate, Sodium Stearoamphopropionate, Sodium Stearoyl Casein, Sodium Stearoyl Glutamate, Sodium Stearoyl Hyaluronate, Sodium Stearoyl Hydrolyzed Collagen, Sodium Stearoyl Hydrolyzed Corn Protein, Sodium Stearoyl Hydrolyzed Silk, Sodium Stearoyl Hydrolyzed Soy Protein, Sodium Stearoyl Hydrolyzed Wheat Protein, Sodium Stearoyl Lactalbumin, Sodium Stearoyl Methyl Isethionate, Sodium Stearoyl Oat Protein, Sodium Stearoyl Pea Protein, Sodium Stearoyl Soy Protein, Sodium Stearyl Dimethyl Glycine, Sodium Stearyl Sulfate, Sodium Sunflowerseedamphoacetate, Sodium Surfactin, Sodium Sweetalmondamphoacetate, Sodium Sweet Almondate, Sodium Tallamphopropionate, Sodium Tallate, Sodium Tallowamphoacetate, Sodium Tallowate, Sodium Tallow Sulfate, Sodium Tamanuseedate, Sodium Taurate, Sodium Taurine Cocoyl Methyltaurate, Sodium Taurine Laurate, Sodium/TEA-Lauroyl Collagen Amino Acids, Sodium/TEA-Lauroyl Hydrolyzed Collagen, Sodium/TEA-Lauroyl Hydrolyzed Keratin, Sodium/TEA- Lauroyl Keratin Amino Acids, Sodium/TEA-Undecylenoyl Collagen Amino Acids, Sodium/TEA- Undecylenoyl Hydrolyzed Collagen, Sodium/TEA-Undecylenoyl Hydrolyzed Corn Protein, Sodium/TEA-Undecylenoyl Hydrolyzed Soy Protein, Sodium/TEA-Undecylenoyl Hydrolyzed Wheat Protein, Sodium Theobroma Grandiflorum Seedate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Trideceth Sulfate, Sodium Tridecylbenzene-sulfonate, Sodium Tridecyl Sulfate, Sodium Trimethylolpropane Hydroxypropyl Iminodiacetate Dendrimer, Sodium Undeceth-5 Carboxylate, Sodium Undecylenate, Sodium Undecylen-oamphoacetate, Sodium Undecylenoamphopropionate, Sodium Undecylenoyl Glutamate, Sodium Wheat Germamphoacetate, Sorbeth-160 Tristearate, Soy Acid, Soyamidopropylamine Oxide, Soyamidopropyl Betaine, Soybean Oil Glycereth-8 Esters, Stearamidopropylamine Oxide, Stearamidopropyl Betaine, Stearamine Oxide, Steareth-15, Steareth-16, Steareth-20, Steareth-21, Steareth-25, Steareth-27, Steareth-30, Steareth- 40, Steareth-50, Steareth-80, Steareth-100, Steareth-2 Phosphate, Steareth-3 Phosphate, Stearic Acid, Stearoxypropyltri-monium Chloride, Stearoyl Glutamic Acid, Stearoyl Sarcosine, Stearyl Betaine, Stearyldimoniumhydroxypropyl Butylglucosides Chloride, Stearyldimoniumhydroxypropyl Decylglucosides Chloride, Stearyldimoniumhydroxypropyl Laurylglucosides Chloride, Sulfated Castor Oil, Sulfated Coconut Oil, Sulfated Glyceryl Oleate, Sulfated Olive Oil, Sulfated Peanut Oil, Sunflow-eramide MEA, Sunflower Seed Acid, Sunflowerseedamidopropyl Hydroxyethyldimonium Chloride, Sunflower Seed Oil Glycereth-8 Esters, Tall Oil Acid, Tallow Acid, Tallowamidoprop-ylamine Oxide, Tallowamidopropyl Betaine, Tallowamidopropyl Hydroxysultaine, Tallowamine Oxide, Tallow Betaine, Tallow Dihydroxyethyl Betaine, Tallowoyl Ethyl Glucoside, TEA-Abietoyl Hydrolyzed Collagen, TEA-C12-14 Alkyl Phosphate, TEA-C10-15 Alkyl Sulfate, TEA-C11-15 Alkyl Sulfate, TEA- C12-13 Alkyl Sulfate, TEA-C12-14 Alkyl Sulfate, TEA-C12-15 Alkyl Sulfate, TEA C14-17 Alkyl Sec Sulfonate, TEA-Canolate, TEA-Cocamide Diacetate, TEA-Cocoate, TEA-Coco-Sulfate, TEA-Cocoyl Alaninate, TEA-Cocoyl Glutamate, TEA-Cocoyl Glutaminate, TEA-Cocoyl Glycinate, TEA-Cocoyl Hydrolyzed Collagen, TEA-Cocoyl Hydrolyzed Soy Protein, TEA-Cocoyl Sarcosinate, TEA- Dimethicone PEG-7 Phosphate, TEA-Dodecylbenzenesulfonate, TEA-Hydrogenated Cocoate, TEA- Hydrogenated Tallowoyl Glutamate, TEA-Isostearate, TEA-Isostearoyl Hydrolyzed Collagen, TEA- Lauraminopropionate, TEA-Laurate, TEA-Laurate/Myristate, TEA-Laureth Sulfate, TEA-Lauroyl Collagen Amino Acids, TEA-Lauroyl Glutamate, TEA-Lauroyl Hydrolyzed Collagen, TEA-Lauroyl Keratin Amino Acids, TEA-Lauroyl Methylaminopropionate, TEA-Lauroyl/Myristoyl Aspartate, TEA- Lauroyl Sarcosinate, TEA-Lauryl Phosphate, TEA-Lauryl Sulfate, TEA-Myristaminopropionate, TEA- Myristate, TEA-Myristoyl Hydrolyzed Collagen, TEA-Oleate, TEA-Oleoyl Hydrolyzed Collagen, TEA- Oleoyl Sarcosinate, TEA-Oleyl Sulfate, TEA-Palmitate, TEA-Palm Kernel Sarcosinate, TEA-PEG-3 Cocamide Sulfate, TEA-Rosinate, TEA-Stearate, TEA-Tallate, TEA-T ridecylbenzenesulfonate, TEA- Undecylenate, TEA-Undecylenoyl Hydrolyzed Collagen, Tetramethyl Decynediol, Tetrasodium Dicarboxyethyl Stearyl Sulfosuccinamate, TIPA-Laureth Sulfate, TIPA-Lauryl Sulfate, TIPA-Myristate, TIPA-Stearate, Tocopheryl Phosphate, Trehalose Undecylenoate, TM-C12-15 Pareth-2 Phosphate, TM-C12-15 Pareth-6 Phosphate, TM-C12-15 Pareth-8 Phosphate, TM-C12-15 Pareth-10 Phosphate, Trideceth-20, Trideceth-50, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid, Trideceth-10 Phosphate, Tridecylbenzenesulfonic Acid, Trilau-reth-9 Citrate, Trimethylolpropane Hydroxypropyl Bis-Hydroxyethylamine Dendrimer, Trisodium Lauroampho PG-Acetate Chloride Phosphate, Undecanoic Acid, Undeceth-5 Carboxylic Acid, Undecylenamidopropylamine Oxide, Undecylenamidopropyl Betaine, Undecylenic Acid, Undecylenoyl Collagen Amino Acids, Undecylenoyl Glycine, Undecylenoyl Hydrolyzed Collagen, Undecylenoyl Wheat Amino Acids, Undecyl Glucoside, Wheat Germ Acid, Wheat Ger-mamidopropylamine Oxide, Wheat Germamidopropyl Betaine, Yucca Schidigera Leaf/Root/Stem Extract, Yucca Schidigera Stem Extract, Zinc Coceth Sulfatea and Zinc Coco-Sulfate.

The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C ₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etC) and/or aliphatic or naphthenic hydrocarbons, such as, for example, squalane, squalene or dialkylcy-clohexanes.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

**Sorbitan esters.** Suitable sorbitan esters are sorbitan monoisostearate, sorbitan ses-quiisostearate, sorbitan diisostearate, sorbitan triisostearate, sorbitan monooleate, sorbitan sesquioleate, sorbitan dioleate, sorbitan trioleate, sorbitan monoerucate, sorbitan sesquieru-cate, sorbitan dierucate, sorbitan trierucate, sorbitan monoricinoleate, sorbitan sesquiricino-leate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomale-ate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

**Polyglycerol esters.** Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

**Amphoteric emulsifiers.** Other suitable emulsifiers are amphboteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example coco-acylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl ami-nopropionate and C_{12/18} acyl sarcosine.

### Superfatting agents and consistency factors

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents and rheology additives

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally deri-vatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicones can be chosen from the group consisting of: Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodi-methicone, Bis(C13-15 Alkoxy) PG- Amodimethicone, Bis-(C1-8 Alkyl Lauroyl Lysine Decylcar-boxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis- Hydroxyethox-ypropyl Dimethicone Bis-Hydroxylauryl, Dimethicone/IPDI Copolymer, Bis- Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-isobutyl PEG-15/Amodimethicone Copolymer, Bis-Isobutyl PEG/PPG-20/35/Amodimethicone Copolymer, Bis- Isobutyl PEG/PPG-10/7/Dimethicone Copolymer, Bis-isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispol-yethylene Dimethicone, Bis- (Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Unde-ceneth-21) Dimethicone, Bis-Stearyl Dimethicone, Bis-Trimethoxysilylethyl Tetramethyl-disiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Per-fluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethox-ymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone?/inyl Dimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol Illipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccin-amide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hex-yltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, lsopolyglyceryl-3 Dimethicone, lsopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG- 10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methox-ycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG- 8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spiruli-nate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Poly-phenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethi-cone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxy-propyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycapry-lylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Tri-methoxycaprylylsilane, Trimethoxysilyl Dimethicone, Trimethyl Pentaphenyl Trisiloxane, Tri-methylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane and Zinc Dimethicone PEG-8 Succinate and mixtures thereof.

More preferably the silicones to be contained in the mixture according to the inventions are Dimethicone, Cyclomethicone, Phenyl Trimethicone, Cyclohexasiloxane and Cyclopentasiloxane.

A detailed overview of suitable volatile silicones can be found in Todd et al. in Cosm. Toil. 91, 27 (1976**).**

### Waxes and stabilisers

Besides natural oils used, waxes may also be present in the preparations, more especially natural waxes such as, for example, candelilla wax, carnauba wax, Japan wax, espar-tograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### Primary sun protection filters

Primary sun protection filters in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivativesand indole derivatives.

In addition, it is advantageous to combine compounds of formula (I) with active ingredients which penetrate into the skin and protect the skin cells from inside against sunlight-induced damage and reduce the level of cutaneous matrix metalloproteases. Preferred respective ingredients, so called aryl hydrocarbon receptor antagonists, are described in WO 2007/128723, Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan^{®}HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150).

Broadband filters which are preferably combined with one or more compounds of formula (I) in a preparation according to the present invention are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine.

The compositions can comprise further typical detergent and cleansing composition ingredients such as UV-A filters which are preferably combined with one or more compounds of formula (I) in a preparation according to the present invention are selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds in accordance with DE 100 55 940 A1 (= WO 2002 038537 A1)

The compositions can comprise further typical detergent and cleansing composition ingredients such as UV filters which are more preferably combined with one or more compounds of formula (I) in a preparation according to the present invention are selected from the group consisting of
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCl name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds in accordance with DE 100 55 940 (= WO 02/38537).

Advantageous primary and also secondary sun protection factors are mentioned in WO 2005 123101 A1**.** Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) so that the formulation according to the invention has a light protection factor of greater than or equal to 2 (preferably greater than or equal to 5). Such formulations according to the invention are particularly suitable for protecting the skin and hair.

### Secondary sun protection filters

Besides the groups of primary sun protection filters mentioned above, secondary sun protection filters of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglu-cose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodi-propionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiq-uinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1**.** The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### Actives modulating skin and/or hair pigmentation

Preferred active ingredients for skin and/or hair lightening are selected from the group consisting of: kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates (preferably one or more cyclohexyl carbamates disclosed in WO 2010/122178 and WO 2010/097480), sulfurcontaining molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract .

Preferred skin lighteners as component (b) are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-lle- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases") as described for example in EP 0 584 178, tetrasubstituted cyclohexene deriva-tives as described for example in WO 2005/032501 , isoprenoids as described in WO 2005/102252 and in WO 2006/010661 , melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, microalgae extracts, in particular Isochrysis galbana, trehalose, erythru-lose and dihydroxyacetone. Flavonoids which bring about skin and hair tinting or brown-ing (e.g. quercetin, rhamnetin, kaempferol, fisetin, genistein, daidzein, chrysin and api-genin, epicatechin, diosmin and diosmetin, morin, quercitrin, naringenin, hesperidin, phloridzin and phloretin) can also be used.

The amount of the aforementioned examples of additional active ingredients for the modulation of skin and hair pigmentation (one or more compounds) in the products according to the invention is then preferably 0.00001 to 30 wt.%, preferably 0.0001 to 20 wt.%, particularly preferably 0.001 to 5 wt.%, based on the total weight of the preparation.

### Anti-ageing actives

In the context of the invention, anti-ageing or biogenic agents are, for example antioxidants, matrix-metalloproteinase inhibitors (MMPI), skin moisturizing agents, glycosaminglycan stimulkators, anti-inflammatory agents, TRPV1 antagonists and plant extracts.

**Antioxidants.** Suitable antioxidants encompass amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysylthreonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.

If vitamin E and/or derivatives thereof are used as the antioxidant(s), it is advantageous to choose their concentrations from the range from about 0.001 to about 10 % b.w. based on the total weight of the formulation. If vitamin A or vitamin A derivatives or carotenes or derivatives thereof are used as the antioxidant(s), it is advantageous to choose their concentrations from the range from about 0.001 to aout 10 % b.w. based on the total weight of the formulation.

**Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCl: hydrolysed rice protein), oenotherol (company Soliance; INCl: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCl: glycosaminoglycans), fermiskin (company Silab/Mawi; INCl: water and lentinus edodes extract), actimp 1.9.3 (company Expanscience/Rahn; INCl: hydrolysed lupine protein), lipobelle soyaglycone (company Mibelle; INCl: alcohol, polysorbate 80, lecithin and soy isoflavones), extracts from green and black tea and further plant extracts, which are listed in WO 02 069992 A1 (see tables 1-12 there, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1**,** as e.g. SymMatrix (company Symrise, INCl: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators.** Preferred compositions comprise substances stimulating the synthesis of glycosaminoglycans selected from the group consisting of hyaluronic acid and derivatives or salts, Subliskin (Sederma, INCl: Sinorhizobium Meliloti Ferment Filtrate, Cetyl Hydroxyethylcellulose, Lecithin), Hyalufix (BASF, INCl: Water, Butylene Glycol, Alpinia galanga leaf extract, Xanthan Gum, Caprylic/Capric Triglyceride), Stimulhyal (Soliance, INCl: Calcium ketogluconate), Syn-Glycan (DSM, INCl: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCl: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1**,** or indirect modulators of TRPV1 by an activation of the µ-receptor, e.g. acetyl tetrapeptide-15, are preferred.

**Desquamating agents.** The compositions may also contain desquamating agents (component b5) in amounts of about 0.1 to about 30 % b.w. preferably about 0.5 to about 15 % b.w., particularly preferably about 1 to about 10 % b.w. based on the total weight of the preparation. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine^{®}, prickly pear extracts such as those marketed under the name Exfolactive^{®} by the company SILAB, or Phytosphingosine SLC^{®} (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, those described in the **documents** FR 2570377 A1**,** EP 0199636 A1**,** EP 0325540 A1**,** EP 0402072 A1**,** chestnut or prickly pear extracts, in particular marketed by SILAB; reducing compounds such as cysteine or cysteine precursors.

Desquamating agents which can be used are also nicotinic acid and its esters and nicotinamide, also called vitamin B3 or vitamin PP, and ascorbic acid and its precursors, as described in particular in application EP 1529522 A1**.**

**Anti-cellulite agents.** Anti-cellulite agents and lipolytic agents are preferably selected from the group consisting of those described in WO 2007/077541, and betaadrenergic receptor agonists such as synephrine and its derivatives, and cyclohexyl carbamates described in WO 2010/097479**.** Agents enhancing or boosting the activity of anti-cellulite agents, in particular agents which stimulate and/or depolarise C nerve fibres, are preferably selected from the group consisting of capsaicin and derivatives thereof, vanillylnonylamid and derivatives thereof, L-carnitine, coenzym A, isoflavonoides, soy extracts, ananas extract and conjugated linoleic acid.

**Fat enhancing agents.** Formulations and products according to the present invention may also comprise one or more fat enhancing and/or adipogenic agents as well as agents enhancing or boosting the activity of fat enhancing agents. A fat enhancing agent is for example hydroxymethoxyphenyl propylmethylmethoxybenzofuran (trade name: Sym3D^{®}).

### Hair growth activators or inhibitors

Formulations and products according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, formulations and products according to the present invention may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### Physiological cooling agents

The compositions may also contain one or more substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (I-menthoxy)-1,2-propandiol, (I-menthoxy)-2-methyl-1,2-propandiol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, monomenthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], as described in US 4,150,052**,** menthanecarboxylic acid-N-(4-cyanophenyl)amide or menthanecarboxylic acid-N-(4-cyanomethylphenyl)amide as described in WO 2005 049553 A1**,** menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates (preferably those described in EP 2033688 A2**)** and [(1R,2S,5R)-2-isopropyl-5-methyl-cyclohexyl] 2-(ethylamino)-2-oxo-acetate (X Cool).

### Anti-inflammatory agents

The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1**,** are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenan-thramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1**),** boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and â-glucans, in particular 1,3-1,4-â-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO2), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

### Anti-microbial agents

Suitable anti-microbial agents are, in principle, all substances effective against Grampositive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide. Particularly preferred is 4-hydroxyacetophenone.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odourneutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinC Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®}, Propandiol caprylate (Crinipan^{®} PMC) (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### Carriers and hydrotropes

Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive"). Particulatly preferred is 4-hydroxyacetophenone.

### Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, ▪ -isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### DYES

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (CI. 16255), patent blue V (CI. 42051), indigotin (CI. 73015), chlorophyllin (CI. 75810), quinoline yellow (CI. 47005), titanium dioxide (CI. 77891), indanthrene blue RS (CI. 69800) and madder lake (CI. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

### Preparations

Preferred compositions according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etC), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or postfoaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary directdyeing hair dye, semi-permanent hair dye, permanent hair dye, hair conditioner, hair mousse, eye care product, make-up, make-up remover or baby product.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation.

### DETERGENT COMPOSITIONS

Another object of the present invention refers to a detergent composition comprising said 1,2-pentanediol of improved quality Suitable examples for detergents encompass heavy duty powder detergents, heavy duty liquid detergents, light duty powder detergents, light duty liquid detergents, fabric softeners, manual dish wash agents, allpurpose cleaners and the like.

The detergent compositions according to the present invention may comprise any of the ingredients customarily found in such compositions, such as, for example, anionic, nonionic, cationic, amphoteric or zwitterionic (co-)surfactants, organic solvents, builders, enzymes and additional auxiliaries such as soil repellents, thickeners, colorants and fragrances or the like. Suitable compounds have already been cited infra, however the following ones are particularly preferred:

### Anionic surfactants

Preferably, surfactants of the sulfonate type, alk(en)yl sulfonates, alkoxylated alk(en)yl sulfates, ester sulfonates and/or soaps are used as the anionic surfactants. Suitable surfactants of the sulfonate type are advantageously C₉₋₁₃ alkylbenzene sulfonates, olefin sulfonates, i.e. mixtures of alkene- and hydroxyalkane sulfonates, and disulfonates, as are obtained, for example, by the sulfonation with gaseous sulfur trioxide of C₁₂₋₁₈ monoolefins having a terminal or internal double bond and subsequent alkaline or acidic hydrolysis of the sulfonation products.

**Alk(en)yl sulfates.** Preferred alk(en)yl sulfates are the alkali and especially the sodium salts of the sulfuric acid half-esters of the C₁₂-C₁₈ fatty alcohols, for example, from coconut butter alcohol, tallow alcohol, lauryl, myristyl, cetyl or stearyl alcohol or from C₈-C₂₀ oxo alcohols and those half-esters of secondary alcohols of these chain lengths. Alk(en)yl sulfates of the cited chain lengths that comprise a synthetic straight chain alkyl group manufactured petrochemically are also preferred. The C₁₂-C₁₆ alkyl sulfates and C₁₂-C₁₅ alkyl sulfates as well as C₁₄-C₁₅ alkyl sulfates and C₁₄-C₁₆ alkyl sulfates are particularly preferred on the grounds of laundry performance. The 2,3-alkyl sulfates, which can be obtained from Shell Oil Company under the trade name DAN^{™}, are also suitable anionic surfactants.

**Alk(en)yl ether sulfates.** Sulfuric acid mono-esters derived from straight-chained or branched C₇-C₂₁ alcohols ethoxylated with 1 to 6 moles ethylene oxide are also suitable, such as 2-methyl-branched C₉-C₁₁ alcohols with an average of 3.5 mol ethylene oxide (EO) or C₁₂-C₁₈ fatty alcohols with 1 to 4 EO.

**Ester sulfonates.** The esters of alpha-sulfo fatty acids (ester sulfonates), e.g., the alpha-sulfonated methyl esters of hydrogenated coco-, palm nut- or tallow acids are likewise suitable.

**Soaps.** Soaps, in particular, can be considered as further anionic surfactants. Saturated fatty acid soaps are particularly suitable, such as the salts of lauric acid, myristic acid, palmitic acid, stearic acid, hydrogenated erucic acid and behenic acid, and especially soap mixtures derived from natural fatty acids such as coconut oil fatty acid, palm kernel oil fatty acid or tallow fatty acid. Those soap mixtures are particularly preferred that are composed of 50 to 100 wt. % of saturated C₁₂-C₂₄ fatty acid soaps and 0 to 50 wt. % of oleic acid soap.

**Ether carboxylic acids.** A further class of anionic surfactants is that of the ether carboxylic acids, obtainable by treating fatty alcohol ethoxylates with sodium chloroacetate in the presence of basic catalysts. They have the general formula: RO(CH₂CH₂O)ₚCH₂COOH with R = C₁-C₁₈ and p = 0.1 to 20. Ether carboxylic acids are insensitive to water hardness and possess excellent surfactant properties.

### Non-ionic surfactants

**Alkohol alkoxylates.** The added nonionic surfactants are preferably alkoxylated and/or propoxylated, particularly primary alcohols having preferably 8 to 18 carbon atoms and an average of 1 to 12 mol ethylene oxide (EO) and/or 1 to 10 mol propylene oxide (PO) per mol alcohol. C₈-C₁₆-Alcohol alkoxylates, advantageously ethoxylated and/or propoxylated C₁₀-C₁₅-alcohol alkoxylates, particularly C₁₂-C₁₄ alcohol alkoxylates, with an ethoxylation degree between 2 and 10, preferably between 3 and 8, and/or a propoxylation degree between 1 and 6, preferably between 1.5 and 5, are particularly preferred. The cited degrees of ethoxylation and propoxylation constitute statistical average values that can be a whole or a fractional number for a specific product. Preferred alcohol ethoxylates and propoxylates have a narrowed homolog distribution (narrow range ethoxylates/propoxylates, NRE/NRP). In addition to these nonionic surfactants, fatty alcohols with more than 12 EO can also be used. Examples of these are (tallow) fatty alcohols with 14 EO, 16 EO, 20 EO, 25 EO, 30 EO or 40 EO.

**Alkylglycosides (APG^{®}).** Furthermore, as additional nonionic surfactants, alkyl glycosides that satisfy the general Formula RO(G)ₓ, can be added, e.g., as compounds, particularly with anionic surfactants, in which R means a primary linear or methyl-branched, particularly 2-methyl-branched, aliphatic group containing 8 to 22, preferably 12 to 18 carbon atoms and G stands for a glycose unit containing 5 or 6 carbon atoms, preferably for glucose. The degree of oligomerization x, which defines the distribution of monoglycosides and oligoglycosides, is any number between 1 and 10, preferably between 1.1 and 1.4.

**Fatty acid ester alkoxylates.** Another class of preferred nonionic surfactants, which are used either as the sole nonionic surfactant or in combination with other nonionic surfactants, in particular, together with alkoxylated fatty alcohols and/or alkyl glycosides, are alkoxylated, preferably ethoxylated or ethoxylated and propoxylated fatty acid alkyl esters preferably containing 1 to 4 carbon atoms in the alkyl chain, more particularly the fatty acid methyl esters which are described, for example, in Japanese Patent Application JP-A-58/217598 or which are preferably produced by the process described in International Patent Application WO-A-90/13533. Methyl esters of C₁₂-C₁₈ fatty acids containing an average of 3 to 15 EO, particularly containing an average of 5 to 12 EO, are particularly preferred.

**Amine oxides.** Nonionic surfactants of the amine oxide type, for example, N-coco alkyl-N,N-dimethylamine oxide and N-tallow alkyl-N,N-dihydroxyethylamine oxide, and the fatty acid alkanolamides may also be suitable. The quantity in which these nonionic surfactants are used is preferably no more than the quantity in which the ethoxylated fatty alcohols are used and, particularly no more than half that quantity.

**Gemini surfactants.** The so-called gemini surfactants can be considered as further surfactants. Generally speaking, such compounds are understood to mean compounds that have two hydrophilic groups and two hydrophobic groups per molecule. As a rule, these groups are separated from one another by a "spacer". The spacer is usually a hydrocarbon chain that is intended to be long enough such that the hydrophilic groups are a sufficient distance apart to be able to act independently of one another. These types of surfactants are generally characterized by an unusually low critical micelle concentration and the ability to strongly reduce the surface tension of water. In exceptional cases, however, not only dimeric but also trimeric surfactants are meant by the term gemini surfactants. Suitable gemini surfactants are, for example, sulfated hydroxy mixed ethers according to German Patent Application DE 4321022 A1 or dimer alcohol bis- and trimer alcohol tris sulfates and ether sulfates according to International Patent Application WO 96/23768 A1. Blocked end group dimeric and trimeric mixed ethers according to German Patent Application DE 19513391 A1 are especially characterized by their bifunctionality and multifunctionality. Gemini polyhydroxyfatty acid amides or polyhydroxyfatty acid amides, such as those described in International Patent Applications WO 95/19953 A1, WO 95/19954 A1 and WO 95/19955 A1 can also be used.

### Cationic surfactants

**Tetraalkyl ammonium salts.** Cationically active surfactants comprise the hydrophobic high molecular group required for the surface activity in the cation by dissociation in aqueous solution. A group of important representatives of the cationic surfactants are the tetraalkyl ammonium salts of the general formula: (R¹R²R³R⁴N⁺) X⁻. Here R1 stands for C₁-C₈ alk(en)yl, R², R³ and R⁴, independently of each other, for alk(en)yl radicals having 1 to 22 carbon atoms. X is a counter ion, preferably selected from the group of the halides, alkyl sulfates and alkyl carbonates. Cationic surfactants, in which the nitrogen group is substituted with two long acyl groups and two short alk(en)yl groups, are particularly preferred.

**Esterquats.** A further class of cationic surfactants particularly useful as cosurfactants for the present invention is represented by the so-called esterquats. Esterquats are generally understood to be quaternised fatty acid triethanolamine ester salts. These are known compounds which can be obtained by the relevant methods of preparative organic chemistry. Reference is made in this connection to International patent application WO 91/01295 A1, according to which triethanolamine is partly esterified with fatty acids in the presence of hypophosphorous acid, air is passed through the reaction mixture and the whole is then quaternised with dimethyl sulphate or ethylene oxide. In addition, German patent DE 4308794 C1 describes a process for the production of solid esterquats in which the quaternisation of triethanolamine esters is carried out in the presence of suitable dispersants, preferably fatty alcohols.

Typical examples of esterquats suitable for use in accordance with the invention are products of which the acyl component derives from monocarboxylic acids corresponding to formula RCOOH in which RCO is an acyl group containing 6 to 10 carbon atoms, and the amine component is triethanolamine (TEA). Examples of such monocarboxylic acids are caproic acid, caprylic acid, capric acid and technical mixtures thereof such as, for example, so-called head-fractionated fatty acid. Esterquats of which the acyl component derives from monocarboxylic acids containing 8 to 10 carbon atoms, are preferably used. Other esterquats are those of which the acyl component derives from dicarboxylic acids like malonic acid, succinic acid, maleic acid, fumaric acid, glutaric acid, sorbic acid, pimelic acid, azelaic acid, sebacic acid and/or dodecanedioic acid, but preferably adipic acid. Overall, esterquats of which the acyl component derives from mixtures of monocarboxylic acids containing 6 to 22 carbon atoms, and adipic acid are preferably used. The molar ratio of mono and dicarboxylic acids in the final esterquat may be in the range from 1:99 to 99:1 and is preferably in the range from 50:50 to 90:10 and more particularly in the range from 70:30 to 80:20. Besides the quaternised fatty acid triethanolamine ester salts, other suitable esterquats are quaternized ester salts of mono-/dicarboxylic acid mixtures with diethanolalkyamines or 1,2-dihydroxypropyl dialkylamines. The esterquats may be obtained both from fatty acids and from the corresponding triglycerides in admixture with the corresponding dicarboxylic acids. One such process, which is intended to be representative of the relevant prior art, is proposed in European patent EP 0750606 B1. To produce the quaternised esters, the mixtures of mono- and dicarboxylic acids and the triethanolamine - based on the available carboxyl functions - may be used in a molar ratio of 1.1:1 to 3:1. With the performance properties of the esterquats in mind, a ratio of 1.2:1 to 2.2:1 and preferably 1.5:1 to 1.9:1 has proved to be particularly advantageous. The preferred esterquats are technical mixtures of mono-, di- and triesters with an average degree of esterification of 1.5 to 1.9.

### Amphoteric or zwitterionic surfactants

**Betaines.** Amphoteric or ampholytic surfactants possess a plurality of functional groups that can ionize in aqueous solution and thereby--depending on the conditions of the medium--lend anionic or cationic character to the compounds (see DIN 53900, July 1972). Close to the isoelectric point (around pH 4), the amphoteric surfactants form inner salts, thus becoming poorly soluble or insoluble in water. Amphoteric surfactants are subdivided into ampholytes and betaines, the latter existing as zwitterions in solution. Ampholytes are amphoteric electrolytes, i.e. compounds that possess both acidic as well as basic hydrophilic groups and therefore behave as acids or as bases depending on the conditions. Especially betaines are known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid for example, is also possible. Examples of suitable betaines are the carboxy alkylation products of secondary and, in particular, tertiary amines which correspond to formula R¹R²R³N-(CH₂)_{q}COOX where R¹ is a an alkyl radical having 6 to 22 carbon atoms, R² is hydrogen or an alkyl group containing 1 to 4 carbon atoms, R³ is an alkyl group containing 1 to 4 carbon atoms, q is a number of 1 to 6 and X is an alkali and/or alkaline earth metal or ammonium. Typical examples are the carboxymethylation products of hexylmethylamine, hexyldimethylamine, octyldimethylamine, decyldimethylamine, C_{12/14}-cocoalkyldimethylamine, myristyldimethylamine, cetyldimethylamine, stearyldimethylamine, stearylethylmethylamine, oleyldimethylamine, C_{16/18}-tallowalkyldimethylamine and their technical mixtures, and particularly dodecyl methylamine, dodecyl dimethylamine, dodecyl ethylmethylamine and technical mixtures thereof.

**Alkylamido betaines.** Other suitable betaines are the carboxyalkylation products of amidoamines corresponding to formula R¹CO(R³)(R⁴)-NH-(CH₂)ₚ-N-(CH₂)_{q}COOX in which R¹CO is an aliphatic acyl radical having 6 to 22 carbon atoms and 0 or 1 to 3 double bonds, R² is hydrogen or an alkyl radical having 1 to 4 carbon atoms, R³ is an alkyl radical having 1 to 4 carbon atoms, p is a number from 1 to 6, q is a number from 1 to 3 and X is an alkali and/or alkaline earth metal or ammonium. Typical examples are reaction products of fatty acids having 6 to 22 carbon atoms, like for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine und N,N-diethylaminopropylamine, which are condensed with sodium chloroacetate. The commercially available products include Dehyton^{®} K and Dehyton^{®} PK (Cognis Deutschland GmbH & Co., KG) as well as Tego^{®} Betaine (Goldschmidt).

**Imidazolines.** Other suitable starting materials for the betaines to be used for the purposes of the invention are imidazolines. These substances are also known and may be obtained, for example, by cyclizing condensation of 1 or 2 moles of C₆⁻C₂₂ fatty acids with polyfunctional amines, such as for example aminoethyl ethanolamine (AEEA) or diethylenetriamine. The corresponding carboxyalkylation products are mixtures of different open-chain betaines. Typical examples are condensation products of the above- mentioned fatty acids with AEEA, preferably imidazolines based on lauric acid, which are subsequently betainised with sodium chloroacetate. The commercially available products include Dehyton^{®} G (Cognis Deutschland GmbH & Co., KG)

The amount of (co-)surfactant comprised in the inventive compositions is advantageously 0.1 wt. % to 90 wt. %, particularly 10 wt. % to 80 wt. % and particularly preferably 20 wt. % to 70 wt.-%.

### Organic solvents

Liquid light or heavy duty detergents may comprise organic solvents, preferably those miscible with water. Polydiols, ethers, alcohols, ketones, amides and/or esters are preferably used as the organic solvent for this in amounts of 0 to 90 wt. %, preferably 0.1 to 70 wt. %, particularly 0.1 to 60 wt. %. Low molecular weight polar substances, such as for example, methanol, ethanol, propylene carbonate, acetone, acetonylacetone, diacetone alcohol, ethyl acetate, 2-propanol, ethylene glycol, propylene glycol, glycerin, diethylene glycol, dipropylene glycol monomethyl ether and dimethylformamide or their mixtures are preferred.

### Enzymes

**Cellulase Enzymes.** Cellulase enzymes optionally used in the instant detergent composition are preferably incorporated, when present, at levels sufficient to provide up to about 5 mg by weight, more preferably about 0.01 mg to about 3 mg, of active enzyme per gram of the composition. Unless stated otherwise, the compositions herein preferably comprise from about 0.001% to about 5%, preferably 0.01%-1% by weight of a commercial enzyme preparation.

The cellulases suitable for the present invention include either bacterial or fungal cellulase. Preferably, they will have a pH optimum of between 5 and 9.5. Suitable cellulases are fungal cellulase produced from Humicola insolens and Humicola strain DSM1800 or a cellulase 212-producing fungus belonging to the genus Aeromonas, and cellulase extracted from the hepatopancreas of a marine mollusk (Dolabella Auricula Solander), suitable cellulases are also disclosed in GB 2,075,028 A**.** In addition, cellulase especially suitable for use herein are disclosed in WO 1992 013057 A1**.** Most preferably, the cellulases used in the instant detergent compositions are purchased commercially from NOVO Industries A/S under the product names CAREZYMEO and CELLUZYMEO.

**Other Enzymes.** Additional enzymes can be included in the detergent compositions herein for a wide variety of fabric laundering purposes, including removal of protein-based, carbohydrate-based, or triglyceride-based stains, for example, and for the prevention of refugee dye transfer, and for fabric restoration. The additional enzymes to be incorporated include proteases, amylases, lipases, and peroxidases, as well as mixtures thereof. Other types of enzymes can also be included. They can be of any suitable origin, such as vegetable, animal, bacterial, fungal and yeast origin. However, their choice is governed by several factors such as pH-activity and/or stability optima, thermostability, stability versus active detergents, builders as well as their potential to cause malodors during use. In this respect bacterial or fungal enzymes are preferred, such as bacterial amylases and proteases.

Enzymes are normally incorporated at levels sufficient to provide up to about 5 mg by weight, more typically about 0.01 mg to about 3 mg, of active enzyme per gram of the composition. Stated otherwise, the compositions herein will typically comprise from about 0.001% to about 5%, preferably 0.01%-1% by weight of a commercial enzyme preparation. Protease enzymes are usually present in such commercial preparations at levels sufficient to provide from 0.005 to 0.1 Anson units (AU) of activity per gram of composition.

Suitable examples of proteases are the subtilisins which are obtained from particular strains of B. subtilis and B. licheniforms. Another suitable protease is obtained from a strain of Bacillus, having maximum activity throughout the pH range of 8-12, developed and sold by Novo Industries A/S under the registered trade name ESPERASE^{®}. The preparation of this enzyme and analogous enzymes is described in GB 1,243,784 of Novo. Proteolytic enzymes suitable for removing protein-based stains that are commercially available include those sold under the trade names ALCALASE^{®} and SAVINASE^{®} by Novo Industries A/S and MAXATASE^{®} by International Bio-Synthetics, Inc.. Other proteases include Protease A; Protease B and proteases made by Genencor International, Inc., according to US 5,204,015 and US 5,244,791.

Amylases include, for example, alpha-amylases like RAPIDASE^{®}, International Bio-Synthetics, Inc. and TERMAMYL^{®}, Novo Industries.

Suitable lipase enzymes for detergent usage include those produced by microorganisms of the Pseudomonas group, such as Pseudomonas stutzeri ATCC 19154. This lipase is available from Amano Pharmaceutical Co. Ltd., under the trade name Lipase P "Amano". Other commercial lipases include Amano-CES, lipases ex Chromobacter viscosum, e.g. Chromobacter viscosum var. lipolyticum NRRLB 3673, commercially available from Toyo Jozo Co., and further Chromobacter viscosum lipases from U.S. Biochemical Corp. and Disoynth Co., and lipases ex Pseudomonas gladioli. The LIPOLASE^{®} enzyme derived from Humicola lanuginosa (commercially available from Novo Industries A/S) is a preferred lipase for use herein.

Peroxidase enzymes are used in combination with oxygen sources, e.g., percarbonate, perborate, persulfate, hydrogen peroxide, etc. They are used for "solution bleaching," i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro- and bromo-peroxidase. Peroxidase-containing detergent compositions are disclosed, for example, in WO 1989 099813 A1**.**

**Enzyme Stabilizers.** The enzymes employed herein are stabilized by the presence of water-soluble sources of calcium and/or magnesium ions in the finished detergent compositions which provide such ions to the enzymes. (Calcium ions are generally somewhat more effective than magnesium ions and are preferred herein if only one type of cation is being used.) Additional stability can be provided by the presence of various other artdisclosed stabilizers, especially borate species, see US 4,537,706**,**

Typical detergents, especially liquids, will comprise from about 1 to about 30, preferably from about 2 to about 20, more preferably from about 5 to about 15, and most preferably from about 8 to about 12, millimoles of calcium ion per liter of finished composition. In solid detergent compositions the formulation can include a sufficient quantity of a water-soluble calcium ion source to provide such amounts in the laundry liquor. In the alternative, natural water hardness can suffice.

It is to be understood that the foregoing levels of calcium and/or magnesium ions are sufficient to provide enzyme stability. More calcium and/or magnesium ions can be added to the compositions to provide an additional measure of grease removal performance. Accordingly, as a general proposition the compositions herein will typically comprise from about 0.05% to about 2% by weight of a water-soluble source of calcium or magnesium ions, or both. The amount can vary, of course, with the amount and type of enzyme employed in the composition.

The compositions herein can also optionally, but preferably, contain various additional stabilizers, especially borate-type stabilizers. Typically, such stabilizers will be used at levels in the compositions from about 0.25% to about 10%, preferably from about 0.5% to about 5%, more preferably from about 0.75% to about 3%, by weight of boric acid or other borate compound capable of forming boric acid in the composition (calculated on the basis of boric acid). Boric acid is preferred, although other compounds such as boric oxide, borax and other alkali metal borates (e.g., sodium ortho-, meta- and pyroborate, and sodium pentaborate) are suitable. Substituted boric acids (e.g., phenylboronic acid, butane boronic acid, and p-bromo phenylboronic acid) can also be used in place of boric acid.

### Builders

**Zeolites.** Fine crystalline, synthetic zeolites containing bound water can be used as builders, for example, preferably zeolite A and/or P. Zeolite MAP.RTM. (commercial product of the Crosfield company), is particularly preferred as the zeolite P. However, zeolite X and mixtures of A, X, Y and/or P are also suitable. A co-crystallized sodium/potassium aluminum silicate from Zeolite A and Zeolite X, which is available as Vegobond^{®} RX. (commercial product from Condea Augusta S.p.A.), is also of particular interest. Preferably, the zeolite can be used as a spray-dried powder. For the case where the zeolite is added as a suspension, this can comprise small amounts of nonionic surfactants as stabilizers, for example, 1 to 3 wt. %, based on the zeolite, of ethoxylated C₁₂-C₁₈ fatty alcohols with 2 to 5 ethylene oxide groups, C₁₂-C₁₄ fatty alcohols with 4 to 5 ethylene oxide groups or ethoxylated isotridecanols. Suitable zeolites have an average particle size of less than 10µm (test method: volumetric distribution Coulter counter) and preferably comprise 18 to 22 wt. %, particularly 20 to 22 wt. % of bound water. Apart from this, phosphates can also be used as builders.

**Layered silicates.** Suitable substitutes or partial substitutes for phosphates and zeolites are crystalline, layered sodium silicates. These types of crystalline layered silicates are described, for example, in European Patent Application EP 0164514 A1. Preferred crystalline layered silicates are those obtained for example, from the process described in International Patent Application WO 91/08171 A1.

**Amorphous silicates.** Preferred builders also include amorphous sodium silicates with a modulus (Na₂O:SiO₂ ratio) of 1:2 to 1:3.3, preferably 1:2 to 1:2.8 and more preferably 1:2 to 1:2.6, which dissolve with a delay and exhibit multiple wash cycle properties. The delay in dissolution compared with conventional amorphous sodium silicates can have been obtained in various ways, for example, by surface treatment, compounding, compressing/compacting or by over-drying. In the context of this invention, the term "amorphous" also means "X-ray amorphous". In other words, the silicates do not produce any of the sharp X-ray reflexions typical of crystalline substances in X-ray diffraction experiments, but at best one or more maxima of the scattered X-radiation, which have a width of several degrees of the diffraction angle. However, particularly good builder properties may even be achieved where the silicate particles produce indistinct or even sharp diffraction maxima in electron diffraction experiments. This is to be interpreted to mean that the products have microcrystalline regions between 10 and a few hundred nm in size, values of up to at most 50 nm and especially up to at most 20 nm being preferred. This type of X-ray amorphous silicates, which similarly possess a delayed dissolution in comparison with the customary water glasses, are described, for example, in German Patent Application DE 4400024 A1. Compacted/densified amorphous silicates, compounded amorphous silicates and over dried X-ray-amorphous silicates are particularly preferred.

**Phosphates.** Also the generally known phosphates can also be added as builders, in so far that their use should not be avoided on ecological grounds. The sodium salts of the orthophosphates, the pyrophosphates and especially the tripolyphosphates are particularly suitable. Their content is generally not more than 25 wt. %, preferably not more than 20 wt. %, each based on the finished composition. In some cases it has been shown that particularly tripolyphosphates, already in low amounts up to maximum 10 wt. %, based on the finished composition, in combination with other builders, lead to a synergistic improvement of the secondary washing power. Preferred amounts of phosphates are under 10 wt. %, particularly 0 wt. %.

### Co-builders

**Polycarboxylic acids.** Useful organic cobuilders are, for example, the polycarboxylic acids usable in the form of their sodium salts of polycarboxylic acids, wherein polycarboxylic acids are understood to be carboxylic acids that carry more than one acid function. These include, for example, citric acid, adipic acid, succinic acid, glutaric acid, malic acid, tartaric acid, maleic acid, fumaric acid, sugar acids, aminocarboxylic acids, nitrilotriacetic acid (NTA) and its derivatives and mixtures thereof. Preferred salts are the salts of polycarboxylic acids such as citric acid, adipic acid, succinic acid, glutaric acid, tartaric acid, sugar acids and mixtures thereof.

**Organic acids.** Acids per se can also be used. Besides their building effect, the acids also typically have the property of an acidifying component and, hence also serve to establish a relatively low and mild pH in detergents or cleansing compositions. Citric acid, succinic acid, glutaric acid, adipic acid, gluconic acid and any mixtures thereof are particularly mentioned in this regard. Further suitable acidifiers are the known pH regulators such as sodium hydrogen carbonate and sodium hydrogen sulfate.

**Polymers.** Particularly suitable polymeric cobuilders are polyacrylates, which preferably have a molecular weight of 2,000 to 20,000 g/mol. By virtue of their superior solubility, preferred representatives of this group are again the short-chain polyacrylates, which have molecular weights of 2,000 to 10,000 g/mol and, more particularly, 3,000 to 5,000 g/mol. Suitable polymers can also include substances that consist partially or totally of vinyl alcohol units or its derivatives.

Further suitable copolymeric polycarboxylates are particularly those of acrylic acid with methacrylic acid and of acrylic acid or methacrylic acid with maleic acid. Copolymers of acrylic acid with maleic acid, which comprise 50 to 90 wt. % acrylic acid and 50 to 10 wt. % maleic acid, have proven to be particularly suitable. Their relative molecular weight, based on free acids, generally ranges from 2,000 to 70,000 g/mol, preferably 20,000 to 50,000 g/mol and especially 30,000 to 40,000 g/mol. The (co)polymeric polycarboxylates can be added either as an aqueous solution or preferably as powder. In order to improve the water solubility, the polymers can also comprise allylsulfonic acids as monomers, such as, for example, allyloxybenzene sulfonic acid and methallyl sulfonic acid as in the EP 0727448 B1**.**

Biodegradable polymers comprising more than two different monomer units are particularly preferred, examples being those comprising, as monomers, salts of acrylic acid and of maleic acid, and also vinyl alcohol or vinyl alcohol derivatives, as in DE 4300772 A1**,** or those comprising, as monomers, salts of acrylic acid and of 2-alkylallyl sulfonic acid, and also sugar derivatives. Further preferred copolymers are those that are described in German Patent Applications DE 4303320 A1 and DE 4417734 A1 and preferably include acrolein and acrylic acid/acrylic acid salts or acrolein and vinyl acetate as monomers.

Similarly, other preferred builders are polymeric aminodicarboxylic acids, salts or precursors thereof. Those polyaspartic acids or their salts and derivatives disclosed in German Patent Application DE 19540086 A1 as having a bleach-stabilizing action in addition to cobuilder properties are particularly preferred.

Further suitable builders are polyacetals that can be obtained by treating dialdehydes with polyol carboxylic acids that possess 5 to 7 carbon atoms and at least 3 hydroxyl groups, as described in European Patent Application EP 0280223 A1. Preferred polyacetals are obtained from dialdehydes like glyoxal, glutaraldehyde, terephthalaldehyde as well as their mixtures and from polycarboxylic acids like gluconic acid and/or glucoheptonic acid.

**Carbohydrates.** Further suitable organic cobuilders are dextrins, for example, oligomers or polymers of carbohydrates that can be obtained by the partial hydrolysis of starches. The hydrolysis can be carried out using typical processes, for example, acidic or enzymatic catalyzed processes. The hydrolysis products preferably have average molecular weights in the range of 400 to 500,000 g/mol. A polysaccharide with a dextrose equivalent (DE) of 0.5 to 40 and, more particularly, 2 to 30 is preferred, the DE being an accepted measure of the reducing effect of a polysaccharide in comparison with dextrose, which has a DE of 100. Both maltodextrins with a DE between 3 and 20 and dry glucose syrups with a DE between 20 and 37 and also so-called yellow dextrins and white dextrins with relatively high molecular weights of 2,000 to 30,000 g/mol may be used. A preferred dextrin is described in British Patent Application 94 19 091.

The oxidized derivatives of such dextrins concern their reaction products with oxidizing compositions that are capable of oxidizing at least one alcohol function of the saccharide ring to the carboxylic acid function. Such oxidized dextrins and processes for their manufacture are known for example, from European Patent Applications EP 0232202 A1**.** A product oxidized at C6 of the saccharide ring can be particularly advantageous.

**Oxydisuccinates** and other derivatives of disuccinates, preferably ethylenediamine disuccinate are also further suitable cobuilders. Here, ethylene diamine-N,N'-disuccinate (EDDS), the synthesis of which is described for example, in US 3,158,615**,** is preferably used in the form of its sodium or magnesium salts. In this context, glycerine disuccinates and glycerine trisuccinates are also particularly preferred, such as those described in US 4,524,009**.** Suitable addition quantities in zeolite-containing and/or silicate-containing formulations range from 3 to 15% by weight.

**(Lactones.** Other useful organic co-builders are, for example, acetylated hydroxycarboxylic acids and salts thereof which optionally may also be present in lactone form and which contain at least 4 carbon atoms, at least one hydroxyl group and at most two acid groups. Such cobuilders are described, for example, in International Patent Application WO 1995 020029 A1**.**

### Bleaching Compounds, Bleaching Agents and Bleach Activators

The detergent compositions herein can optionally contain bleaching agents or bleaching compositions containing a bleaching agent and one or more bleach activators. When present, bleaching agents will typically be at levels of from about 1% to about 30%, more typically from about 5% to about 20%, of the detergent composition, especially for fabric laundering. If present, the amount of bleach activators will typically be from about 0.1% to about 60%, more typically from about 0.5% to about 40% of the bleaching composition comprising the bleaching agent-plus-bleach activator.

The bleaching agents used herein can be any of the bleaching agents useful for detergent compositions in textile cleaning, hard surface cleaning, or other cleaning purposes that are now known or become known. These include oxygen bleaches as well as other bleaching agents. Perborate bleaches, e.g., sodium perborate (e.g., mono- or tetra-hydrate) can be used herein.

Another category of bleaching agent that can be used without restriction encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid.

Peroxygen bleaching agents can also be used. Suitable peroxygen bleaching compounds include sodium carbonate peroxyhydrate and equivalent "percarbonate" bleaches, sodium pyrophosphate peroxyhydrate, urea peroxyhydrate, and sodium peroxide. Persulfate bleach (e.g., OXONEO^{®}, manufactured commercially by DuPont) can also be used.

A preferred percarbonate bleach comprises dry particles having an average particle size in the range from about 500 micrometers to about 1,000 micrometers, not more than about 10% by weight of said particles being smaller than about 200 micrometers and not more than about 10% by weight of said particles being larger than about 1,250 micrometers. Optionally, the percarbonate can be coated with silicate, borate or water-soluble surfactants. Percarbonate is available from various commercial sources.

Mixtures of bleaching agents can also be used.

Peroxygen bleaching agents, the perborates, the percarbonates, etc., are preferably combined with bleach activators, which lead to the in situ production in aqueous solution (i.e., during the washing process) of the peroxy acid corresponding to the bleach activator. The nonanoyloxybenzene sulfonate (NOBS) and tetraacetyl ethylene diamine (TAED) activators are typical, and mixtures thereof can also be used.

Preferred amido-derived bleach activators include (6-octanamido-caproyl)oxyben-zene-sulfonate, (6-nonanamidocaproyl)oxybenzenesulfonate, (6-decanamido-caproyl)-oxyben-zenesulfonate, and mixtures thereof.

Another class of bleach activators comprises the benzoxazin-type activators disclosed in US 4,966,723**,**

Highly preferred lactam activators include benzoyl caprolactam, octanoyl caprolactam, 3,5,5-trimethylhexanoyl caprolactam, nonanoyl caprolactam, decanoyl caprolactam, undecenoyl caprolactam, benzoyl valerolactam, octanoyl valerolactam, decanoyl valerolactam, undecenoyl valerolactam, nonanoyl valerolactam, 3,5,5-trimethylhexanoyl valerolactam and mixtures thereof, optionally adsorbed into solid carriers, e.g acyl caprolactams, preferably benzoyl caprolactam, adsorbed into sodium perborate.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. If used, detergent compositions will typically contain from about 0.025% to about 1.25%, by weight, of such bleaches, especially sulfonate zinc phthalocyanine.

If desired, the bleaching compounds can be catalyzed by means of a manganese compound. Such manganese-based catalysts are well known in the art and include Mn^{IV}₂ (u-O)₃ (1,4,7-trimethyl-1,4,7-triazacyclononane)₂ (PF₆)₂, Mn^{III}₂ (u-O)₁ (u-OAc)₂ (1,4,7-trimethyl-1,4,7-triazacyclononane)₂(ClO₄)₂, Mn^{IV}₄ (u-O)₆ (1,4,7-triazacyclononane)₄ (ClO₄)₄, Mn^{III}Mn^{IV}₄ (u-O)₁ (u-OAc)₂ (1,4,7-trimethyl-1,4,7-triazacyclononane)₂ (ClO₄)₃, Mn^{IV} (1,4,7-trimethyl-1,4,7-triazacyclononane)-(OCH₃)₃ (PF₆), and mixtures thereof.

As a practical matter, and not by way of limitation, the compositions and processes herein can be adjusted to provide on the order of at least one part per ten million of the active bleach catalyst species in the aqueous washing liquor, and will preferably provide from about 0.1 ppm to about 700 ppm, more preferably from about 1 ppm to about 500 ppm, of the catalyst species in the laundry liquor.

### Polymeric Soil Release Agents

Any polymeric soil release agent known to those skilled in the art can optionally be employed in the detergent compositions and processes of this invention. Polymeric soil release agents are characterized by having both hydrophilic segments, to hydrophilize the surface of hydrophobic fibers, such as polyester and nylon, and hydrophobic segments, to deposit upon hydrophobic fibers and remain adhered thereto through completion of washing and rinsing cycles and, thus, serve as an anchor for the hydrophilic segments. This can enable stains occurring subsequent to treatment with the soil release agent to be more easily cleaned in later washing procedures.

The polymeric soil release agents useful herein especially include those soil release agents having: (a) one or more nonionic hydrophile components consisting essentially of (i) polyoxyethylene segments with a degree of polymerization of at least 2, or (ii) oxypropylene or polyoxypropylene segments with a degree of polymerization of from 2 to 10, wherein said hydrophile segment does not encompass any oxypropylene unit unless it is bonded to adjacent moieties at each end by ether linkages, or (iii) a mixture of oxyalkylene units comprising oxyethylene and from 1 to about 30 oxypropylene units wherein said mixture contains a sufficient amount of oxyethylene units such that the hydrophile component has hydrophilicity great enough to increase the hydrophilicity of conventional polyester synthetic fiber surfaces upon deposit of the soil release agent on such surface, said hydrophile segments preferably comprising at least about 25% oxyethylene units and more preferably, especially for such components having about 20 to 30 oxypropylene units, at least about 50% oxyethylene units; or (b) one or more hydrophobe components comprising (i) C₃ oxyalkylene terephthalate segments, wherein, if said hydrophobe components also comprise oxyethylene terephthalate, the ratio of oxyethylene terephthalate: C₃ oxyalkylene terephthalate units is about 2:1 or lower, (ii) C₄ - C₆ alkylene or oxy C₄ - C₆ alkylene segments, or mixtures therein, (iii) poly (vinyl ester) segments, preferably polyvinyl acetate), having a degree of polymerization of at least 2, or (iv) C₁ - C₄ alkyl ether or C₄ hydroxyalkyl ether substituents, or mixtures therein, wherein said substituents are present in the form of C₁ - C₄ alkyl ether or C₄ hydroxyalkyl ether cellulose derivatives, or mixtures therein, and such cellulose derivatives are amphiphilic, whereby they have a sufficient level of C₁ - C₄ alkyl ether and/or C₄ hydroxyalkyl ether units to deposit upon conventional polyester synthetic fiber surfaces and retain a sufficient level of hydroxyls, once adhered to such conventional synthetic fiber surface, to increase fiber surface hydrophilicity, or a combination of (a) and (b).

Typically, the polyoxyethylene segments of (a) (i) will have a degree of polymerization of from about 200, although higher levels can be used, preferably from 3 to about 150, more preferably from 6 to about 100. Suitable oxy C₄ - C₆ alkylene hydrophobe segments include, but are not limited to, end-caps of polymeric soil release agents.

Polymeric soil release agents useful in the present invention also include cellulosic derivatives such as hydroxyether cellulosic polymers, copolymeric blocks of ethylene terephthalate or propylene terephthalate with polyethylene oxide or polypropylene oxide terephthalate, and the like. Such agents are commercially available and include hydroxyethers of cellulose such as METHOCEL^{®} (Dow). Cellulosic soil release agents for use herein also include those selected from the group consisting of C₁ - C₄ alkyl and C₄ hydroxyalkyl cellulose.

Soil release agents characterized by poly(vinyl ester) hydrophobe segments include graft copolymers of poly(vinyl ester), e.g., C₁ - C₆ vinyl esters, preferably poly(vinyl acetate) grafted onto polyalkylene oxide backbones, such as polyethylene oxide backbones, see EP 0 219 048, incorporated herein in its entirety. Commercially available soil release agents of this kind include the SOKALAN^{®} type of material, e.g., SOKALAN^{®} HP-22, available from BASF.

One type of preferred soil release agent is a copolymer having random blocks of ethylene terephthalate and polyethylene oxide (PEO) terephthalate. The molecular weight of this polymeric soil release agent preferably is in the range of from about 25,000 to about 55,000.

Another preferred polymeric soil release agent is a polyester with repeat units of ethylene terephthalate units contains 10-15% by weight of ethylene terephthalate units together with 90-80% by weight of polyoxyethylene terephthalate units, derived from a polyoxyethylene glycol of average molecular weight 300-5,000. Examples of this polymer include the commercially available material ZELCON^{®} 5126 (from DuPont) and MILEASE^{®} T (from ICI).

Another preferred polymeric soil release agent is a sulfonated product of a substantially linear ester oligomer comprised of an oligomeric ester backbone of terephthaloyl and oxyalkyleneoxy repeat units and terminal moieties covalently attached to the backbone. These soil release agents are described fully in US 4,968,451. Other suitable polymeric soil release agents include the terephthalate polyesters of US 4,711,730, the anionic end-capped oligomeric esters of US 4,721,580, the block polyester oligomeric compounds of US 4,702,857, and anionic, especially sulfoaroyl, end-capped terephthalate esters of US 4,877,896

Still another preferred soil release agent is an oligomer with repeat units of terephthaloyl units, sulfoisoterephthaloyl units, oxyethyleneoxy and oxy-1,2-propylene units. The repeat units form the backbone of the oligomer and are preferably terminated with modified isethionate end-caps. A particularly preferred soil release agent of this type comprises about one sulfoisophthaloyl unit, 5 terephthaloyl units, oxyethyleneoxy and oxy-1,2-propyleneoxy units in a ratio of from about 1.7 to about 1.8, and two end-cap units of sodium 2-(2-hydroxyethoxy)-ethanesulfonate. Said soil release agent also comprises from about 0.5% to about 20%, by weight of the oligomer, of a crystalline-reducing stabilizer, preferably selected from the group consisting of xylene sulfonate, cumene sulfonate, toluene sulfonate, and mixtures thereof.

If utilized, soil release agents will generally comprise from about 0.01% to about 10.0%, by weight, of the detergent compositions herein, typically from about 0.1% to about 5%, preferably from about 0.2% to about 3.0%.

### Polymeric dispersing agents

Polymeric dispersing agents can advantageously be utilized at levels from about 0.1% to about 7%, by weight, in the detergent compositions herein, especially in the presence of zeolite and/or layered silicate builders. Suitable polymeric dispersing agents include polymeric polycarboxylates and polyethylene glycols, although others known in the art can also be used. It is believed, though it is not intended to be limited by theory, that polymeric dispersing agents enhance overall detergent builder performance, when used in combination with other builders (including lower molecular weight polycarboxylates) by crystal growth inhibition, particulate soil release peptization, and anti-redeposition.

Polymeric polycarboxylate materials can be prepared by polymerizing or copolymerizing suitable unsaturated monomers, preferably in their acid form. Unsaturated monomeric acids that can be polymerized to form suitable polymeric polycarboxylates include acrylic acid, maleic acid (or maleic anhydride), fumaric acid, itaconic acid, aconitic acid, mesaconic acid, citraconic acid and methylenemalonic acid. The presence in the polymeric polycarboxylates herein or monomeric segments, containing no carboxylate radicals such as vinylmethyl ether, styrene, ethylene, etc. is suitable provided that such segments do not constitute more than about 40% by weight.

Particularly suitable polymeric polycarboxylates can be derived from acrylic acid. Such acrylic acid-based polymers which are useful herein are the water-soluble salts of polymerized acrylic acid. The average molecular weight of such polymers in the acid form preferably ranges from about 2,000 to 10,000, more preferably from about 4,000 to 7,000 and most preferably from about 4,000 to 5,000. Water-soluble salts of such acrylic acid polymers can include, for example, the alkali metal, ammonium and substituted ammonium salts. Soluble polymers of this type are known materials. Use of polyacrylates of this type in detergent compositions has been disclosed, for example US 3,308,067**.**

Acrylic/maleic-based copolymers can also be used as a preferred component of the dispersing/anti-redeposition agent. Such materials include the water-soluble salts of copolymers of acrylic acid and maleic acid. The average molecular weight of such copolymers in the acid form preferably ranges from about 2,000 to 100,000, more preferably from about 5,000 to 75,000, most preferably from about 7,000 to 65,000. The ratio of acrylate to maleate segments in such copolymers will generally range from about 30:1 to about 1:1, more preferably from about 10:1 to 2:1. Water-soluble salts of such acrylic acid/maleic acid copolymers can include, for example, the alkali metal, ammonium and substituted ammonium salts. Soluble acrylate/maleate copolymers of this type are known materials which are described in EP 0193360 A1, which also describes such polymers comprising hydroxypropylacrylate. Still other useful dispersing agents include the maleic/acrylic/vinyl alcohol terpolymers, for example, a 45/45/10 terpolymer of acrylic/maleic/vinyl alcohol.

Another polymeric material which can be included is polyethylene glycol (PEG). PEG can exhibit dispersing agent performance as well as act as a clay soil removal-antiredeposition agent. Typical molecular weight ranges for these purposes range from about 500 to about 100,000, preferably from about 1,000 to about 50,000, more preferably from about 1,500 to about 10,000.

Polyaspartate and polyglutamate dispersing agents can also be used, especially in conjunction with zeolite builders. Dispersing agents such as polyaspartate preferably have a molecular weight (avg.) of about 10,000.

### Foam inhibitors/Sud supressors

Especially when used in automatic washing processes, it can be advantageous to add conventional foam inhibitors to the compositions. Suitable foam inhibitors include for example, soaps of natural or synthetic origin, which have a high content of C₁₈-C₂₄ fatty acids. Suitable non-surface-active types of foam inhibitors are, for example, organopolysiloxanes and mixtures thereof with microfine, optionally silanised silica and also paraffins, waxes, microcrystalline waxes and mixtures thereof with silanised silica or bis-stearyl ethylenediamide. Mixtures of various foam inhibitors, for example, mixtures of silicones, paraffins or waxes, are also used with advantage. Preferably, the foam inhibitors, especially silicone-containing and/or paraffin-containing foam inhibitors, are loaded onto a granular, water-soluble or dispersible carrier material. Especially in this case, mixtures of paraffins and bis-stearylethylene diamides are preferred.

Compounds for reducing or suppressing the formation of suds can be incorporated into the detergent compositions of the present invention. Suds suppression can be of particular importance in the so-called "high concentration cleaning process" and in frontloading European-style washing machines.

A wide variety of materials can be used as suds suppressors, and suds suppressors are well known to those skilled in the art. See, for example, Kirk Othmer Encyclopedia of Chemical Technology, Third Edition, Volume 7, pages 430-447 (John Wiley & Sons, Inc., 1979). One category of suds suppressor of particular interest encompasses monocarboxylic fatty acid and soluble salts therein. The monocarboxylic fatty acids and salts thereof used as suds suppressor typically have hydrocarbyl chains of 10 to about 24 carbon atoms, preferably 12 to 18 carbon atoms. Suitable salts include the alkali metal salts such as sodium, potassium, and lithium salts, and ammonium and alkanolammonium salts.

The detergent compositions herein can also contain non-surfactant suds suppressors. These include, for example: high molecular weight hydrocarbons such as paraffin, fatty acid esters (e.g., fatty acid triglycerides), fatty acid esters of monovalent alcohols, aliphatic C₁₈- C₄₀ ketones (e.g., stearone), etc. Other suds inhibitors include N-alkylated amino triazines such as tri- to hexa-alkylmelamines or di- to tetra-alkyldiamine chlortriazines formed as products of cyanuric chloride with two or three moles of a primary or secondary amine containing 1 to 24 carbon atoms, propylene oxide, and monostearyl phosphates such as monostearyl alcohol phosphate ester and monostearyl di-alkali metal (e.g., K, Na, and Li) phosphates and phosphate esters. The hydrocarbons such as paraffin and haloparaffin can be utilized in liquid form. The liquid hydrocarbons will be liquid at room temperature and atmospheric pressure, and will have a pour point in the range of about - 40°C and about 50°C, and a minimum boiling point not less than about 110°C (atmospheric pressure). It is also known to utilize waxy hydrocarbons, preferably having a melting point below about 100°C. Hydrocarbon suds suppressors are known in the art and include aliphatic, alicyclic, aromatic, and heterocyclic saturated or unsaturated hydrocarbons having from about 12 to about 70 carbon atoms. The term "paraffin," as used in this suds suppressor discussion, is intended to include mixtures of true paraffins and cyclic hydrocarbons.

Another preferred category of non-surfactant suds suppressors comprises silicone suds suppressors. This category includes the use of polyorganosiloxane oils, such as polydimethylsiloxane, dispersions or emulsions of polyorganosiloxane oils or resins, and combinations of polyorganosiloxane with silica particles wherein the polyorganosiloxane is chemisorbed or fused onto the silica. Silicone suds suppressors are well known in the art.

Other silicone suds suppressors are disclosed in US 3,455,839**,** which relates to compositions and processes for defoaming aqueous solutions by incorporating therein small amounts of polydimethylsiloxane fluids.

Mixtures of silicone and silanated silica are described, for instance, in DE-OS 2124526**,** Silicone defoamers and suds controlling agents in granular detergent compositions are disclosed in US 4,652,392**,**

In the preferred silicone suds suppressor used herein, the solvent for a continuous phase is made up of certain polyethylene glycols or polyethylene-polypropylene glycol copolymers or mixtures thereof (preferred), or polypropylene glycol. The primary silicone suds suppressor is branched/crosslinked and preferably not linear.

The silicone suds suppressor herein preferably comprises polyethylene glycol and a copolymer of polyethylene glycol/polypropylene glycol, all having an average molecular weight of less than about 1,000, preferably between about 100 and 800. The polyethylene glycol and polyethylene/polypropylene copolymers herein have a solubility in water at room temperature of more than about 2 weight %, preferably more than about 5 weight %.

The preferred solvent herein is polyethylene glycol having an average molecular weight of less than about 1,000, more preferably between about 100 and 800, most preferably between 200 and 400, and a copolymer of polyethylene glycol/polypropylene glycol, preferably PPG 200/PEG 300. Preferred is a weight ratio of between about 1:1 and 1:10, most preferably between 1:3 and 1:6, of polyethylene glycol:copolymer of polyethylene-polypropylene glycol.

The preferred silicone suds suppressors used herein do not contain polypropylene glycol, particularly of 4,000 molecular weight. They also preferably do not contain block copolymers of ethylene oxide and propylene oxide, like PLURONIC^{®} L101.

Other suds suppressors useful herein comprise the secondary alcohols (e.g., 2-alkyl alkanols) and mixtures of such alcohols with silicone oils. The secondary alcohols include the C₆ - C₁₆ alkyl alcohols having a C₁ - C₁₆ chain. A preferred alcohol is 2-butyl octanol, which is available from Condea under the trademark ISOFOL^{®} 12. Mixtures of secondary alcohols are available under the trademark ISALCHEM^{®} 123 from Enichem. Mixed suds suppressors typically comprise mixtures of alcohol+silicone at a weight ratio of 1:5 to 5:1.

The compositions herein will generally comprise from 0% to about 5% of suds suppressor. When utilized as suds suppressors, monocarboxylic fatty acids, and salts therein, will be present typically in amounts up to about 5%, by weight, of the detergent composition. Preferably, from about 0.5% to about 3% of fatty monocarboxylate suds suppressor is utilized. Silicone suds suppressors are typically utilized in amounts up to about 2.0%, by weight, of the detergent composition, although higher amounts can be used. This upper limit is practical in nature, due primarily to concern with keeping costs minimized and effectiveness of lower amounts for effectively controlling sudsing. Preferably from about 0.01% to about 1% of silicone suds suppressor is used, more preferably from about 0.25% to about 0.5%. As used herein, these weight percentage values include any silica that can be utilized in combination with polyorganosiloxane, as well as any adjunct materials that can be utilized. Monostearyl phosphate suds suppressors are generally utilized in amounts ranging from about 0.1% to about 2%, by weight, of the composition. Hydrocarbon suds suppressors are typically utilized in amounts ranging from about 0.01% to about 5.0%, although higher levels can be used. The alcohol suds suppressors are typically used at 0.2%-3% by weight of the finished compositions.

### Sequestrants and chelating agents

The salts of polyphosphonic acid can be considered as sequestrants or as stabilizers, particularly for peroxy compounds and enzymes, which are sensitive towards heavy metal ions. Here, the sodium salts of, for example, 1-hydroxyethane-1,1-diphosphonate, diethylenetriamine pentamethylene phosphonate or ethylenediamine tetramethylene phosphonate are used in amounts of 0.1 to 5 wt. %.

The detergent compositions herein can also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates. It is understood that some of the detergent builders described hereinbefore can function as chelating agents and is such detergent builder is present in a sufficient quantity, it can provide both functions.

Amino carboxylates useful as optional chelating agents include ethylenediaminetetracetates, N-hydroxyethylethylenediaminetriacetates, nitrilotriacetates, ethylenediamine tetraproprionates, triethylenetetraaminehexacetates, diethylenetriaminepentaacetates, and ethanoldiglycines, alkali metal, ammonium, and substituted ammonium salts therein and mixtures therein.

Amino phosphonates are also suitable for use as chelating agents in the compositions of the invention when at lease low levels of total phosphorus are permitted in detergent compositions, and include ethylenediaminetetrakis (methylenephosphonates) as DEQUEST. Preferred, these amino phosphonates to not contain alkyl or alkenyl groups with more than about 6 carbon atoms.

Polyfunctionally-substituted aromatic chelating agents are also useful in the compositions herein. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer.

If utilized, these chelating agents will generally comprise from about 0.1% to about 10% by weight of the detergent compositions herein. More preferably, if utilized, the chelating agents will comprise from about 0.1% to about 3.0% by weight of such compositions.

### Clay Soil Removal/Anti-redeposition Agents

The detergent compositions of the present invention can also optionally contain water-soluble ethoxylated amines having clay soil removal and antiredeposition properties. Granular detergent compositions which contain these compounds typically contain from about 0.01% to about 10.0% by weight of the water-soluble ethoxylates amines; liquid detergent compositions typically contain about 0.01% to about 5%.

The most preferred soil release and anti-redeposition agent is ethoxylated tetraethylenepentamine. Exemplary ethoxylated amines are further described in US 4,597,898**.** Other groups of preferred clay soil removal-antiredeposition agents are the cationic compounds disclosed in EP 0111965 A1**,** the ethoxylated amine polymers disclosed in EP 0111984 A1**,** the zwitterionic polymers disclosed in EP 0112592 A1**,** and the amine oxides disclosed in US 4,548,744**.** Another type of preferred antiredeposition agent includes the carboxy methyl cellulose (CMC) materials. These materials are well known in the art.

### Graying inhibitors

Graying inhibitors have the function of maintaining the dirt that was removed from the fibers suspended in the washing liquor, thereby preventing the dirt from resettling. Water-soluble colloids of mostly organic nature are suitable for this, for example, the water-soluble salts of (co)polymeric carboxylic acids, glue, gelatins, salts of ether carboxylic acids or ether sulfonic acids of starches or celluloses, or salts of acidic sulfuric acid esters of celluloses or starches. Water-soluble, acid group-containing polyamides are also suitable for this purpose. Moreover, soluble starch preparations and others can be used as the above-mentioned starch products, e.g., degraded starches, aldehyde starches etc. Polyvinyl pyrrolidone can also be used. Preference, however, is given to the use of cellulose ethers such as carboxymethyl cellulose (Na salt), methyl cellulose, hydroxyalkyl celluloses and mixed ethers such as methyl hydroxyethyl cellulose, methyl hydroxypropyl cellulose, methyl carboxymethyl cellulose and mixtures thereof, as well as polyvinyl pyrrolidone, which can be added, for example, in amounts of 0.1 to 5 wt. %, based on the composition.

### Optical brighteners and UV adsorbers

Any optical brighteners or other brightening or whitening agents known in the art can be incorporated at levels typically from about 0.05% to about 1.2%, by weight, into the detergent compositions herein. Commercial optical brighteners which can be useful in the present invention can be classified into subgroups, which include, but are not necessarily limited to, derivatives of stilbene, pyrazoline, coumarin, carboxylic acid, methinecyanines, dibenzothiphene-5,5-dioxide, azoles, 5- and 6-membered-ring heterocycles, and other miscellaneous agents.

Preferred brighteners include the PHORWHITE^{®} series of brighteners from Verona. Other brighteners disclosed in this reference include: Tinopal^{®} UNPA, Tinopal CBS and Tinopal 5BM; available from Ciba-Geigy; Artic White^{®} CC and Artic White CWD, available from Hilton-Davis; the 2-(4-stryl-phenyl)-2H-napthol [1,2-d]triazoles; 4,4'-bis-(1,2,3-triazol-2-yl)-stilbenes; 4,4'-bis(stryl)bisphenyls; and the aminocoumarins. Specific examples of these brighteners include 4-methyl-7-diethyl-amino coumarin; 1,2-bis(-venzimidazol-2-yl)ethylene; 1,3-diphenyl-phrazolines; 2,5-bis(benzoxazol-2-yl)thiophene; 2-stryl-napth- [1,2-d] oxazole; and 2-(stilbene-4-yl)-2H-naphtho- [1,2-d]triazole. Anionic brighteners are preferred herein.

The compositions may comprise e.g., derivatives of diaminostilbene disulfonic acid or alkali metal salts thereof as the optical brighteners. Suitable optical brighteners are, for example, salts of 4,4'-bis-(2-anilino-4-morpholino-1,3,5-triazinyl-6-amino)stilbene-2,2'-disulfonic acid or compounds of similar structure which contain a diethanolamino group, a methylamino group, an anilino group or a 2-methoxyethylamino group instead of the morpholino group. Brighteners of the substituted diphenylstyryl type may also be present, for example, the alkali metal salts of 4,4'-bis(2-sulfostyryl)diphenyl, 4,4'-bis(4-chloro-3-sulfostyryl)diphenyl or 4-(4-chlorostyryl)-4'-(2-sulfostyryl)diphenyl. Mixtures of the mentioned brighteners may also be used.

In addition, UV absorbers may also be added. These are compounds with distinct absorption abilities for ultra violet radiation, which contribute as UV stabilizers as well as to improve the light stability of colorants and pigments both for textile fibers as well as for the skin of the wearer of textile products by protecting against the UV radiation that penetrates the fabric. In general, the efficient radiationless deactivating compounds are derivatives of benzophenone, substituted with hydroxyl and/or alkoxy groups, mostly in position(s) 2 and/or 4. Also suitable are substituted benzotriazoles, additionally acrylates that are phenylsubstituted in position 3 (cinnamic acid derivatives), optionally with cyano groups in position 2, salicylates, organic Ni complexes, as well as natural substances such as umbelliferone and the endogenous urocanic acid. In a preferred embodiment, the UV absorbers absorb UV-A and UV-B radiation as well as possible UV-C radiation and re-emit light with blue wavelengths, such that they additionally have an optical brightening effect. Preferred UV absorbers encompass triazine derivatives, e.g., hydroxyaryl-1,3,5-triazine, sulfonated 1,3,5-triazine, o-hydroxyphenylbenzotriazole and 2-aryl-2H-benzotriazole as well as bis(anilinotriazinyl-amino)stilbene disulfonic acid and their derivatives. Ultra violet absorbing pigments like titanium dioxide can also be used as UV absorbers.

### Dye Transfer Inhibiting Agents

The detergent compositions of the present invention can also include one or more materials effective for inhibiting the transfer of dyes from one fabric to another during the cleaning process. Generally, such dye transfer inhibiting agents include polyvinyl pyrrolidone polymers, polyamine N-oxide polymers, copolymers of N-vinylpyrrolidone and N-vinylimidazole, manganese phthalocyanine, peroxidases, and mixtures thereof. If used, these agents typically comprise from about 0.01% to about 10% by weight of the composition, preferably from about 0.01% to about 5%, and more preferably from about 0.05% to about 2%.

More specifically, the polyamine N-oxide polymers preferred for use herein are described in US 6,491,728**,**

Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof. These polymers include random or block copolymers where one monomer type is an amine N-oxide and the other monomer type is an N-oxide. The amine N-oxide polymers typically have a ratio of amine to the amine N-oxide of 10:1 to 1:1,000,000. However, the number of amine oxide groups present in the polyamine oxide polymer can be varied by appropriate copolymerization or by an appropriate degree of N-oxidation. The polyamine oxides can be obtained in almost any degree of polymerization. Typically, the average molecular weight is within the range of 500 to 1,000,000; more preferred 1,000 to 500,000; most preferred 5,000 to 100,000. This preferred class of materials can be referred to as "PVNO".

The most preferred polyamine N-oxide useful in the detergent compositions herein is poly(4-vinylpyridine-N-oxide) which as an average molecular weight of about 50,000 and an amine to amine N-oxide ratio of about 1:4.

Copolymers of N-vinylpyrrolidone and N-vinylimidazole polymers (referred to as a class as "PVPVI") are also preferred for use herein. Preferably the PVPVI has an average molecular weight range from 5,000 to 1,000,000, more preferably from 5,000 to 200,000, and most preferably from 10,000 to 20,000. The PVPVI copolymers typically have a molar ratio of N-vinylimidazole to N-vinylpyrrolidone from 1:1 to 0.2:1, more preferably from 0.8:1 to 0.3:1, most preferably from 0.6:1 to 0.4:1. These copolymers can be either linear or branched.

The present invention compositions also can employ a polyvinylpyrrolidone ("PVP") having an average molecular weight of from about 5,000 to about 400,000, preferably from about 5,000 to about 200,000, and more preferably from about 5,000 to about 50,000. PVP's are known to persons skilled in the detergent field. Compositions containing PVP can also contain polyethylene glycol ("PEG") having an average molecular weight from about 500 to about 100,000, preferably from about 1,000 to about 10,000. Preferably, the ratio of PEG to PVP on a ppm basis delivered in wash solutions is from about 2:1 to about 50:1, and more preferably from about 3:1 to about 10:1.

The detergent compositions herein can also optionally contain from about 0.005% to 5% by weight of certain types of hydrophilic optical brighteners which also provide a dye transfer inhibition action. If used, the compositions herein will preferably comprise from about 0.01% to 1% by weight of such optical brighteners.

One preferred brightener is 4,4',-bis[(4-anilino-6-(N-2-bis-hydroxyethyl)-s-triazine-2-yl)amino]-2,2'-stilbenedisulfonic acid and disodium salt. This particular brightener species is commercially marketed under the trade name Tinopal-UNPA-GX^{®} by Ciba-Geigy Corporation. Tinopal-UNPA-GX is the preferred hydrophilic optical brightener useful in the detergent compositions herein.

Another preferred brightener is 4,4'-bis[(4-anilino-6-(N-2-hydroxyethyl-N-methylamino)-s-triazine-2-yl)amino]2,2'-stilbenedisulfonic acid disodium salt. This particular brightener species is commercially marketed under the trade name Tinopal 5BM-GX^{®} by Ciba-Geigy Corporation.

Another preferred brightener brightener is 4,4'-bis[(4-anilino-6-morphilino-s-triazine-2-yl)amino]2,2'-stilbenedisulfonic acid, sodium salt. This particular brightener species is commercially marketed under the trade name Tinopal AMS-GX^{®} by Ciba Geigy Corporation.

The specific optical brightener species selected for use in the present invention provide especially effective dye transfer inhibition performance benefits when used in combination with the selected polymeric dye transfer inhibiting agents hereinbefore described. The combination of such selected polymeric materials (e.g., PVNO and/or PVPVI) with such selected optical brighteners (e.g., Tinopal UNPA-GX, Tinopal 5BM-GX and/or Tinopal AMS-GX) provides significantly better dye transfer inhibition in aqueous wash solutions than does either of these two detergent composition components when used alone. Without being bound by theory, it is believed that such brighteners work this way because they have high affinity for fabrics in the wash solution and therefore deposit relatively quick on these fabrics. The extent to which brighteners deposit on fabrics in the wash solution can be defined by a parameter called the "exhaustion coefficient". The exhaustion coefficient is in general as the ratio of a) the brightener material deposited on fabric to b) the initial brightener concentration in the wash liquor. Brighteners with relatively high exhaustion coefficients are the most suitable for inhibiting dye transfer in the context of the present invention.

Of course, it will be appreciated that other, conventional optical brightener types of compounds can optionally be used in the present compositions to provide conventional fabric "brightness" benefits, rather than a true dye transfer inhibiting effect. Such usage is conventional and well-known to detergent formulations.

### Thickeners

The compositions can also comprise common thickeners and anti-deposition compositions as well as viscosity regulators such as polyacrylates, polycarboxylic acids, polysaccharides and their derivatives, polyurethanes, polyvinyl pyrrolidones, castor oil derivatives, polyamine derivatives such as quaternized and/or ethoxylated hexamethylenediamines as well as any mixtures thereof. Preferred compositions have a viscosity below 10,000 mPa*s, measured with a Brookfield viscosimeter at a temperature of 20°C and a shear rate of 50 min⁻¹.

### Inorganic salts

Further suitable ingredients of the composition are water-soluble inorganic salts such as bicarbonates, carbonates, amorphous silicates or mixtures of these; alkali carbonate and amorphous silicate are particularly used, principally sodium silicate with a molar ratio Na₂O:SiO₂ of 1:1 to 1:4.5, preferably of 1:2 to 1:3.5. Preferred compositions comprise alkaline salts, builders and/or cobuilders, preferably sodium carbonate, zeolite, crystalline, layered sodium silicates and/or trisodium citrate, in amounts of 0.5 to 70 wt. %, preferably 0.5 to 50 wt. %, particularly 0.5 to 30 wt. % anhydrous substance.

### Perfumes and colorants

The compositions can comprise further typical detergent and cleansing composition ingredients such as perfumes and/or colorants, wherein such colorants are preferred that leave no or negligible coloration on the fabrics being washed. Preferred amounts of the totality of the added colorants are below 1 wt. %, preferably below 0.1 wt. %, based on the composition. The compositions can also comprise white pigments such as e.g., TiO₂.

### INDUSTRIAL APPLICATION

Another object of the present invention refers to a method for masking the unpleasant odour of 1,2-alkanediols or products comprising said 1,2-alkanediols comprising or consisting of the following steps:
(a) providing a source of at least one 1,2-alkanediol with an unpleasant odour or a product comprising said at least one 1,2-alkanediol with an unpleasant odour; and
(b) adding at least one malodor reduction material having a Malodor Reduction Value (MORV) of at least 0.5 as explained above.

The present invention also encompasses the use of at least one malodor reduction material having a Malodor Reduction Value (MORV) of at least 0.5 as outlined above for masking the unpleasant odour of 1,2-alkanediols or products comprising them.

To avoid ambiguities, it should be noted that all preferred embodiments, including preferred species and ratios, apply in the same way for the claimed methods and uses and therefore any repetition is superfluous.

### The MORV concept

1) Search Chemical Abstracts using the name and/or the CAS Registry number for the material of interest and use the structure provided. If there are multiple isomers for a given material, use the structure provided by Chemical Abstracts or use the isomer that is most prevalent in the material. Input structure of material into winMolconn (Hall Associates, version 1.1.2.1).
2) Input values from 1.) above into the following four equations:
   a) MORV = -8.5096 + 2.8597x(dxp9) + U253x(knotpv) - 0.34484x(eIC2O2) - 0.00046231 x(iclw) + 3.3509x(idcbar) + 0.1 1 158x(n2pag22)
   b} MORV = -5.2917 + 2.1741 x(dxvp5) - 2.6595 x(dxvp8) + 0.45297x(el C2C2d} 0,6202x(cIC2O2) + 1.3542x(CdCH2) + 0.68I05x(CaasC) + 1.7129x(idcbar)
   c) MORV = -0.0035 + 0.8028x(SHCsatu) + 2, 1673x(xvp7) - 1.3507x(cICIC3d) + 0.61496x(cICIO2) + 0.00403 x(idc) - 0.23286x(nd2).
   d) MORV = -0.9926 - 0.03882x(SdO) + 0.1869x(Ssp3OH) + 2.1847x(xp7) + 0.34344x(e C3O2) - 0.45767x(clC2C3) + 0.7684x(CKetone)

   Equation a) is for the malodor trans-3-methyl-2-hexenoic acid (carboxylic acid based malodors)
   Equation b) is for the malodor trimethylamine (amine based malodors)
   Equation c) is for the malodor 3-mercapto-3-methylhexan-1-ol (thiol based malodors)
   Equation d) is for the malodor skatole (indole based malodors)
3) For purpose of the present application, a material's MORV is the highest MORV value from equations 2a) through 2d).
4) If all MORV values from equations 2a) through 2d) above are greater than 0.5, the subject material has a so-called "Universal MORV".

### EXAMPLES 1 TO 5, COMPARATIVE EXAMPLES C1 TO C4

Various malodour compositions were prepared from individual malodor reduction agents all showing a MORV value of at least 1.0; the compositions are described in **Table 1.** Subsequently these compositions were added to various 1,2-alkanediols in amounts ranging from 1 to 10 wt.-percent and the odor was evaluated by a panel of experienced examiners according to the following scale: (5) serious rancid odor to (1) odorless. The results are presented in **Table 2** and represent mean scores.

**Table 1**

| Malodor reduction compositions for 1,2-alkanediols | | | |
|---|---|---|---|
| **Malodor Reducing Agent** | **M1** | **M2** | **M3** |
| 2,3-Dihydro-5,6-dimethoxy-2-(4- piperidinylmethylene)-1H-inden-1-one | - | - | 20 |
| 3-methyl cyclopentadecenone | - | - | 20 |
| Decyl aldehyde | 20 | - | - |
| Diphenyl oxide | 20 | - | - |
| Isononylacetate | 20 | - | 20 |
| Lauric Aldehyde | 20 | - | 20 |
| Methanoinden-1-yl propanoate | 20 | 20 | 20 |
| Methyl hexadecanoate | - | 20 | - |
| Methyl isoeugenol | - | 20 | - |
| Trans-4-decenal | - | 20 | - |
| Undecyl aldehyde | - | 20 | - |

**Table 2**

| 1,2-alkanediol compositions | | | | | | | | | |
|---|---|---|---|---|---|---|---|---|---|
| **Blend** | **C1** | **C2** | **C3** | **C4** | **1** | **2** | **3** | **4** | **5** |
| 1,2-pentanediol | 100 | - | - | - | - | - | 95 | - | - |
| 1,2-hexanediol | - | 100 | - | - | - | - | - | - | - |
| 1,2-octanediol | - | - | 100 | - | 95 | - | - | - | - |
| 1,2-decanediol | - | - | - | 100 | - | 95 | - | 90 | - |
| M1 | - | - | - | - | 5 | - | - | 10 | - |
| M2 | - | - | - | - | - | 5 | - | - | 10 |
| M3 | - | - | - | - | - | - | 5 | - | - |

| ***Olfactory evaluation*** | | | | | | | | | |
|---|---|---|---|---|---|---|---|---|---|
| After 4 hours | 4 | 3 | 3 | 3 | 1 | 1 | 1 | 1 | 1 |

The examples and comparative examples clearly indicate that adding 10 to 20 wt.-percent of one of the malodor rediction compositions to any of the 1,2-alkanediols removes the rancid odor completely.

It should be noted that the maolodor reduction agents and the compositions comprising them are particularly preferred.

## Claims

1. A mixture comprising or consisting of
(a) at least one 1,2-alkanediol
(b) at least one malodor causing compound existing as an impurity or degradation product within 1,2-alkanediols, and
(c) at least one malodor reduction material having a Malodor Reduction Value (MORV) of at least 0.5, selected from the group consisting of:
((1s,4s)-4- isopropylcyclohexyl)methanol
(-)-alpha-Pinene
(-)-Citroneliol
(+)- alpha-Pinene
(+)-Carvone
(+)-Dihydrocarveol
(1R, 5R)-4,6,6-trimethylbicyclo[3.1.1 ]hept-3-en-2-one
(1s,4s)-4- isopropylcyclohexyl)methanol
(2,2-dimethoxyethyl)benzene
(2Z,5Z)-5,6,7-trimethylocta-2,5-dien-4-one
(2Z,6E)-nona-2,6-dienenitrile
(3Z)-1-(2-buten-1-yloxy)-3-hexene
(d)-Citronelial
(E)-1-(1-methoxypropoxy)hex-3-ene
(E)-1-1-methoxypropoxylhex-3-ene
(E)-3,7-dimethylocta-4,6-dien-3-ol
(E)-4,8-dimethyldeca-4,9-dienal
(E)-4-methyldec-3-en-5-ol
(I)-Citronellal
(R)-(-)-Linalool
(S)-(+)-Linalool
(S)-(1R,5R)-4,6,6-trimethylbicyclo[3.1, 1]hept-3- en-2-one
(Z)-1-((1R,2S)-2,6,6-trimethylcyclohex-3-en--1-yl)but-2-en-1-one
(Z)-1-((2-methylallyl)oxy)hex-3-ene
(Z)-3-dodecenal
(Z)-3-hepten-1-yl acetate
(Z)-3-hexen-1 yl-2 cyclopenten-1 -one
(Z)-5-methylheptan-3-one oxime
(Z)-6-ethylideneoctahydro-2H-5,8-methanochrome
(Z)-dodec-4-enal
(Z)-hex-3-en-1-yl cyclopropanecarboxylate
(Z)-hex-3-en-1-yl isobutyrate
(Z)-hex-3-en-1-yl methyl carbonate
(Z)-hex-3-en-I-yl isobutyrate
1-(3,3-dimethyleyclohexyl)ethyl formate
1,1,2,3,3-Pentamethylindan
1,1-dimethoxynon-2-yne,
1,2-dihydrolinalool
1,2-dimethyl-3-(prop-1-en-2-yl)cyclopentan-1-ol
1,3,3-trimethyl-2- norbornanyl acetate
1,3,5-undecatriene
1,4-cineole
1,8-thiocineol
1',1',5',5'-tetramethylhexahydro-2',5'H-spiro[[1,3]dioxolane-2,8'-[2,4a]methanonaphthalene] K
10-undecenal
1-acetate
1-cyclohexylethyl-(E)-but-2-enoate
1-decanol
1-ethoxy-4-(tert-pentyl)cyclohexane
1-ethyl-3-methoxytricyclo[2,2.1.02,6]heptane
1-Ethyl-3-methoxytricyclo[2.2..1.02,6]heptane
1-hepten-1-ol,
1-limonene
1-methoxy- 3a,4,5,6,7,7a-hexahydro-1 H-4,7-methanoindene
1-oxaspiro(4,5)decan-2-one
1-Phenyl-2-pentanol
1-phenylethyl acetate
2- Heptyl tetrahydrofuran
2- isopropyl-4-methylthiazole
2-(1-ethoxyethoxy)ethyl)benzene
2-(2,2,3-trimethyleyclopent-3-en-1-yl)acetonitrile:
2-(6.6-dimethylbicyclo[3.1.1]hept-2-en-2-yl)acetaldehyde
2-(heptan-3-yl)-1,3-dioxolane
2-(I-ethoxyethoxy)ethyl)benzene
2-(sec-butyl)cyclohexan-1-one
2-(tert-butyl)cyclohexan-1-ol
2-(tert-butyl)cyclohexyl ethyl carbonate
2,2,5-trimetriyl-5-pentylcyc1opentan-1-one
2,4-dimethyl-4-phenyltetrahydrofuran
2,4-Nonadienal
2,5-dimethyl-4-methoxy-3(2H)-ruranone
2,6,10,10-tetramethyl-1-oxaspiro[4,5]dec-6-ene
2,6,6-trimethylester
2,6,9,10-tetramethyl-1-oxaspiro(4.5)deca- 3,6-diene
2,6-dimethyloct-7-en-4-one
2,6-dimethyl-octanal
2,6-nonadien-1-ol
2,6-nonadienal
2-butoxyethanol
2-butyl-4,4,6-trimethyl-1,3-dioxane
2-decenal
2-ethoxy-2,6,6-trimethyl-9-methylenebicyclo[3,3.1]nonane
2-heptyl tetrahydrofuran
2-heptylcyclopentan-1-one
2-hexen-1-ol
2-hexylcyclopent-2-en-1-one
2-hexylcyclopentan-1-one
2-hexylidene cyciopentanone
2-isopropoxyethylbenzene
2-isopropyl-1,7,7-trimethylspiro[bicyclo[2.2.1]heptane- 2,4'-[1,3]dioxane]
2-isopropyl-5-methyl-2-hexenal
2-isopropyl-N,2,3-trimethylbutyramide
2-mercapto-2-methylpentan-1-ol
2-methoxy-3-(1-methylpropyl)pyrazine
2-methoxynaphthalene
2-methyl-4-phenyl-1,3-dioxolane
2-methyldecanenitrile
2-metoxynaphthalene
2-nonanol
2-nonanone
2-nonanone propylene glycol acetal
2-nonen-1-al
2-pentylcyclopentan-1-ol
2-pentylcyclopentan-1-one
2-phenethyl propionate
2-phenoxyethanol
2-phenylethyl acetate
2-propylheptanenitrile
2-trans-6-trans- onadienal
2-undecenal
2-undecenenitrile
3 -(3 isopropylphenyl)butanal
3 -dimethyl-5(2,2,3-trimethyl-3-cyclopenten-Iyl)-4-penten-2-ol
3-(2-ethylphenyl)-2,2-dimethylpropanal
3-(3-isopropylphenyl)butanal
3-(4-methoxyphenyl)-2-methylpropanal
3-(4-methylcyclohex-3-en-1-yl)butanal
3-(p-Isopropylphenyl)propionaldehyde
3,3-dimethyl-3-5(2,2,3-Trimethyl-3 -Cyclopentenyl)-4-penten-2-ol
3,6-nonadien-1-ol
3,6-dimethyl-3-oetanyi acetate
3,7-dimethyl-1-octanol
3,7-dimethyl-2-methylene-6-octenal
3a,4,5,6,7,7a-hexahydro-1H-4,7-methanoinden-6-yl acetate
3-cyclohexene-1-carboxylic acid
3-hexenol
3-hexenyl isovalerate
3-hydroxybutan-2-one
3-methoxy-3-methyl butanol
3-methoxy-7,7-dimethyl-10-methylenebicyclo[4.3.1]decane
3-methyl-1,2-cyclopentanedione
3-methylphenethyl alcohol
3-nonylacrolein
4-(2-methoxypropan-2-yl)-3 -methylcyclohex-1-ene
4-(tert-butyl)cyclohexyl acetate
4-(tert-pentyl)cyclohexan-1-one
4,5,6,7-tetrahydro-3,6-dimethylbenzofuran
4-carvomenthenol
4-cyclohexyl-2-methylbutan-2-ol
4-ethylguaiacol
4-methyl-2-phenyl-3,6-dihydro-2H-pyran
4-methyl-2-phenyltetrahydro-2H-pyran
4-methylquinoline
4-pentenophenone
4-terpinenol
4-tetraethyl-2-phenyl-3,6-dihydro-2H-pyran
4-vinylphenol
5- isopropenyl-2-methyl-2-vinyltetrahydrofuran
5-ethyl-4-hydroxy-2-methylfuran-3(2H)-one
5-methyl-3-heptanone
6,6"dimethyl-2-norpmene-2-propionaidehyde
6,6-dimethoxy-2,5,5-trimethylhex-2-ene
6,6-dimethyl-2-methylenebicyclo [3.1.1 ]heptan-3 -ol
6,6-dimethyl-2-norpinene-2-propionaldehyde
6,8-diethyl-2-nonanol
6-Isopropylquinoline
6-methylquinoline
7-epi-alpha-Selinene
7-epi-sesquithujene
7-isopropyl-8,8-dimethyl-6,10-dioxaspiro[4.5]decane
9-decenal
Acetaldehyde benzyl 2-methoxyethyl acetal
Acetaldehyde dipropyl acetal
Acetaldehyde phenylethyl propyl acetal
Acetate C9
Acetoanisole
Aldehyde C-11
Alloaromadendrene
Allyl 2-(isopentyloxy)acetate
Allyl heptanoate
Allyl phenethyl ether
Allyl-2-(isopentyloxy)acetate
alpha -Thujone
alpha-4-dimethyl benzenepropanal
alpha-Acetoxystyrene
alpha-Amorphene
alpha-Bergamotene
alpha-Cadinene
alpha-Cedrene epoxide
alpha-Cubebene
alpha-Fenchene
alpha-Gurjunene
alpha-Limonene
alpha-methyl cinnamaldehyde
alpha-Methyl-cyclohexanepropanol
alpha-Muurolene
alpha-Patchouiene
alpha-Santalene
alpha-Selinene
alpha-Terpinyl acetate
alpha-Terpinyl propionate
alpha-Thujone
Anethole
Anisyl acetate
Anisyl formate
Benzyl alcohol
Benzyl butyrate
Benzyl dimethyl carbinol
Benzyl isobutyrate
Benzyl isovalerate
Benzyl tert-butanol
Benzylacetone
beta-Caryophyllene
beta-Cedrene
beta-Copaene
beta-Farnesene
beta-Patchoulline
beta-Phellandrene
beta-Pinene
beta-Pinene epoxide
beta-Selinene
beta-Sesquiphellandrene
beta-Terpineol
beta-Terpinyl acetate
Bigarade oxide
Borneol
Bornyl acetate
Bornyl isobutyrate
Camphene
Camphor
Camphorquinone
Capraldehyde
Caproyl alcohol
Caprylic acid
Caprylic alcohol
Caprylnitrile
Carbitol
Carvacrol
Carvone
Chloroxylenol
Cinnamic alcohol
Cinnamic aldehyde
Cinnamyl formate
Cinnamyl nitrile
cis-2-hexenol
cis-3,cis-6-nonadienol
cis-3-hexen-1-ol
cis-3-Hexenyl 2-methylbutyrate
cis-3-hexenyl butyrate
cis-3-hexenyl propionate
cis-3-hexenyl tiglate
cis-3-hexenyl valerate
cis-3-hexenyl-cis-3-hexenoate
cis-4-(tert-butyl)cyclohexyl acetate
cis-4-decen-1-al
cis-6-nonenol
cis-Carveol
cis-Limonene oxide
cis-Ocimene
cis-Pinane
Citral
Citral dimethyl acetal
Citral propylene glycol acetal
Citronellal
Citronellyl formate
Citronellyl nitrile
Cosmene
Creosol
Cumic alcohol
Cyclohexylethyl acetate
Decahydro-3H-spiro[furan-2,5 '-[4,7]methanoindene]
Decahydro-beta-naphthol
Decanal diethyl acetal
Decyl propionate
delta-3-Carene
delta-Elemente
Dibutyl sulfide
Dihydro linalool
Dihydro-alpha-ionone
Dihydro-alpha-terpinyl acetate
Dihydrocarveol
Dihydrocarveol
Dihydrocarveol acetate
Dihydrocarvone
Dihydroisophorone
Dihydrojasmone
Dihydromyrcenol
Diphenylmethane
Diphenyloxide
d-Limonene
Dodecanal dimethyl acetal
d-p-8(9)-Menthen-2-one
Ethyl (1R, 6S)-2,2,6-trimethylcyclohexane-1-carboxylate
Ethyl (3aR,4S,7R,7aR)-octahydro-3aH-4,7-methanoindene-3a-carboxylate
Ethyl 2-(cyclohexyl)propionate
Ethyl 2-ethyl-6,6-dimethylcyclohex-2-ene-1-carboxylate
Ethyl decanoate
Ethyl gamma-safranate
Ethyl hexyl ketone
Ethyl nonanoate
Ethyl octanoate
Ethyl-(1R,2R,3R,4R)-3-isopropylbicyclo[2.2.1]hept-5-ene-2-carboxylate
Eucalyptol
Fenchyl alcohol
Furforyl hexanoate
gamma-Cadinene
gamma-Himachalene
gamma-Muurolene
gamma-Terpeninyl acetate
gamma-Terpinene
gamma-Terpineol
Geranial
Geranyl formate
Geranyl nitriie
Gyrane
Heliotropin
Heptaldehyde
Heptyl acetate
Heptyl alcohol
Hexenyl tiglate
Hexyl 2-methylbutanoate
Hexyl butyrate
Hexyl hexanoate
Hexyl propanoate
Hexyl tiglate
Hydratopic alcohol
Hydratropaldehyde dimethyl acetal
Hydrocinnamyl alcohol
Hydrotropaldehyde dimethyl acetal
i-(3,3-dimethylcyclohexyl)ethyl acetate
Irisnitrile
Isoamyl isobutyrate
Isoamyl octanoate
Isoborneol
Isobornyl propionate
Isobornyl acetate
Isobornyl isobutyrate
Isocyclocitral
Isomenthone
Isononanol
Isononyl acetate
Isopentyrate
Isopropyl 2-methylbiityrate
Isopropylvinylcarbinol
Isopulegol
L- Carvone
Lauraldehyde
Leaf acetal
Linalool oxide (furanoid)
Linalyl formate
Linalyl isobutyrate
Linalyl propionate
Maceal
Menthone
Methoxycitronellal
Methoxycyclododecane
Methoxymelonat
Methyl (1s,4s)-1,4-dimethylcyclohexane-1-carboxylate
Methyl (E)-non-2-enoate
Methyl (E)-octa-4,7-dienoate
Methyl 2-methylbutyrate
Methyl 2-octynoate
Methyl alpha-cyclogeranate
Methyl chavicol
Methyl cinnamate
Methyl cyclopentylideneacetate
Methyl diphenyl ether
Methyl eugenol
Methyl geraniate
Methyl isoeugenol
Methyl isoeugenol
Methyl nonyl acetaldehyde
Methyl nonyl acetaldehyde dimethyl acetal
Methyl nonyl ketone
Methyl octine carbonate
Methyl octyl acetaldehyde
Methyl phenyl carbinyl propionate
Methyl phenylacetate
Methyl phenylethyl carbinol
Methyl-2,2-dimethyl-6-methylenecyclohexane-1-carboxylate
Methyl-2-methylbutyrate
Myrcene
Myrcenol
Myrcenyl acetate
Myroxide
Myrtenal
Nerol
Neryl Formate
n-Hexyl-2-butenoate
Nonaldehyde
Nonyl alcohol
O -Methyl linalool
Ocimenol
Octahydro-1H-4,7-methanoinden-5-yl acetate
Octahydro-1H-4,7-methanoindene-1-carbaldehyde
Octanal
Octanal dimethyl acetal
Octanal propylene glycol acetal
Octyl acetate
para-Cymen-8-ol
p-cresyl isobutyrate
p-Cymene
Perillaldehyde
Perillyl acetate
Phenethyl alcohol
Phenethyl formate
Phenylacetaldehyde ethyleneglycol acetal
p-Isopropylphenylacetaldehyde
p-Menth-3-en-1-ol
p-Propyl anisole
Propyl (S)-2-(tert-pentyloxy)propanoate
Propylene glycol
p-t-butyl phenyl acetaldehyde
p-tert-Amyl cyclohexanol
Racemic alpha-Pinene
Rhodinol
Sabinene
Sabinene hydrate
Sabinol
Safrole
Selina-3,7(11)-diene
Spirodecane
Tetrahydrogeranial
Tetrahydrojasmone
Tetrahydrolinalool
Tetrahydrolmalyl acetate
Thujopsene
Thymol
Thymol methyl ether
trans,trans-2,4-nonadienal
trans-2,cis-6-nonadienal
trans-2-decenal
trans-2-hexenol
trans-2-nonen-1-al
trans-2-tert-butylcyclohexanol
trans-3, cis-6-nonadienol
trans-4-Decen-1-al
trans-Anethole
trans-beta-ocimene
trans-beta-ociraene
trans-Dihydrocarvone
trans-Geraniol
Undecanal
Valencene

2. The mixture of Claim 1 wherein the 1,2-alkanediols (component a) are selected from the group consisting of 1,2-pentanediol, 1,2-hexanediol, 1,2-heptanediol, 1,2-octanediol, 1,2-nonanediol, 1,2-decanediol, 1,2-undecanediol, 1,2-dodecandiole and their mixtures.

3. The mixture of Claim 2, wherein said 1,2-alkanediol is selected from 1,2-hexanediol, 1,2-octanediol and mixtures thereof.

4. The mixture of Claim 1 wherein said malodor causing compounds (component b) are selected from the group consisting of
(b1) butyric acid, valeric acid, caproic acid, enanthic acid, caprylic acid, pelargonic acid, capric acid, and methyl-, ethyl- esters thereof. 1-hexene-3-one, 1-heptene-3-one, 1-octene-3-one, 1-nonene-3-one, 1-decene-3-one, octalactone, 1-hexene, 1-heptene, 1-octene, 1-nonene, 1-decene, 2-ethyl-1-butene, 2-ethyl-1-pentene, 2-ethyl-1-hexene, 2-ethyl-1-heptene, 2-ethyl-1-octene, cis-3-methyl-2-pentene, cis-3-methyl-2-hexene, cis-3-methyl-2-heptene, cis-3-methyl-2-octene, cis-3-methyl-2-nonene, trans-3-methyl-2-pentene, trans-3-methyl-2-hexene, trans-3-methyl-2-heptene, trans-3-methyl-2-octene, trans-3-methyl-2-nonene, n-hexane, n-heptane, n-octane, n-nonane, n-decane, cis-2-hexene, cis-2-heptene, cis-2-octene, cis-2-nonene, cis-2-decene, trans-2-hexene, trans-2-heptene, trans-2-octene, trans-2-nonene, trans-2-decene, cis-3-hexene, cis-3-heptene, cis-3-octene, cis-3-nonene, cis-3-decene, trans-3-hexene, trans-3-heptene, trans-3-octene, trans-3-nonene, trans-3-decene, cis-2,3-hexanediol, cis-2,3-heptanediol, cis-2,3-octanediol, cis-2,3-nonenediol, cis-2,3-decanediol, trans-2,3-hexanediol, trans-2,3-heptanediol, trans-2,3-octanediol, trans-2,3-nonenediol, trans-2,3-decanediol, cis-3,4-hexanediol, cis-3,4-heptanediol, cis-3,4-octanediol, cis-3,4-nonenediol, cis-3,4-decanediol, trans-3,4-hexanediol, trans-3,4-heptanediol, trans-3,4-octanediol, trans-3,4-nonenediol, trans-3,4-decanediol, 3-methylpentane-2,3-diol, 3-methylhexane-2,3-diol, 3-methylheptane-2,3-diol, 3-methyloctane-2,3-diol, 3-methylnonane-2,3-diol, 2-ethylbutane-1,2-diol, 2-ethylpentane-1,2-diol, 2-ethylhexane-1,2-diol, 2-ethylheptane-1,2-diol, 2-ethyloctane-1,2-diol, or mixtures thereof, or
(b2) or one, two, three or more compounds according to one of the following formulas (I) to (IV) 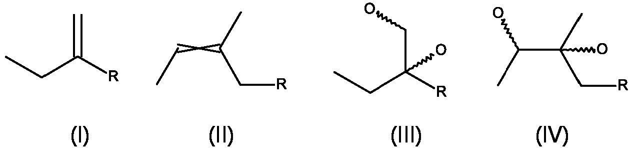 in which R stands for a linear branched alkyl radical having 1 to 10 carbon atoms, including all cis and trans stereoisomers, and

5. The mixture of Claim 1, wherein the components (a) and (c) are present in a ratio by weight of from about 90:10 to about 99.99:0.01 and wherein component (b) is present in an amount of from about 10 ppm to about 60.000 ppm (0,001 -to 6 wt.-percent)

6. The mixture of Claim 1, wherein component (b) is present in an amount of from about 50 ppm to about 20.000 ppm (0,005 to 2 wt.-percent).

7. The mixture of Claim 1, wherein component (b) is present in an amount of from about 100 ppm to about 10.000 ppm (0,01 to 1 wt.-percent).

8. The mixture of Claim 1, further comprising a liquid carrier.

9. The mixture of Claim 8, wherein said liquid carrier is selected from the group consisting of water, C₁-C₄ aliphatic alcohols, and polyols having 2 to 12 carbon atoms and 1 to 5 hydroxyl groups.

10. A consumer product comprising the composition of Claim 1.

11. The product of Claim 10, representing a cosmetic composition, a pharmaceutical composition, an oral composition, a detergent composition or a food composition.

12. The product of Claim 10, comprising said mixture of Claim 1 in an amount of from about 0.0001 to about 10 wt.-percent - calculated on the composition.

13. The product of Claim 10, comprising said mixture of Claim 1 in an amount of from about 0.005 to about 5 wt.-percent - calculated on the composition.

14. The product of Claim 10, comprising said mixture of Claim 1 in an amount of from about 0.01 to about 3 wt.-percent - calculated on the composition.

15. A method for masking the unpleasant odour of 1,2-alkanediols or products comprising said 1,2-alkanediols comprising or consisting of the following steps:
(a) providing a source of at least one 1,2-alkanediol with an unpleasant odour or a product comprising said at least one 1,2-alkanediol with an unpleasant odour; and
(b) adding at least one malodor reduction material having a Malodor Reduction Value (MORV) of at least 0.5 according to Claim 1.

16. The use of at least one malodor reduction material having a Malodor Reduction Value (MORV) of at least 0.5 according to Claim 1 for masking the unpleasant odor of 1,2-alkanediols or products comprising them.

## Patentansprüche

1. Mischung, umfassend oder bestehend aus
(a) mindestens einem 1,2-Alkandiol
(b) mindestens einer geruchsverursachenden Verbindung, die als Verunreinigung oder Abbauprodukt in 1,2-Alkandiolen vorliegt, und
(c) mindestens ein geruchsreduzierendes Material mit einem Geruchsreduktionswert (MORV) von mindestens 0,5, ausgewählt aus der Gruppe bestehend aus:
((1s,4s)-4- isopropylcyclohexyl)methanol
(-)-alpha-Pinene
(-)-Citroneliol
(+)- alpha-Pinene
(+)-Carvone
(+)-Dihydrocarveol
(1R, 5R)-4,6,6-trimethylbicyclo[3.1.1]hept-3-en-2-one
(1s,4s)-4- isopropylcyclohexyl)methanol
(2,2-dimethoxyethyl)benzene
(2Z,5Z)-5,6,7-trimethylocta-2,5-dien-4-one
(2Z,6E)-nona-2,6-dienenitrile
(3Z)-1-(2-buten-1-yloxy)-3-hexene
(d)-Citronelial
(E)-1-(1-methoxypropoxy)hex-3-ene
(E)-1-1-methoxypropoxylhex-3-ene
(E)-3,7-dimethylocta-4,6-dien-3-ol
(E)-4,8-dimethyldeca-4,9-dienal
(E)-4-methyldec-3-en-5-ol
(I)-Citronellal
(R)-(-)-Linalool
(S)-(+)-Linalool
(S)-(1R,5R)-4,6,6-trimethylbicyclo[3.1, 1]hept-3- en-2-one
(Z)-1-((1R,2S)-2,6,6-trimethylcyclohex-3-en--1-yl)but-2-en-1-one
(Z)-1-((2-methylallyl)oxy)hex-3-ene
(Z)-3-dodecenal
(Z)-3-hepten-1-yl acetate
(Z)-3-hexen-1 yl-2 cyclopenten-1 -one
(Z)-5-methylheptan-3-one oxime
(Z)-6-ethylideneoctahydro-2H-5,8-methanochrome
(Z)-dodec-4-enal
(Z)-hex-3-en-1-yl cyclopropanecarboxylate
(Z)-hex-3-en-1-yl isobutyrate
(Z)-hex-3-en-1-yl methyl carbonate
(Z)-hex-3-en-I-yl isobutyrate
1-(3,3-dimethyleyclohexyl)ethyl formate
1,1,2,3,3-Pentamethylindan
1,1-dimethoxynon-2-yne,
1,2-dihydrolinalool
1,2-dimethyl-3-(prop-1-en-2-yl)cyclopentan-1-ol
1,3,3-trimethyl-2- norbornanyl acetate
1,3,5-undecatriene
1,4-cineole
1,8-thiocineol
1',1',5',5'-tetramethylhexahydro-2',5'H-spiro[[1,3]dioxolane-2,8'-[2,4a]methanonaphthalene] K
10-undecenal
1-acetate
1-cyclohexylethyl-(E)-but-2-enoate
1-decanol
1-ethoxy-4-(tert-pentyl)cyclohexane
1-ethyl-3-methoxytricyclo[2,2.1.02,6]heptane
1-Ethyl-3-methoxytricyclo[2.2..1.02,6]heptane
1-hepten-1-ol,
1-limonene
1-methoxy- 3a,4,5,6,7,7a-hexahydro-1H-4,7-methanoindene
1-oxaspiro(4,5)decan-2-one
1-Phenyl-2-pentanol
1-phenylethyl acetate
2- Heptyl tetrahydrofuran
2- isopropyl-4-methylthiazole
2-(1-ethoxyethoxy)ethyl)benzene
2-(2,2,3-trimethyleyclopent-3-en-1-yl)acetonitrile:
2-(6.6-dimethylbicyclo[3.1.1]hept-2-en-2-yl)acetaldehyde
2-(heptan-3-yl)-1,3-dioxolane
2-(I-ethoxyethoxy)ethyl)benzene
2-(sec-butyl)cyclohexan-1-one
2-(tert-butyl)cyclohexan-1-ol
2-(tert-butyl)cyclohexyl ethyl carbonate
2,2,5-trimetriyl-5-pentylcyc1opentan-1-one
2,4-dimethyl-4-phenyltetrahydrofuran
2,4-Nonadienal
2,5-dimethyl-4-methoxy-3(2H)-ruranone
2,6,10,10-tetramethyl-1-oxaspiro[4,5]dec-6-ene
2,6,6-trimethylester
2,6,9,10-tetramethyl-1-oxaspiro(4.5)deca- 3,6-diene
2,6-dimethyloct-7-en-4-one
2,6-dimethyl-octanal
2,6-nonadien-1-ol
2,6-nonadienal
2-butoxyethanol
2-butyl-4,4,6-trimethyl-I,3-dioxane
2-decenal
2-ethoxy-2,6,6-trimethyl-9-methylenebicyclo[3,3.1]nonane
2-heptyl tetrahydrofuran
2-heptylcyclopentan-1-one
2-hexen-1-ol
2-hexylcyclopent-2-en-1-one
2-hexylcyclopentan-1-one
2-hexylidene cyciopentanone
2-isopropoxyethylbenzene
2-isopropyl-1,7,7-trimethylspiro[bicyclo[2.2.1]heptane- 2,4'-[1,3]dioxane]
2-isopropyl-5-methyl-2-hexenal
2-isopropyl-N,2,3-trimethylbutyramide
2-mercapto-2-methylpentan-1-ol
2-methoxy-3-(1-methylpropyl)pyrazine
2-methoxynaphthalene
2-methyl-4-phenyl-1,3-dioxolane
2-methyldecanenitrile
2-metoxynaphthalene
2-nonanol
2-nonanone
2-nonanone propylene glycol acetal
2-nonen-1-al
2-pentylcyclopentan-1-ol
2-pentylcyclopentan-1-one
2-phenethyl propionate
2-phenoxyethanol
2-phenylethyl acetate
2-propylheptanenitrile
2-trans-6-trans- onadienal
2-undecenal
2-undecenenitrile
3 -(3 isopropylphenyl)butanal
3 -dimethyl-5(2,2,3-trimethyl-3-cyclopenten-lyl)-4-penten-2-ol
3-(2-ethylphenyl)-2,2-dimethylpropanal
3-(3-isopropylphenyl)butanal
3-(4-methoxyphenyl)-2-methylpropanal
3-(4-methylcyclohex-3-en-1-yl)butanal
3-(p-Isopropylphenyl)propionaldehyde
3,3-dimethyl-3-5(2,2,3-Trimethyl-3 -Cyclopentenyl)-4-penten-2-ol
3,6-nonadien-1-ol
3,6-dimethyl-3-oetanyi acetate
3,7-dimethyl-1-octanol
3,7-dimethyl-2-methylene-6-octenal
3a,4,5,6,7,7a-hexahydro-1H-4,7-methanoinden-6-yl acetate
3-cyclohexene-1-carboxylic acid
3-hexenol
3-hexenyl isovalerate
3-hydroxybutan-2-one
3-methoxy-3-methyl butanol
3-methoxy-7,7-dimethyl-10-methylenebicyclo[4.3.1]decane
3-methyl-1,2-cyclopentanedione
3-methylphenethyl alcohol
3-nonylacrolein
4-(2-methoxypropan-2-yl)-3 -methylcyclohex-1-ene
4-(tert-butyl)cyclohexyl acetate
4-(tert-pentyl)cyclohexan-1-one
4,5,6,7-tetrahydro-3,6-dimethylbenzofuran
4-carvomenthenol
4-cyclohexyl-2-methylbutan-2-ol
4-ethylguaiacol
4-methyl-2-phenyl-3,6-dihydro-2H-pyran
4-methyl-2-phenyltetrahydro-2H-pyran
4-methylquinoline
4-pentenophenone
4-terpinenol
4-tetraethyl-2-phenyl-3,6-dihydro-2H-pyran
4-vinylphenol
5- isopropenyl-2-methyl-2-vinyltetrahydrofuran
5-ethyl-4-hydroxy-2-methylfuran-3(2H)-one
5-methyl-3-heptanone
6,6"dimethyl-2-norpmene-2-propionaidehyde
6,6-dimethoxy-2,5,5-trimethylhex-2-ene
6,6-dimethyl-2-methylenebicyclo [3.1.1 ]heptan-3 -ol
6,6-dimethyl-2-norpinene-2-propionaldehyde
6,8-diethyl-2-nonanol
6-Isopropylquinoline
6-methylquinoline
7-epi-alpha-Selinene
7-epi-sesquithujene
7-isopropyl-8,8-dimethyl-6,10-dioxaspiro[4.5]decane
9-decenal
Acetaldehyde benzyl 2-methoxyethyl acetal
Acetaldehyde dipropyl acetal
Acetaldehyde phenylethyl propyl acetal
Acetate C9
Acetoanisole
Aldehyde C-11
Alloaromadendrene
Allyl 2-(isopentyloxy)acetate
Allyl heptanoate
Allyl phenethyl ether
Allyl-2-(isopentyloxy)acetate
alpha -Thujone
alpha-4-dimethyl benzenepropanal
alpha-Acetoxystyrene
alpha-Amorphene
alpha-Bergamotene
alpha-Cadinene
alpha-Cedrene epoxide
alpha-Cubebene
alpha-Fenchene
alpha-Gurjunene
alpha-Limonene
alpha-methyl cinnamaldehyde
alpha-Methyl-cyclohexanepropanol
alpha-Muurolene
alpha-Patchouiene
alpha-Santalene
alpha-Selinene
alpha-Terpinyl acetate
alpha-Terpinyl propionate
alpha-Thujone
Anethole
Anisyl acetate
Anisyl formate
Benzyl alcohol
Benzyl butyrate
Benzyl dimethyl carbinol
Benzyl isobutyrate
Benzyl isovalerate
Benzyl tert-butanol
Benzylacetone
beta-Caryophyllene
beta-Cedrene
beta-Copaene
beta-Farnesene
beta-Patchoulline
beta-Phellandrene
beta-Pinene
beta-Pinene epoxide
beta-Selinene
beta-Sesquiphellandrene
beta-Terpineol
beta-Terpinyl acetate
Bigarade oxide
Borneol
Bornyl acetate
Bornyl isobutyrate
Camphene
Camphor
Camphorquinone
Capraldehyde
Caproyl alcohol
Caprylic acid
Caprylic alcohol
Caprylnitrile
Carbitol
Carvacrol
Carvone
Chloroxylenol
Cinnamic alcohol
Cinnamic aldehyde
Cinnamyl formate
Cinnamyl nitrile
cis-2-hexenol
cis-3,cis-6-nonadienol
cis-3-hexen-1-ol
cis-3-Hexenyl 2-methylbutyrate
cis-3-hexenyl butyrate
cis-3-hexenyl propionate
cis-3-hexenyl tiglate
cis-3-hexenyl valerate
cis-3-hexenyl-cis-3-hexenoate
cis-4-(tert-butyl)cyclohexyl acetate
cis-4-decen-1-al
cis-6-nonenol
cis-Carveol
cis-Limonene oxide
cis-Ocimene
cis-Pinane
Citral
Citral dimethyl acetal
Citral propylene glycol acetal
Citronellal
Citronellyl formate
Citronellyl nitrile
Cosmene
Creosol
Cumic alcohol
Cyclohexylethyl acetate
Decahydro-3H-spiro[furan-2,5'-[4,7]methanoindene]
Decahydro-beta-naphthol
Decanal diethyl acetal
Decyl propionate
delta-3-Carene
delta-Elemente
Dibutyl sulfide
Dihydro linalool
Dihydro-alpha-ionone
Dihydro-alpha-terpinyl acetate
Dihydrocarveol
Dihydrocarveol
Dihydrocarveol acetate
Dihydrocarvone
Dihydroisophorone
Dihydrojasmone
Dihydromyrcenol
Diphenylmethane
Diphenyloxide
d-Limonene
Dodecanal dimethyl acetal
d-p-8(9)-Menthen-2-one
Ethyl (1R, 6S)-2,2,6-trimethylcyclohexane-1-carboxylate
Ethyl (3aR,4S,7R,7aR)-octahydro-3aH-4,7-methanoindene-3a-carboxylate
Ethyl 2-(cyclohexyl)propionate
Ethyl 2-ethyl-6,6-dimethylcyclohex-2-ene-1-carboxylate
Ethyl decanoate
Ethyl gamma-safranate
Ethyl hexyl ketone
Ethyl nonanoate
Ethyl octanoate
Ethyl-(1R,2R,3R,4R)-3-isopropylbicyclo[2.2.1]hept-5-ene-2-carboxylate
Eucalyptol
Fenchyl alcohol
Furforyl hexanoate
gamma-Cadinene
gamma-Himachalene
gamma-Muurolene
gamma-Terpeninyl acetate
gamma-Terpinene
gamma-Terpineol
Geranial
Geranyl formate
Geranyl nitriie
Gyrane
Heliotropin
Heptaldehyde
Heptyl acetate
Heptyl alcohol
Hexenyl tiglate
Hexyl 2-methylbutanoate
Hexyl butyrate
Hexyl hexanoate
Hexyl propanoate
Hexyl tiglate
Hydratopic alcohol
Hydratropaldehyde dimethyl acetal
Hydrocinnamyl alcohol
Hydrotropaldehyde dimethyl acetal
i-(3,3-dimethylcyclohexyl)ethyl acetate
Irisnitrile
Isoamyl isobutyrate
Isoamyl octanoate
Isoborneol
Isobornyl propionate
Isobornyl acetate
Isobornyl isobutyrate
Isocyclocitral
Isomenthone
Isononanol
Isononyl acetate
Isopentyrate
Isopropyl 2-methylbiityrate
Isopropylvinylcarbinol
Isopulegol
L- Carvone
Lauraldehyde
Leaf acetal
Linalool oxide (furanoid)
Linalyl formate
Linalyl isobutyrate
Linalyl propionate
Maceal
Menthone
Methoxycitronellal
Methoxycyclododecane
Methoxymelonat
Methyl (1s,4s)-1,4-dimethylcyclohexane-1-carboxylate
Methyl (E)-non-2-enoate
Methyl (E)-octa-4,7-dienoate
Methyl 2-methylbutyrate
Methyl 2-octynoate
Methyl alpha-cyclogeranate
Methyl chavicol
Methyl cinnamate
Methyl cyclopentylideneacetate
Methyl diphenyl ether
Methyl eugenol
Methyl geraniate
Methyl isoeugenol
Methyl isoeugenol
Methyl nonyl acetaldehyde
Methyl nonyl acetaldehyde dimethyl acetal
Methyl nonyl ketone
Methyl octine carbonate
Methyl octyl acetaldehyde
Methyl phenyl carbinyl propionate
Methyl phenylacetate
Methyl phenylethyl carbinol
Methyl-2,2-dimethyl-6-methylenecyclohexane-1-carboxylate
Methyl-2-methylbutyrate
Myrcene
Myrcenol
Myrcenyl acetate
Myroxide
Myrtenal
Nerol
Neryl Formate
n-Hexyl-2-butenoate
Nonaldehyde
Nonyl alcohol
O -Methyl linalool
Ocimenol
Octahydro-1H-4,7-methanoinden-5-yl acetate
Octahydro-1H-4,7-methanoindene-1-carbaldehyde
Octanal
Octanal dimethyl acetal
Octanal propylene glycol acetal
Octyl acetate
para-Cymen-8-ol
p-cresyl isobutyrate
p-Cymene
Perillaldehyde
Perillyl acetate
Phenethyl alcohol
Phenethyl formate
Phenylacetaldehyde ethyleneglycol acetal
p-Isopropylphenylacetaldehyde
p-Menth-3-en-1-ol
p-Propyl anisole
Propyl (S)-2-(tert-pentyloxy)propanoate
Propylene glycol
p-t-butyl phenyl acetaldehyde
p-tert-Amyl cyclohexanol
Racemic alpha-Pinene
Rhodinol
Sabinene
Sabinene hydrate
Sabinol
Safrole
Selina-3,7(11)-diene
Spirodecane
Tetrahydrogeranial
Tetrahydrojasmone
Tetrahydrolinalool
Tetrahydrolmalyl acetate
Thujopsene
Thymol
Thymol methyl ether
trans,trans-2,4-nonadienal
trans-2,cis-6-nonadienal
trans-2-decenal
trans-2-hexenol
trans-2-nonen-1-al
trans-2-tert-butylcyclohexanol
trans-3, cis-6-nonadienol
trans-4-Decen-1-al
trans-Anethole
trans-beta-ocimene
trans-beta-ociraene
trans-Dihydrocarvone
trans-Geraniol
Undecanal .

2. Mischung nach Anspruch 1, wobei die 1,2-Alkandiole (Komponente a) ausgewählt sind aus der Gruppe bestehend aus 1,2-Pentandiol, 1,2-Hexandiol, 1,2-Heptandiol, 1,2-Octandiol, 1,2-Nonandiol, 1,2-Decandiol, 1,2-Undecandiol, 1,2-Dodecandiol und deren Gemischen.

3. Mischung nach Anspruch 2, wobei das 1,2-Alkandiol aus 1,2-Hexandiol, 1,2-Octandiol und Gemischen davon ausgewählt ist.

4. Mischung nach Anspruch 1, wobei die geruchsverursachenden Verbindungen (Komponente b) ausgewählt sind aus der Gruppe bestehend aus
(b1) Buttersäure, Valeriansäure, Capronsäure, Enanthsäure, Caprylsäure, Pelargonsäure, Caprinsäure und Methyl-, Ethylester davon. 1-Hexen-3-on, 1-Hepten-3-on, 1-Octen-3-on, 1-Nonen-3-on, 1-Decen-3-on, Octalacton, 1-Hexen, 1-Hepten, 1-Octen, 1-Nonen, 1-Decen, 2-Ethyl-1-buten, 2-Ethyl-1-penten, 2-Ethyl-1-hexen, 2-Ethyl-1-hepten, 2-Ethyl-1-octen, cis-3-Methyl-2-penten, cis-3-Methyl-2-hexen, cis-3-Methyl-2-hepten, cis-3-Methyl-2-octen, cis-3-Methyl-2-nonen, trans-3-Methyl-2-penten, trans-3-Methyl-2-hexen, trans-3-Methyl-2-hepten, trans-3-Methyl-2-octen, trans-3-Methyl-2-nonen, n-Hexan, n-Heptan, n-Octan, n-Nonan, n-Decan, cis-2-hexen, cis-2-hepten, cis-2-octen, cis-2-Nonen, cis-2-Decen, trans-2-Hexen, trans-2-Hepten, trans-2-Octen, trans-2-Nonen, trans-2-Decen, cis-3-Hexen, cis-3-Hepten, cis-3-Octen, cis-3-Nonen, cis-3-Decen, trans-3-Hexen, trans-3-Hepten, trans-3-Octen, trans-3-Nonen, trans-3-Decen, cis-2,3-Hexandiol, cis-2,3-Heptandiol, cis-2,3-Octandiol, cis-2,3-Nonendiol, cis-2,3-Decandiol, trans-2,3-Hexandiol, trans-2,3-Heptandiol, trans-2,3-Octandiol, trans-2,3-Nonendiol, trans-2,3-Decandiol, cis-3,4-Hexandiol, cis-3,4-Heptandiol, cis-3,4-Octandiol, cis-3,4-Nonendiol, cis-3,4-Decandiol, trans-3,4-Hexandiol, trans-3,4-Heptandiol, trans-3,4-Octandiol, trans-3,4-Nonendiol, trans-3,4-Decandiol, 3-Methylpentan-2,3-diol, 3-Methylhexan-2,3-diol, 3-Methylheptan-2,3-diol, 3-Methyloctan-2,3-diol, 3-Methylnonan-2,3-diol, 2-Ethylbutan-1,2-diol, 2-Ethylpentan-1,2-diol, 2-Ethylhexan-1,2-diol, 2-Ethylheptan-1,2-diol, 2-Ethyloctan-1,2-diol oder Gemische davon, oder
(b2) oder eine, zwei, drei oder mehr Verbindungen gemäß einer der folgenden Formeln (I) bis (IV) 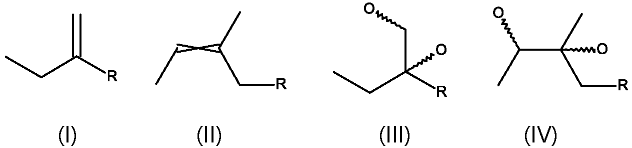 in der R für einen linearen verzweigten Alkylrest mit 1 bis 10 Kohlenstoffatomen, einschließlich aller cis- und trans-Stereoisomere, sowie deren Mischungen steht.

5. Mischung nach Anspruch 1, wobei die Komponenten (a) und (c) in einem Gewichtsverhältnis von etwa 90:10 bis etwa 99,99:0,01 vorliegen und wobei die Komponente (b) in einer Menge von etwa 10 ppm bis etwa 60.000 ppm (0,001 - bis 6 Gew.-%) vorliegt

6. Mischung nach Anspruch 1, wobei die Komponente (b) in einer Menge von etwa 50 ppm bis etwa 20.000 ppm (0,005 bis 2 Gew.-%) vorhanden ist.

7. Mischung nach Anspruch 1, wobei die Komponente (b) in einer Menge von etwa 100 ppm bis etwa 10.000 ppm (0,01 bis 1 Gew.-%) vorhanden ist.

8. Mischung nach Anspruch 1, ferner umfassend einen flüssigen Träger

9. Mischung nach Anspruch 8, wobei der flüssige Träger ausgewählt ist aus der Gruppe bestehend aus Wasser, aliphatischen C₁-C₄-Alkoholen und Polyolen mit 2 bis 12 Kohlenstoffatomen und 1 bis 5 Hydroxylgruppen

10. Verbraucherprodukt, umfassend die Zusammensetzung nach Anspruch 1.

11. Produkt nach Anspruch 10, das eine kosmetische Zusammensetzung, eine pharmazeutische Zusammensetzung, eine orale Zusammensetzung, eine Waschmittelzusammensetzung oder eine Lebensmittelzusammensetzung darstellt.

12. Produkt nach Anspruch 10, enthaltend die Mischung nach Anspruch 1 in einer Menge von etwa 0,0001 bis etwa 10 Gew.-% - berechnet auf die Zusammensetzung.

13. Produkt nach Anspruch 10, umfassend die Mischung nach Anspruch 1 in einer Menge von etwa 0,005 bis etwa 5 Gew.-% - berechnet auf die Zusammensetzung.

14. Produkt nach Anspruch 10, enthaltend die Mischung nach Anspruch 1 in einer Menge von etwa 0,01 bis etwa 3 Gew.-% - bezogen auf die Zusammensetzung.

15. Verfahren zur Maskierung des unangenehmen Geruchs von 1,2-Alkandiolen oder Produkten, die diese 1,2-Alkandiole enthalten, umfassend oder bestehend aus den folgenden Schritten:
(a) Bereitstellen einer Quelle für mindestens ein 1,2-Alkandiol mit unangenehmem Geruch oder eines Produkts, das das mindestens eine 1,2-Alkandiol mit unangenehmem Geruch enthält; und
(b) Zugabe mindestens eines Geruchsreduktionsmaterials mit einem Geruchsreduktionswert (MORV) von mindestens 0,5 gemäß Anspruch 1.

16. Verwendung mindestens eines Geruchsminderungsmaterials mit einem Geruchsminderungswert (MORV) von mindestens 0,5 nach Anspruch 1 zur Maskierung des unangenehmen Geruchs von 1,2-Alkandiolen oder diese enthaltenden Produkte.

## Revendications

1. Mélange comprenant ou consistant en
(a) au plus tard d'un 1,2-Alkandiol
(b) d'au moins un composé générateur d'odeur présent sous forme d'impureté ou de produit de dégradation dans les 1,2-alcanediols, et
(c) au moins un matériau à teneur réduite en germes avec un pouvoir de réduction des germes (MORV) d'au moins 0,5, provenant du groupe en question :
((1s,4s)-4- isopropylcyclohexyl)méthanol
(-)-alpha-Pinène
(-)-Citroneliol
(+)- alpha-Pinène
(+)-carvone
(+)-Dihydrocarvéol
(1R, 5R)-4,6,6-triméthylbicyclo[3.1.1 ]hept-3-en-2-one
(1s,4s)-4- isopropylcyclohexyl)méthanol
(2,2-diméthoxyéthyl)benzène
(2Z,5Z)-5,6,7-trimethylocta-2,5-dien-4-one
(2Z,6E)-nona-2,6-diénonitrile
(3Z)-1-(2-buten-1-yloxy)-3-hexene
(d)-Citronelial
(E)-1-(1-méthoxypropoxy)hex-3-ène
(E)-1-1-méthoxypropoxylhex-3-ène
(E)-3,7-dimethylocta-4,6-dien-3-ol
(E)-4,8-diméthyldéca-4,9-diénal
(E)-4-methyldec-3-en-5-ol
(I)-Citronellal
(R)-(-)-Linalol
(S)-(+)-Linalol
(S)-(1R,5R)-4,6,6-triméthylbicyclo[3.1, 1]hept-3- en-2-one
(Z)-1-((1R,2S)-2,6,6-trimethylcyclohex-3-en--1-yl)but-2-en-1-one
(Z)-1-((2-méthylallyl)oxy)hex-3-ène
(Z)-3-dodécénal
Acétate **de** (Z)-3-heptène-1-yl
(Z)-3-hexène-1 yl-2 cyclopentène-1 -one
(Z)-5-méthylheptan-3-one oxime
(Z)-6-éthylidèneoctahydro-2H-5,8-méthanochrome
(Z)-dodéc-4-énal
(Z)-hex-3-én-1-yl cyclopropanecarboxylate
(Z)-hex-3-en-1-yl isobutyrate
Carbonate de méthyle (Z)-hex-3-en-1-yl
Isobutyrate de (Z)-hex-3-en-I-yl
Formiate **de** 1-(3,3-diméthylécyclohexyle)éthyle
1,1,2,3,3-Pentaméthylindane
1,1-diméthoxynon-2-yne,
1,2-dihydrolinalool
1,2-dimethyl-3-(prop-1-en-2-yl)cyclopentan-1-ol
Acétate **de** 1,3,3-triméthyl-2-norbornanyle
1,3,5-undécatriène
1,4-cinéole
1,8-thiocinéol
1',1',5',5'-tétraméthylhexahydro-2',5'H-spiro[[1,3]dioxolane-2,8'-[2,4a]méthanonaphtalène]]. K
10-undécénal
1-acétate
1-cyclohexyléthyle-(E)-but-2-énoate
1-décanol
1-éthoxy-4-(tert-pentyl)cyclohexane
1-ethyl-3-methoxytricyclo[2,2.1.02,6]heptane
1-Ethyl-3-methoxytricyclo[2.2..1.02,6]heptane
1-heptène-1-ol,
1-limonène
1-methoxy- 3a,4,5,6,7,7a-hexahydro-1H-4,7-methanoindene
1-oxaspiro(4,5)décan-2-one
1-Phényl-2-pentanol
Acétate de phényléthyle
2- tétrahydrofurane d'héptyle
2- Isopropyl-4-méthylthiazole
2-(1-éthoxyéthoxy)éthyl)benzène
2-(2,2,3-triméthyleyclopent-3-en-1-yl)acétonitrile :
2-(6,6-diméthylbicyclo[3.1.1]hept-2-en-2-yl)acétaldéhyde
2-(heptan-3-yl)-1,3-dioxolane
2-(I-éthoxyéthoxy)éthyl)benzène
2-(sec-butyl)cyclohexan-1-one
2-(tert-butyl)cyclohexan-1-ol
Carbonate de 2-(tert-butyl)cyclohexyle et d'éthyle
2,2,5-trimétriyl-5-pentylcyc1opentan-1-one
2,4-diméthyl-4-phényltétrahydrofurane
2,4-Nonadiénal
2,5-diméthyl-4-méthoxy-3(2H)-ruranone
2,6,10,10-tetramethyl-1-oxaspiro[4,5]dec-6-ene
2,6,6-triméthylester
2,6,9,10-tetramethyl-1-oxaspiro(4.5)deca- 3,6-diene
2,6-dimethyloct-7-en-4-one
2,6-diméthyl-octanal
2,6-nonadien-1-ol
2,6-nonadienal
2-butoxyéthanol
2-butyl-4,4,6-triméthyl-I,3-dioxane
2-décénal
2-éthoxy-2,6,6-triméthyl-9-méthylènebicyclo[3,3.1]nonane
2-heptyl tétrahydrofurane
2-heptylcyclopentan-1-one
2-hexène-1-ol
2-hexylcyclopent-2-en-1-one
2-hexylcyclopentan-1-one
2-hexylidène cyciopentanone
2-isopropoxyéthylbenzène
2-isopropyl-1,7,7-trimethylspiro[bicyclo[2.2.1]heptane- 2,4'-[1,3]dioxane]
2-isopropyl-5-méthyl-2-hexénal
2-isopropyl-N,2,3-triméthylbutyramide
2-mercapto-2-méthylpentan-1-ol
2-méthoxy-3-(1-méthylpropyl)pyrazine
2-méthoxynaphtalène
2-methyl-4-phenyl-1,3-dioxolane
2-méthyldécanenitrile
2-métoxynaphtalène
2-nonanol
2-nonanone
Acétal de propylène glycol de la 2-nonanone
2-nonen-1-al
2-pentylcyclopentan-1-ol
2-pentylcyclopentan-1-one
Propionate de 2-phényle
2-phénoxyéthanol
Acétate de 2-phényléthyle
2-propylheptanenitrile
2-trans-6-trans- onadienal
2-undécénal
2-undécénénitrile
3 -(3 isopropylphényl)butanal
3 -dimethyl-5(2,2,3-trimethyl-3-cyclopenten-lyl)-4-penten-2-ol
3-(2-éthylphényl)-2,2-diméthylpropanal
3-(3-isopropylphényl)butanal
3-(4-méthoxyphényl)-2-méthylpropanal
3-(4-méthylcyclohex-3-en-1-yl)butanal
3-(p-Isopropylphényl)propionaldéhyde
3,3-dimethyl-3-5(2,2,3-Trimethyl-3 -Cyclopentenyl)-4-penten-2-ol
3,6-nonadien-1-ol
Acétate de 3,6-diméthyl-3-oetanyi
3,7-diméthyl-1-octanol
3,7-diméthyl-2-méthylène-6-octénal
3a,4,5,6,7,7a-hexahydro-1H-4,7-methanoinden-6-yl acetate
Acide 3-cyclohexène-1-carboxylique
3-hexénol
Isovalérate de 3-hexényle
3-hydroxybutan-2-one
3-méthoxy-3-méthyl-butanol
3-méthoxy-7,7-diméthyl-10-méthylènebicyclo[4.3.1]décane
3-méthyl-1,2-cyclopentanedione
Alcool de 3-méthylphényle
3-nonylacroléine
4-(2-méthoxypropan-2-yl)-3-méthylcyclohex-1-ène
Acétate de 4-(tert-butyl)cyclohexyle
4-(tert-pentyl)cyclohexan-1-one
4,5,6,7-tétrahydro-3,6-diméthylbenzofurane
4-carvomenthenol
4-cyclohexyl-2-méthylbutan-2-ol
4-éthylguaiacol
4-methyl-2-phenyl-3,6-dihydro-2H-pyran
4-méthyl-2-phényltétrahydro-2H-pyran
4-méthylquinoléine
4-penténophénone
4-terpinénol
4-tétraéthyl-2-phényl-3,6-dihydro-2H-pyran
4-vinylphénol
5- isopropényl-2-méthyl-2-vinyltétrahydrofurane
5-ethyl-4-hydroxy-2-methylfuran-3(2H)-one
5-méthyl-3-heptanone
6,6 "diméthyl-2-norpmène-2-propionaïdéhyde
6,6-dimethoxy-2,5,5-trimethylhex-2-ene
6,6-diméthyl-2-méthylènebicyclo [3.1.1]heptan-3 -ol
6,6-diméthyl-2-norpinène-2-propionaldéhyde
6,8-diéthyl-2-nonanol
6-Isopropylquinoline
6-méthylquinoléine
7-épi-alpha-sélinène
7-epi-sesquithujene
7-isopropyl-8,8-dimethyl-6,10-dioxaspiro[4.5]decane
9-décénal
Acétaldéhyde benzyle 2-méthoxyéthyle acétal
Acétaldéhyde dipropyle acétal
Acétaldéhyde phényléthyle propyle acétal
Acétate C9
Acétoanisole
Aldéhyde C-11
Alloaromadendrène
Acétate d'allyle 2-(isopentyloxy)
Heptanoate d'allyle
Éther phénéthylique d'allyle
Acétate d'allyle-2-(isopentyloxy)
alpha -Thujone
alpha-4-diméthyl benzènepropanal
alpha-acétoxystyrène
alpha-Amorphène
alpha-Bergamotène
alpha-Cadinène
alpha-Cédrène époxyde
alpha-Cubébène
alpha-Fenchène
alpha-Gurjunene
alpha-Limonène
alpha-méthyl cinnamaldéhyde
alpha-Méthyl-cyclohexanepropanol
alpha-Muurolène
alpha-Patchouiene
alpha-Santalène
alpha-Sélinène
alpha-Terpinyl acétate
alpha-Terpinyl propionate
alpha-Thujone
Anéthole
Acétate d'anisyle
Formiate d'anisyle
Alcool benzylique
Butyrate de benzyle
Carbinol de benzyle et de diméthyle
Isobutyrate de benzyle
Isovalérate de benzyle
Tert-butanol de benzyle
Benzylacétone
bêta-caryophyllène
bêta-cédrène
bêta-Copaène
bêta-Farnesène
bêta-Patchoulline
bêta-Phellandrène
bêta-Pinène
bêta-Pinène époxyde
bêta-Sélinène
bêta-Sesquiphellandrène
bêta-Terpineol
acétate de bêta-terpinyle
Oxyde de bigarade
Bornéol
Acétate de bornyle
Isobutyrate de bornyle
Camphène
Camphre
Camphorquinone
Capraldéhyde
Alcool caprolique
Acide caprylique
Alcool caprylique
Caprylnitrile
Carbitol
Carvacrol
Carvone
Chloroxylénol
Alcool cinnamique
Aldéhyde cinnamique
Formiate de cinnamyle
Nitrile de cinnamyle
cis-2-hexénol
cis-3,cis-6-nonadienol
cis-3-hexène-1-ol
cis-3-hexényle 2-méthylbutyrate
cis-3-hexényle butyrate
cis-3-hexényl propionate
cis-3-hexényl tiglate
cis-3-hexényl valérate
cis-3-hexényl-cis-3-hexénoate
acétate de cis-4-(tert-butyl)cyclohexyle
cis-4-décène-1-al
cis-6-nonenol
cis-Carveol
oxyde de cis-Limonène
cis-Ocimène
cis-Pinane
Citral
Citral acétal de diméthyle
Citral acétal de propylène glycol
Citronellal
Formiate de citronnellyle
Citronellyl nitrile
Cosmène
Créosol
Alcool cumique
Acétate de cyclohexylethyle
Décahydro-3H-spiro[furan-2,5'-[4,7]méthanoindène]
Décahydro-bêta-naphtol
Décanal acétal diéthylique
Propionate de décyle
delta-3-Carène
delta-élément
Sulfure de dibutyle
Dihydro linalool
Dihydro-alpha-ionone
Acétate de dihydro-alpha-terpinyle
Dihydrocarveol
Dihydrocarveol
Acétate de dihydrocarvéol
Dihydrocarvone
Dihydroisophorone
Dihydrojasmone
Dihydromyrcénol
Diphénylméthane
Diphényloxyde
d-Limonène
Dodécanal diméthyl acétal
d-p-8(9)-Menthen-2-one
(1R, 6S)-2,2,6-triméthylcyclohexane-1-carboxylate d'éthyle
(3aR,4S,7R,7aR)-octahydro-3aH-4,7-méthanoindène-3a-carboxylate d'éthyle
Propionate d'éthyle et de 2-(cyclohexyle)
2-éthyl-6,6-diméthylcyclohex-2-ène-1-carboxylate d'éthyle
Décanoate d'éthyle
Gamma-safranate d'éthyle
Ethyl hexyl cétone
Nonanoate d'éthyle
Octanoate d'éthyle
Ethyl-(1R,2R,3R,4R)-3-isopropylbicyclo[2.2.1]hept-5-ene-2-carboxylate
Eucalyptol
Alcool fenchylique
Hexanoate de furforyle
gamma-Cadinène
gamma-Himachalène
gamma-Muurolène
Acétate de gamma-terpéninyle
gamma-Terpinène
gamma-Terpineol
Géranium
Formiate de géranyle
Nitrile de géranyle
Gyrane
Héliotropine
Heptaldéhyde
Acétate d'heptyle
Alcool d'heptyle
Tiglate d'hexényle
2-méthylbutanoate d'hexyle
Butyrate d'hexyle
Hexanoate d'hexyle
Propanoate d'hexyle
Tiglate d'hexyle
Alcool hydratopique
Acétal diméthylique de l'hydropaldéhyde
Alcool hydrocinnamylique
Acétal diméthylique de l'hydrotropaldéhyde
Acétate d'i-(3,3-diméthylcyclohexyle)éthyle
Irisnitrile
Isoamyl isobutyrate
Octanoate d'isoamyle
Isobornéol
Propionate d'isobornyle
Acétate d'isobornyle
Isobornyl isobutyrate
Isocyclocitral
Isomenthone
Isononanol
Acétate d'isononyle
Isopentyrate
2-méthylbiityrate d'isopropyle
Isopropylvinylcarbinol
Isopulegol
L- Carvone
Lauraldéhyde
Acétal de feuille
Oxyde de linalol (furanoïde)
Formiate de linalyle
Isobutyrate de linalyle
Propionate de linalyle
Macéal
Menthone
Méthoxycitronellal
Méthoxycyclododécane
Méthoxymelonat
(1s,4s)-1,4-diméthylcyclohexane-1-carboxylate de méthyle
(E)-non-2-énoate de méthyle
Diénoate de méthyle (E)-octa-4,7
2-méthylbutyrate de méthyle
2-octynoate de méthyle
Alpha-cyclogéranate de méthyle
Chavicol de méthyle
Cinnamate de méthyle
Acétate de méthyle cyclopentylidène
Éther diphénylique de méthyle
Eugénol de méthyle
Géraniate de méthyle
Isoeugénol de méthyle
Méthyl isoeugénol
Acétaldéhyde de méthyle nonyle
Méthyl nonyl acétaldéhyde diméthyl acétal
Méthylnonylcétone
Carbonate de méthyle octine
Acétaldéhyde de méthyle et d'octyle
Propionate de méthyle phényl carbinyle
Phénylacétate de méthyle
Phényléthylcarbinol de méthyle
Carboxylate de méthyle-2,2-diméthyl-6-méthylènecyclohexane-1
Méthyl-2-méthylbutyrate
Myrcène
Myrcénol
Acétate de myrcényle
Myroxide
Myrténal
Nérol
Formiate de néryle
n-Hexyl-2-butenoate
Nonaldéhyde
Alcool nonylique
O -Méthyl linalol
Ocimenol
Octahydro-1H-4,7-méthanoinde-5-yl acétate
Octahydro-1H-4,7-méthanoindène-1-carbaldéhyde
Octanal
Octanal acétal **de** diméthyle
Octanal acétal **de** propylène glycol
Acétate d'octyle
para-Cymen-8-ol
Isobutyrate **de** p-crésyle
p-Cymène
Perillaldéhyde
Acétate **de** périllyle
Alcool phénéthylique
Formiate **de** phényle
Phénylacétaldéhyde acétal d'éthylèneglycol
p-Isopropylphénylacétaldéhyde
p-Menth-3-en-1-ol
p-Propyl anisole
(S)-2-(tert-pentyloxy)propanoate **de** propyle
Propylène glycol
p-t-butyl phényl acétaldéhyde
p-tert-Amyl cyclohexanol
Alpha-Pinène racémique
Rhodinol
Sabinène
Sabinène hydraté
Sabinol
Safrole
Sélina-3,7(11)-diène
Spirodécane
Tétrahydrogeranial
Tétrahydrojasmone
Tétrahydrolinalool
Acétate de tétrahydrolmalyle
Thujopsène
Thymol
Éther méthylique de thymol
trans,trans-2,4-nonadienal
trans-2,cis-6-nonadienal
trans-2-décénal
trans-2-hexénol
trans-2-nonen-1-al
trans-2-tert-butylcyclohexanol
trans-3, cis-6-nonadienol
trans-4-Decen-1-al
trans-Anethole
trans-bêta-ocimène
trans-beta-ociraene
trans-Dihydrocarvone
trans-Géraniol
Undécanal

2. Mélange selon la revendication 1, dans lequel les 1,2-alcanediols (composant a) sont choisis dans le groupe constitué par le 1,2-pentanediol, le 1,2-hexanediol, le 1,2-heptanediol, le 1,2-octanediol, le 1,2-nonanediol, le 1,2-décanediol, le 1,2-undecanediol, le 1,2-dodécanediol et leurs mélanges.

3. Mélange selon la revendication 2, dans lequel le 1,2-alcanediol est choisi parmi le 1,2-hexanediol, le 1,2-octanediol et leurs mélanges.

4. Mélange selon la revendication 1, dans lequel les composés générateurs d'odeur (composant b) sont choisis dans le groupe constitué de
(b1) Acide butyrique, acide valérique, acide caproïque, acide énanthique, acide caprylique, acide pélargonique, acide caprique et leurs esters méthyliques, éthyliques. 1-hexène-3-one, 1-heptène-3-one, 1-octène-3-one, 1-nonène-3-one, 1-décène-3-one, octalactone, 1-hexène, 1-heptène, 1-octène, 1-nonène, 1-décène, 2-éthyl-1-butène, 2-éthyl-1-pentène, 2-éthyl-1-hexène, 2-éthyl-1-heptène, 2-éthyl-1-octène, cis-3-méthyl-2-pentène, cis-3-méthyl-2-hexène, cis-3-méthyl-2-heptène, cis-3-méthyl-2-octène, cis-3-méthyl-2-nones, trans-3-méthyl-2-pentène, trans-3-méthyl-2-hexène, trans-3-méthyl-2-heptène, trans-3-méthyl-2-octène, trans-3-méthyl-2-nones, n-hexane, n-heptane, n-octane, n-nonane, n-décane, cis-2-hexène, cis-2-heptène, cis-2-octène, cis-2-nones, cis-2-décène, trans-2-heptène, trans-2-heptène, trans-2-octène, trans-2-nones, trans-2-décène, cis-3-heptène, cis-3-heptène, cis-3-octène, cis-3-nones, cis-3-décène, trans-3-heptène, trans-3-heptène, trans-3-octène, trans-3-nones, trans-3-décène, cis-2,3-hexanediol, cis-2,3-heptanediol, cis-2,3-octanediol, cis-2,3-nonendiol, cis-2,3-décanediol, trans-2,3-hexanediol, trans-2, 3-heptanediol, trans-2,3-octanediol, trans-2,3-nonène-diol, trans-2,3-décanediol, cis-3,4-hexanediol, cis-3,4-heptanediol, cis-3,4-octanediol, cis-3,4-nonènediol, cis-3,4-décanediol, trans-3, 4-hexanediol, trans-3,4-heptanediol, trans-3,4-octanediol, trans-3,4-nonènediol, trans-3,4-décanediol, 3-mé-thylpentane-2,3-diol, 3-méthylhexane-2,3-diol, 3-méthylheptane-2,3-diol, 3-mé-thyloctane-2, 3-diol, 3-méthylnonane-2,3-diol, 2-éthylbutane-1,2-diol, 2-éthyl-pentane-1,2-diol, 2-éthylhexane-1,2-diol, 2-éthylheptane-1,2-diol, 2-éthyloctane-1,2-diol, ou leurs mélanges ; ou
(b2) ou un, deux, trois ou plusieurs composés selon l'une quelconque des formules (I) à (IV) suivantes 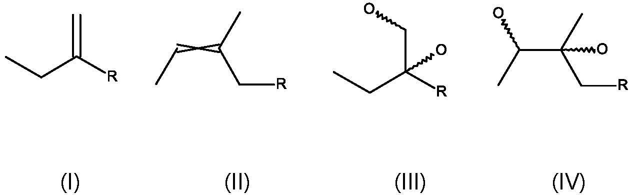 dans laquelle R représente un radical alkyle linéaire ramifié comportant de 1 à 10 atomes de carbone, y compris tous les stéréoisomères cis et trans, ainsi que leurs mélanges.

5. Mélange selon la revendication 1, dans lequel les composants (a) et (c) sont présents dans un rapport pondéral d'environ 90:10 à environ 99,99:0,01 et dans lequel le composant (b) est présent en une quantité d'environ 10 ppm à environ 60 000 ppm (0,001 - à 6 % en poids)

6. Mélange selon la revendication 1, dans lequel le composant (b) est présent en une quantité d'environ 50 ppm à environ 20 000 ppm (0,005 à 2 % en poids).

7. Mélange selon la revendication 1, dans lequel le composant (b) est présent en une quantité d'environ 100 ppm à environ 10 000 ppm (0,01 à 1 % en poids).

8. Mélange selon la revendication 1, comprenant en outre un véhicule liquide.

9. Mélange selon la revendication 8, dans lequel le support liquide est choisi dans le groupe constitué par l'eau, les_{alcools aliphatiques en C1-C4} et les polyols ayant de 2 à 12 atomes de carbone et de 1 à 5 groupes hydroxyle.

10. Produit de consommation comprenant la composition selon la revendication 1.

11. Produit selon la revendication 10, qui est une composition cosmétique, une composition pharmaceutique, une composition orale, une composition détergente ou une composition alimentaire.

12. Produit selon la revendication 10, contenant le mélange selon la revendication 1 en une quantité d'environ 0,0001 à environ 10 % en poids - calculée par rapport à la composition.

13. Produit selon la revendication 10, comprenant le mélange selon la revendication 1 en une quantité d'environ 0,005 à environ 5 % en poids - calculée sur la composition.

14. Produit selon la revendication 10, contenant le mélange selon la revendication 1 en une quantité d'environ 0,01 à environ 3 % en poids - par rapport à la composition.

15. Procédé pour masquer l'odeur désagréable de 1,2-alcanediols ou de produits contenant ces 1,2-alcanediols, comprenant ou consistant en les étapes suivantes :
(a) fournir une source d'au moins un 1,2-alcanediol ayant une odeur désagréable ou un produit contenant ledit au moins un 1,2-alcanediol ayant une odeur désagréable ; et
(b) l'addition d'au moins un matériau de réduction des odeurs ayant une valeur de réduction des odeurs (MORV) d'au moins 0,5 selon la revendication 1.

16. Utilisation d'au moins un matériau de réduction des odeurs ayant une valeur de réduction des odeurs (MORV) d'au moins 0,5 selon la revendication 1 pour masquer l'odeur désagréable des 1,2-alcanediols ou des produits les contenant.
